# EUROPEAN PATENT SPECIFICATION

(11) **EP 0 409 069 B1**
(45) Date of publication and mention of the grant of the patent: **02.06.1999**
(21) Application number: 90113296.9
(22) Date of filing: 12.07.1990
(51) Int. Cl.: C07C 261/02, C07C 261/04, C08G 73/06

(54) **Mesogenic polycyanates and polycyanamides and thermosets thereof**
Mesogene Polycyanate und Polycyanamide und ihre hitzegehärteten Gegenstände
Polycyanates et polycyanamides mésogènes et leurs produits thermodurcis

(30) Priority: 17.07.1989 US 380938
(43) Date of publication of application: 23.01.1991
(73) Proprietor: THE DOW CHEMICAL COMPANY, Midland, Michigan 48674 (US)
(72) Inventor: Hefner, Robert E., Jr., Lake Jackson, Texas 77566 (US); Earls, Jimmy D., Lake Jackson, Texas 77566 (US); Puckett, Paul M., Lake Jackson, Texas 77566 (US)
(74) Representative: Kremer, Robert A.M.

(56) References cited:
- EP-A- 0 291 552
- EP-A- 0 295 381
- US-A- 4 559 399
- Kirk-Othmer, Encyclopedia of Chemical Technology, third edition, volume 14, 1981, pages 414-418
- ACS Symposium Series 435, Liquid-Crystalline Polymers, R.A.Weis and C.K.Ober, 1990, page 2
- Polymeric Liquid Crystals, A.Blumstein, Plenum Press, 1983, pages 1-5
- Polymer Liquid Crystals, A.Ciferri et. al, Academic Press, 1982, pages 1-9
- Comprehensive Polymer Science, G.Allen et. al., Pergamon Press, 1989, page 723
- Critical Reports on Applied Chemistry Vol. 22, "Thermotropic Liquid Crystals" G.W.Gray, 1987, pages 76-77

## Description

The present invention concerns polycyanate or polycyanamides compositions containing one or more mesogenic or rodlike moieties.

Aromatic polycyanates which are thermosettable to polytriazines are known, for example, from U.S. Patent Nos. 3,448,079; 3,553,244; 3,694,410; 3,740,348; 3,755,402; 4,094,852 and 4,097,455. Said polytriazines possess excellent heat resistance, however, an improvement in their mechanical properties, especially tensile and flexural strength, tensile and flexural modulus and tensile elongation while maintaining or even increasing glass transition temperature, would be desirable. The present invention provides a method for improving one or more of the aforementioned properties by incorporation into the polymer chains of the polytriazines one or more mesogenic or rodlike structure(s). Incorporation of said mesogenic or rodlike structures can lead to a molecular level ordering of the polytriazine thermoset thereof.

EP-A-0295,381 discloses cyanate terminated Schiff's compounds and cites the dicyanate of bis (4'-hydroxy phenyl)-1,4-diiminomethylbenzene - no liquid crystallinity is reported or expected since the rigid central linkage is reacted out to give a crosslinked polymer structure.

US-A -4,559,399 relates to polymer modified cyanate compositions which don't carry mesogenic or rodlike moieties bridging two phenyl groups.

US-A -3,491,060 discloses polyimidocarbonic esters and cites among polyvalent cyanic acid esters, the 4,4'-dicyanatostilbene.

US-A- 4,738,900 relates to thermosetting compositions and mentions the 4,4'-dicyanamidoazobenzene, the 4,4'-dicyanamidobenzanilide and the 4,4'-dicyanamidophenyl benzoate.

US-A- 3,694,410 relates to the use of chelates in preparing polycyanurates and discloses ester containing diisocyanates.

The present invention also provides polymerizable mixtures containing one or more of the polycyanates (polycyanamides) containing mesogenic or rodlike structure(s) with, for example, one or more polycyanates (polycyanamides) which do not contain mesogenic or rodlike moieties, epoxy resins, polymaleimides, polyamines, polyphenols, polymerizable ethylenically unsaturated compounds, compounds which simultaneously contain both a cyanate or cyanamide group and a polymerizable ethylenically unsaturated group, compounds which simultaneously contain both a 1,2-epoxide group and a polymerizable ethylenically unsaturated group, compounds which simultaneously contain both a maleimide group and a cyanate or cyanamide group and materials which contain one or more mesogenic or rodlike structure(s) and an average of one cyanate or cyanamide group per molecule. The thermoset compositions prepared from the aforementioned polymerizable mixtures typically possess improvements in physical and mechanical properties relative to those thermoset compositions prepared using polycyanates (polycyanamides) which do not contain mesogenic or rodlike structure(s).

Certain of the polycyanates containing mesogenic or rodlike structure(s) exhibit novel thermally induced self-curing behavior at onset temperatures much lower than those encountered with the polycyanates which do not contain mesogenic or rodlike structure(s). Two distinct variations of this modified curing behavior are provided: As a specific example, when the azomethine group (-CH=N-) is present as a mesogenic or rodlike structure, it can lower the onset temperature to curing without participating in the formation of the curing structure of the thermoset product. As a second example, when the secondary amide group (-NH-CO-) is present, it too can lower the onset temperature to curing, but appears to participate in the formation of the curing structure of the thermoset product via reaction of the amide proton. When used as a component in the polymerizable mixtures of the present invention, these same polycyanates can often be used to reduce the onset temperature required to cure (thermoset) said mixture.

The present invention pertains to polycyanate or polycyanamide compositions containing one or more mesogenic or rodlike moieties represented by the following formulas I, II, III or IV: 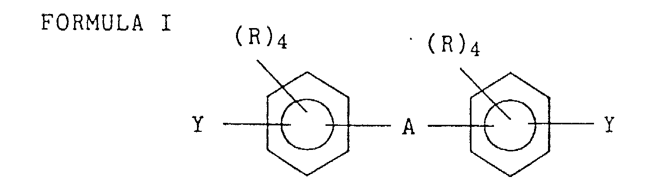 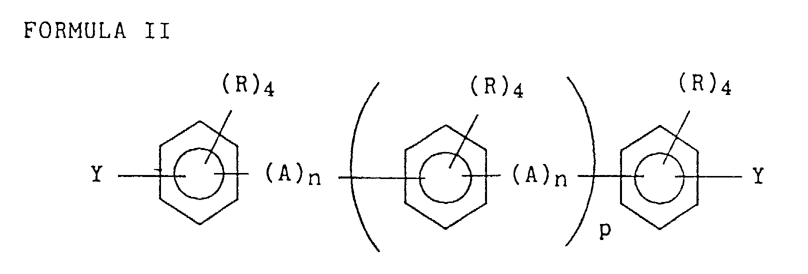 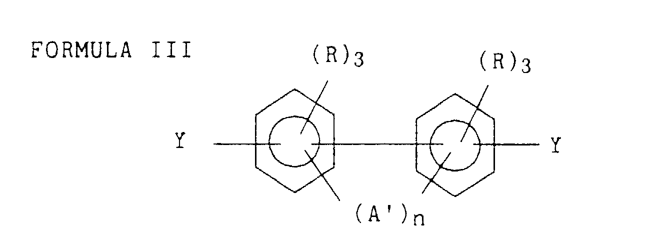 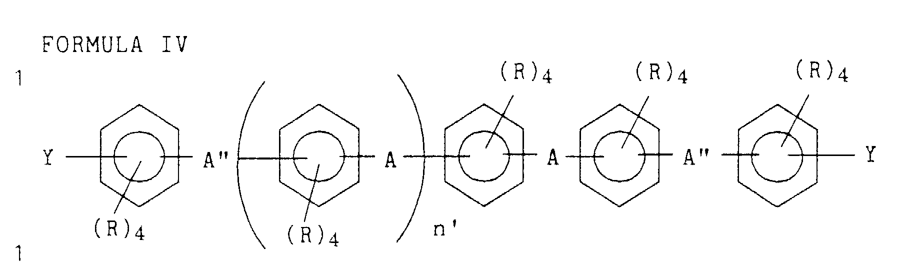 wherein at least about 80 percent of the -A- linkages, the direct bond in Formula III and the Y groups are in the para position with respect to each other; each Y is independently a -O-C≡N or a -NR¹-C≡N group; each A is independently -CR¹=CR¹-, -C≡C-, -N=N-, -CR¹=N-, -O-CO-, -NR¹-CO-, -CR¹=N-N=CR¹-, -CR¹=CR¹-CO, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 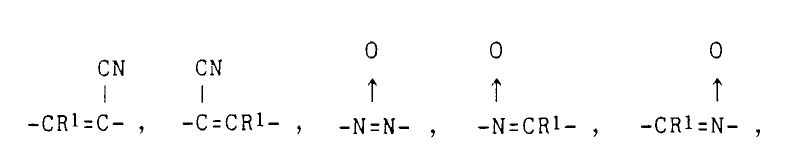 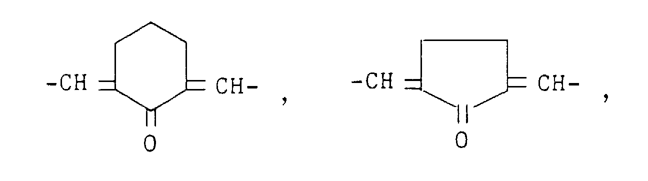 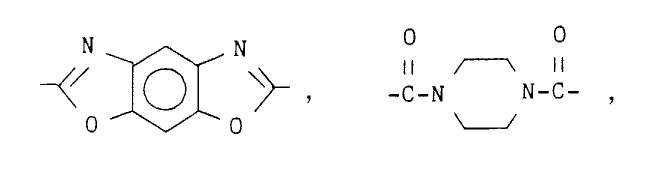 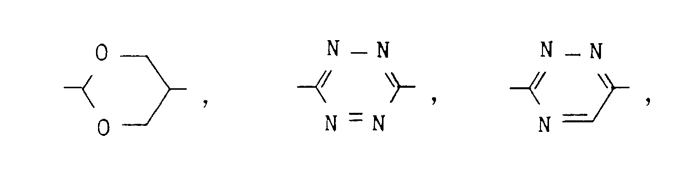 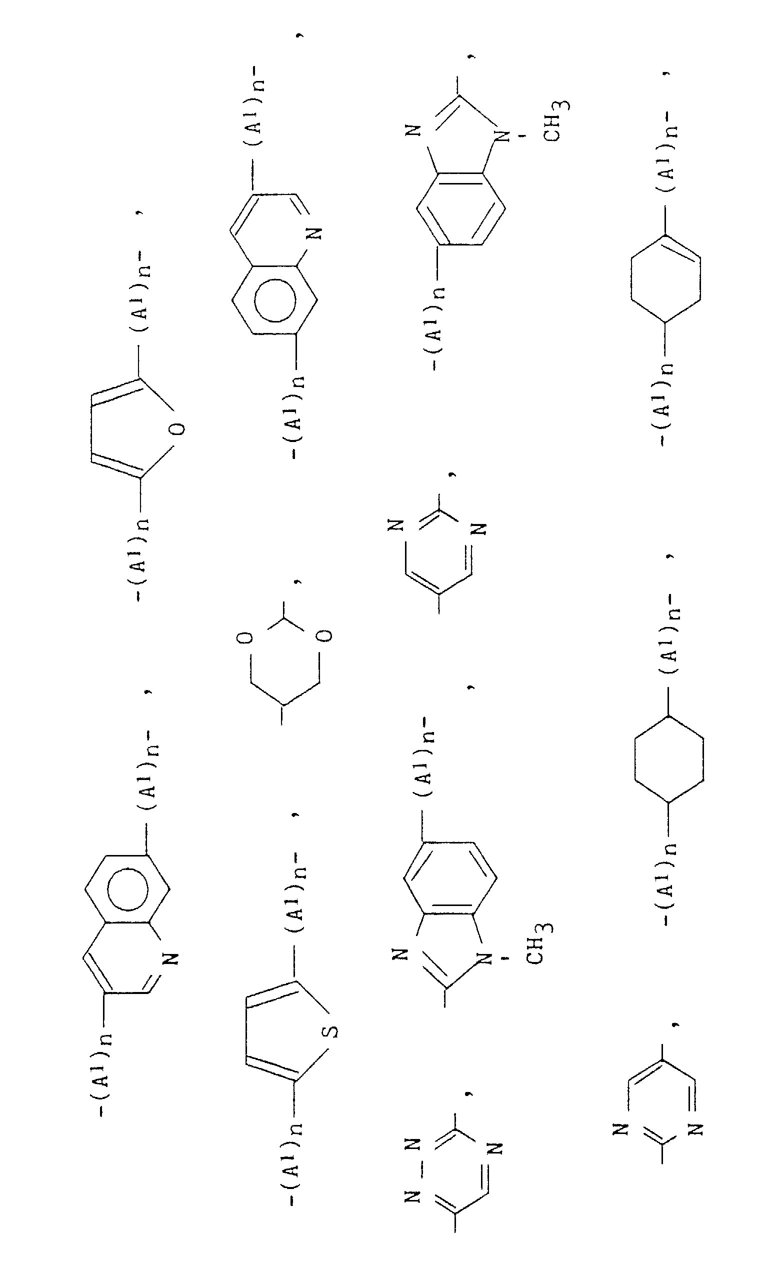 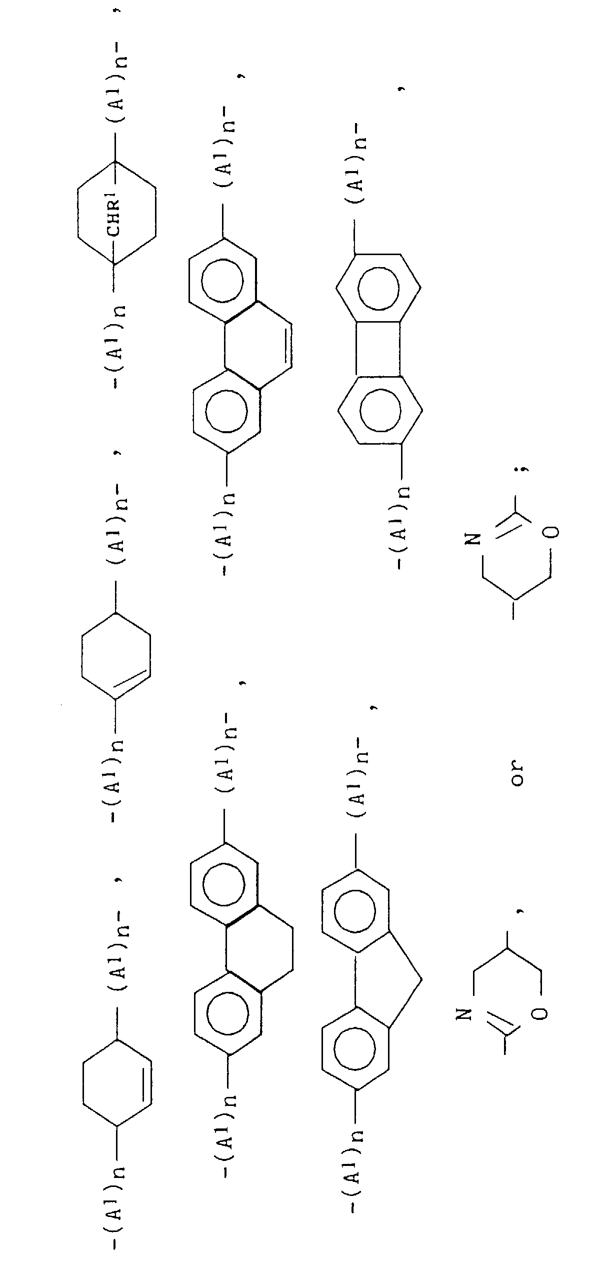 each A' is independently a divalent hydrocarbyl group having from 1 to 10, preferably from 1 to 4 carbon atoms; each A" is independently an alkylene group having from 1 to 10 carbon atoms, preferably from 1 to 4 carbon atoms, a direct bond, -O-, -CO-, -S-, -S-S-, -SO-, -SO₂- or -O-CO-O-; each A¹ is independently a -CO-, -O-CO-,-CO-O-, -CO-NR¹-, or -NR¹-CO- group; each R is independently hydrogen or a hydrocarbyl or hydrocarbyloxy group having from 1 to 10, preferably from 1 to 4 carbon atoms, a halogen atom, preferably chlorine or bromine, a nitro group, a nitrile group, a phenyl group or a -CO-R¹ group; each R¹ is independently hydrogen or a hydrocarbyl group having 1 to 3 carbon atoms; n has a value of zero or one; n' has a value from 1 to 6, preferably 1 to 3; p has a value from 1 to 30, preferably from 1 to 3; with the proviso that in formula I, where R = H and Y = -O-C≡N, A may not be -CH=CH- and with the proviso that in formula I, where R = H and Y = -NH-C≡N, A may not be -N=N-, -NH-CO- or -CO-O-, and with the proviso that in formula IV, where R is -H or - CH₃, n = 1, Y = -O-C≡N and A" is as hereinbefore defined, both A groups may not simultaneously be -O-CO-, the diisocyanate of bis (4'-hydroxyphenyl)-1,4-diiminomethylbenzene being excluded. The aromatic rings can also contain one or more heteroatoms selected from, for example, N, O or S.

The term "hydrocarbyl", as employed herein, means any aliphatic, cycloaliphatic, aromatic, aryl substituted aliphatic or cycloaliphatic, or aliphatic or cycloaliphatic substituted aromatic groups. The aliphatic or cycloaliphatic groups can be saturated or unsaturated. When applied to the A' group of Formula III, the hydrocarbyl group can also contain one or more heteroatoms selected from, for example, N, O or S. Likewise, the term "hydrocarbyloxy" means a hydrocarbyl group having an oxygen linkage between it and the carbon atom to which it is attached.

Another aspect of the present invention is directed to a process for preparing the above polycyanate or polycyanamide compositions containing one or more mesogenic or rodlike moieties.

Another aspect of the present invention pertains to compositions resulting from curing (thermosetting) one or more of the polycyanates or polycyanamides containing one or more mesogenic or rodlike moieties, optionally in the presence of one or more curing agents or curing catalysts.

Another aspect of the present invention is directed to polymerizable compositions comprising a mixture containing
(A) at least one thermosettable polycyanate or polycyanamide containing one or more mesogenic or rodlike moieties as defined in claim 1; and
(B) at least one of
   (1) at least one polycyanate or polycyanamide which does not contain mesogenic or rodlike structures;
   (2) at least one epoxy resin;
   (3) at least one polymaleimide;
   (4) at least one polyamine;
   (5) at least one polyphenol;
   (6) at least one compound containing one or more polymerizable ethylenically unsaturated group(s);
   (7) at least one compound which contains in the same molecule both a cyanate or cyanamide group and a polymerizable ethylenically unsaturated group;
   (8) at least one compound which contains in the same molecule both a 1,2-epoxide group and a polymerizable ethylenically unsaturated group;
   (9) at least one compound which contains in the same molecule both a maleimide group and a cyanate group;
   (10) at least one compound which contains one or more mesogenic or rodlike moieties and only one cyanate or cyanamide group per molecule;
   (11) at least one prepolymer of any of the aforesaid components (1) through (10) or any combination of any two or more of said components; or
   (12) a mixture of any two or more of components (1) through (11) in any proportion and any combination, with the proviso that component (A) may include 4,4'-dicyanatostilbene (formula I, R = H, Y = -O-C≡N, A = -CH=CH-) if components (B-4) or (B-5) are not used and with the proviso that component (A) may include 4,4'-dicyanamidoazobenzene (formula I, R = H, Y = -NH-C≡N, A = -N=N-), 4,4'-dicyanamidobenzanilide (formula I, R = H, Y = -NH-C≡N, A = -NH-CO-) or 4,4'-dicyanamidophenylbenzoate (formula I, R = H, Y = -NH-C≡N, A = -CO-O-) if components (B-3) or (B-4) are not used and with the proviso that component (A) may include the compositions of formula IV where R is -H or -CH₃, n = 1, Y = -O-C≡N, A" is as hereinbefore defined, where both A groups may simultaneously be -O-CO-.

Another aspect of the present invention is directed to a process for preparing the aforementioned polymerizable compositions.

Another aspect of the present invention pertains to compositions resulting from polymerizing the aforementioned polymerizable compositions.

A further aspect of the present invention pertains to products resulting from orienting any of the aforementioned polymerizable compositions.

The term prepolymers as employed herein means that the compound has been homooligomerized or cooligomerized or interoligomerized or homopolymerized or copolymerized or interpolymerized so as to cause an increase in molecular weight, but not to such an extent that the product has become cured, i.e. insoluble and infusible, but rather, the product is capable of being subsequently cured to an insoluble, infusible state.

### PREPARATION OF THE POLYCYANATES OR POLYCYANAMIDES CONTAINING ONE OR MORE MESOGENIC OR RODLIKE MOIETIES

The polycyanates or polycyanamides of the present invention are prepared by reacting one or more of the polyphenols, polyamines or aminophenols containing one or more mesogenic or rodlike moieties with a stoichiometric quantity or a slight stoichiometric excess (up to about 20 percent excess) of a cyanogen halide per -OH or -NHR¹ group in the presence of a stoichiometric quantity or a slight stoichiometric excess (up to about 20 percent excess) of a base compound per -OH or -NHR¹ group and in the presence of a suitable solvent.

Reaction temperatures of from -40°C to 60°C are operable, with reaction temperatures of -15°C to 10°C being preferred. Reaction times can vary substantially, for example, as a function of the reactants being employed, the reaction temperature, solvent(s) used and the scale of the reaction, but are generally between 15 minutes and 4 hours, with reaction times of 30 minutes to 90 minutes being preferred.

Suitable polyphenols, polyamines or aminophenols which can be employed herein to prepare the polycyanates or polycyanamides containing one or more mesogenic or rodlike moieties include, for example, any compound which has an average of more than one aromatic hydroxyl group, aromatic primary or secondary amino group or a combination of said hydroxyl and amino groups per molecule and include, for example, those represented by the following formulas V, VI, VII or VIII: 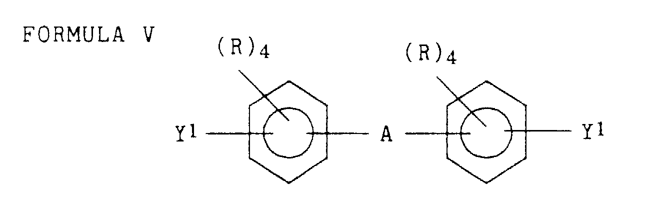 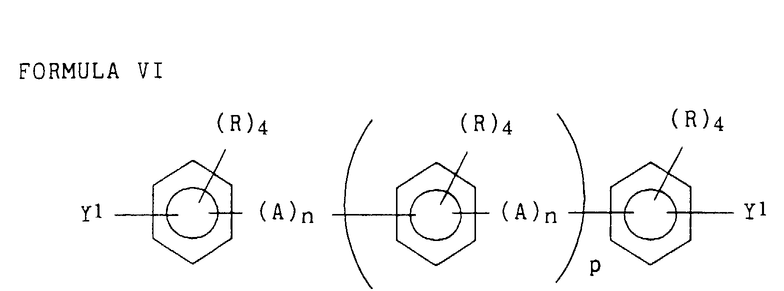 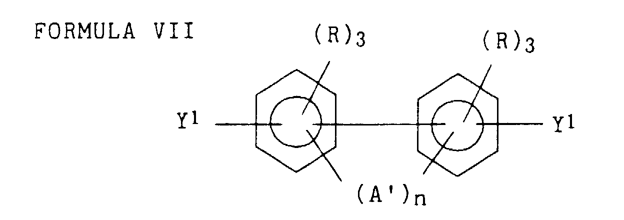 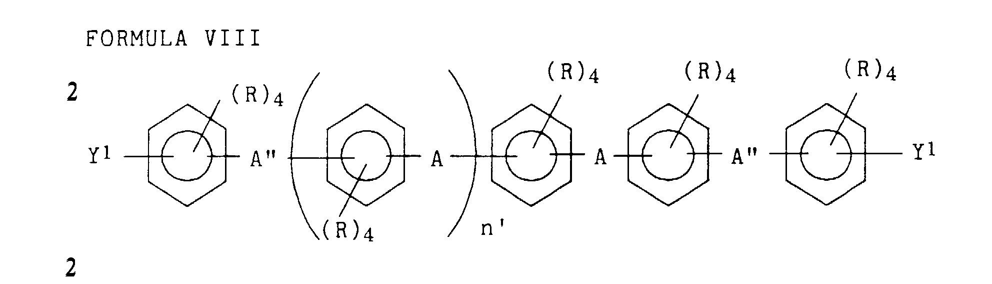 wherein at least about 80 percent of the -A- linkages in formulas V, VI and VIII and the direct bond between the two aromatic rings in Formula VII and the Y¹ groups are in the para position with respect to each other; each Y¹ is independently a -OH or -NHR¹ group; each A, A', A", A¹, R, R¹, n, n' and p are as hereinbefore defined. The aromatic rings can also contain one or more heteroatoms selected from, for example, N, O or S.

The term hydrocarbyl as employed herein means any aliphatic, cycloaliphatic, aromatic, aryl substituted aliphatic or cycloaliphatic, or aliphatic or cycloaliphatic substituted aromatic groups. The aliphatic or cycloaliphatic groups can be saturated or unsaturated. When applied to the A' group of Formula VII, the hydrocarbyl group can also contain one or more heteroatoms selected from, for example, N, O or S. Likewise, the term hydrocarbyloxy means a hydrocarbyl group having an oxygen linkage between it and the carbon atom to which it is attached.

Particularly suitable polyphenols are, for example, 4,4'-dihydroxy-alpha-methylstilbene, 4,4'-dihydroxy-chalcone, 4,4-dihydroxydiphenylacetylene, 4,4'-di-hydroxydiphenylazomethine, 4,4'-dihydroxyazobenzene, 4,4'-dihydroxyazoxybenzene, 4,4'-bis(4-hydroxy-phenoxy)diphenyl, 4,4'-dihydroxy-alpha-cyanostilbene, 4,4'-dihydroxybenzanilide, 4-hydroxyphenyl-4-hydroxybenzoate,

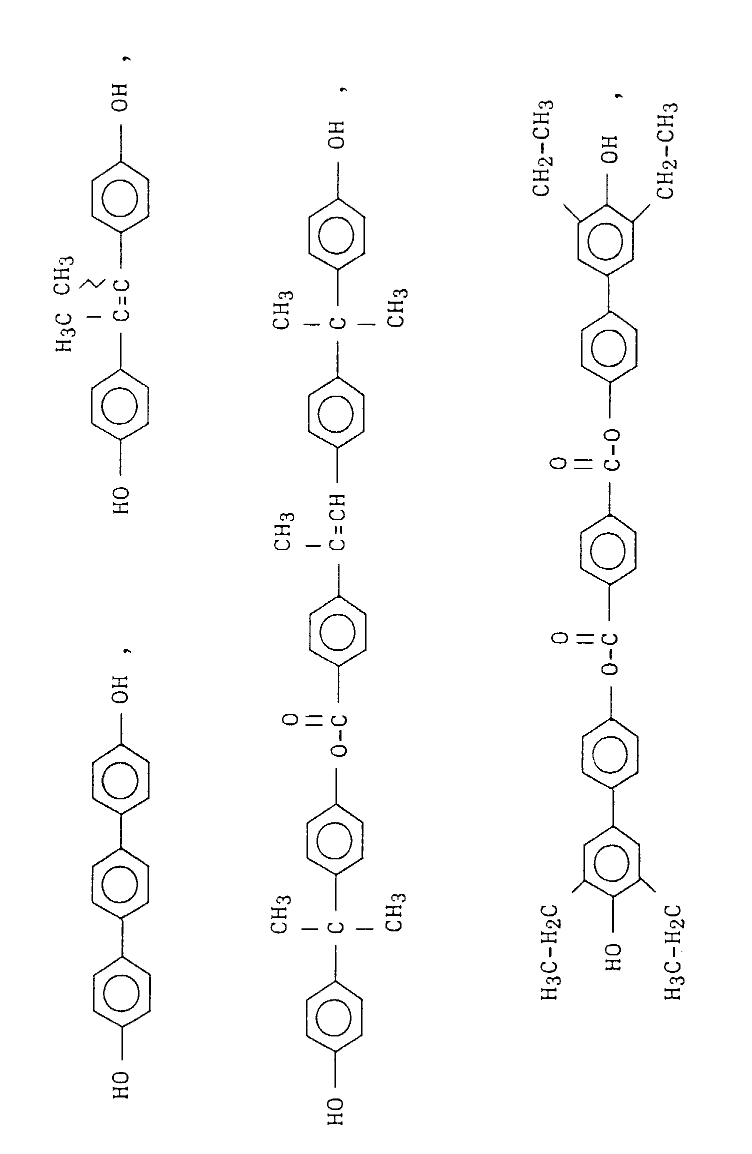

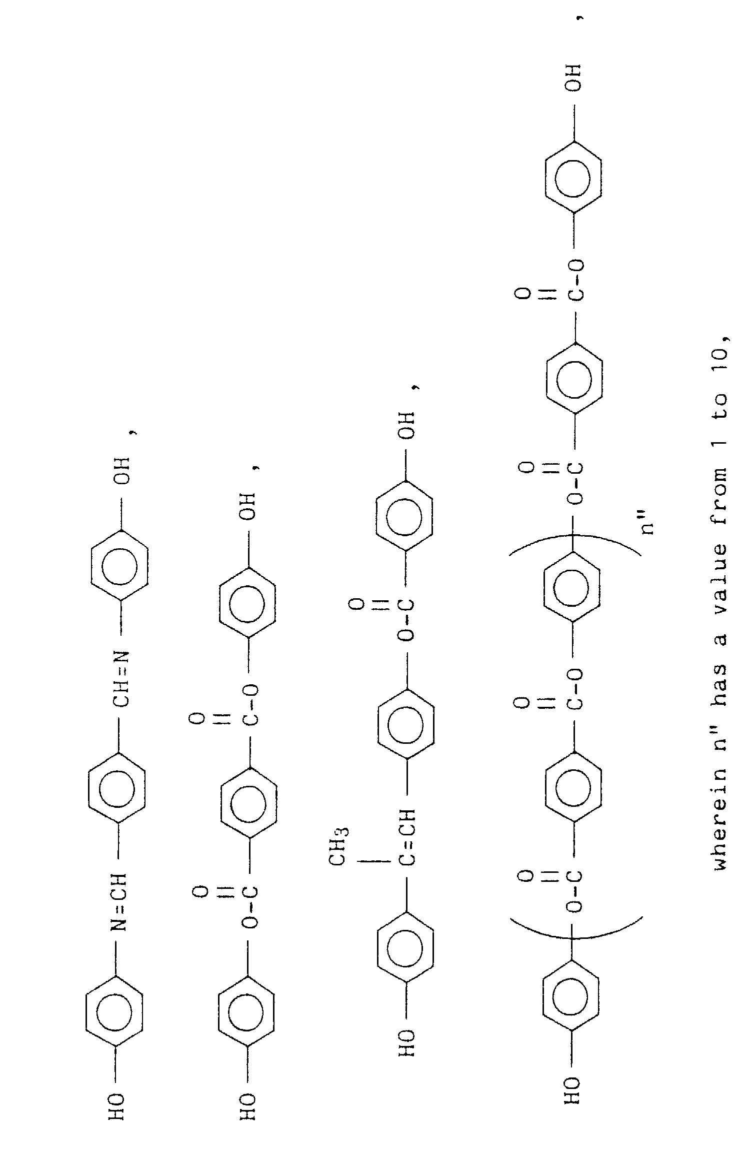

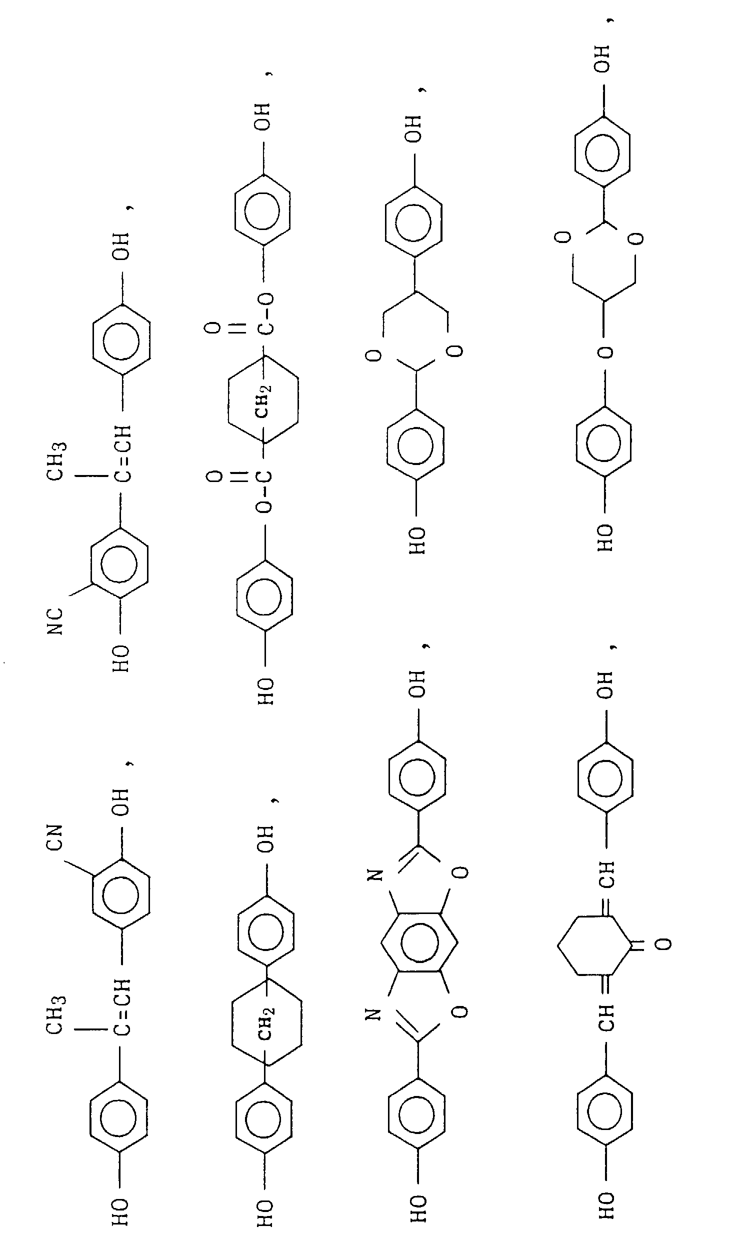

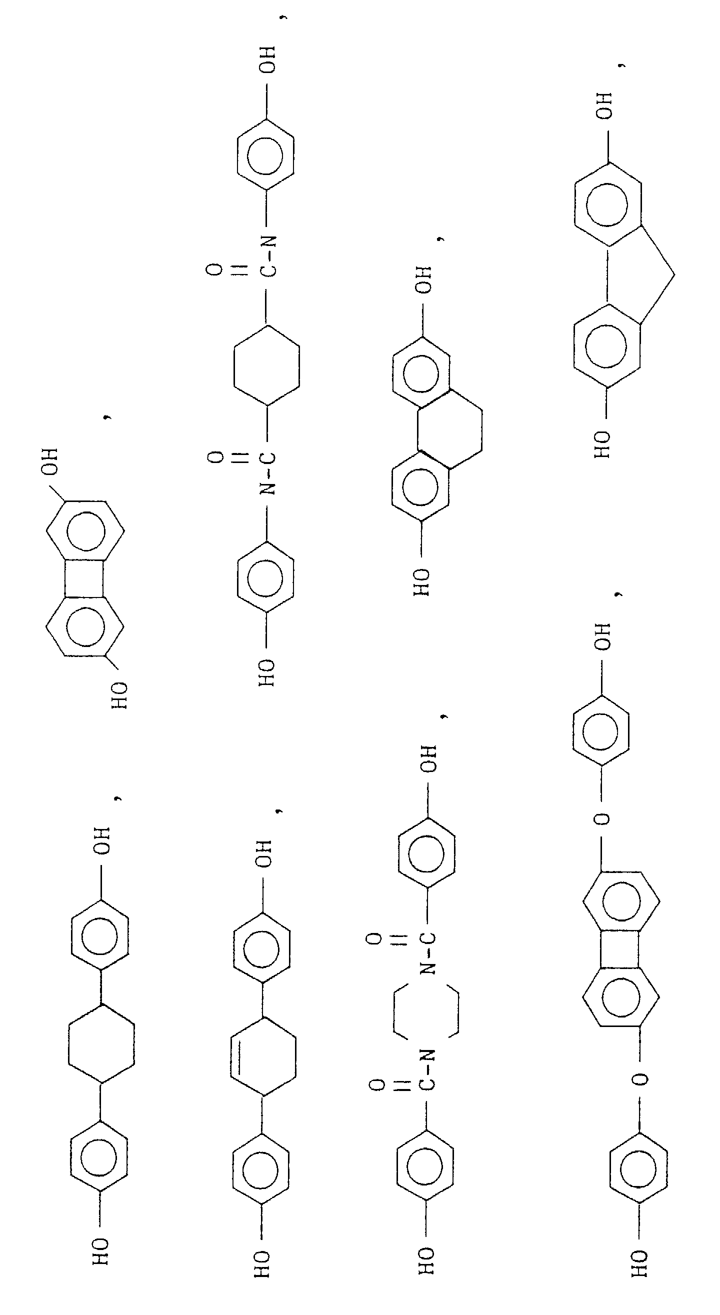

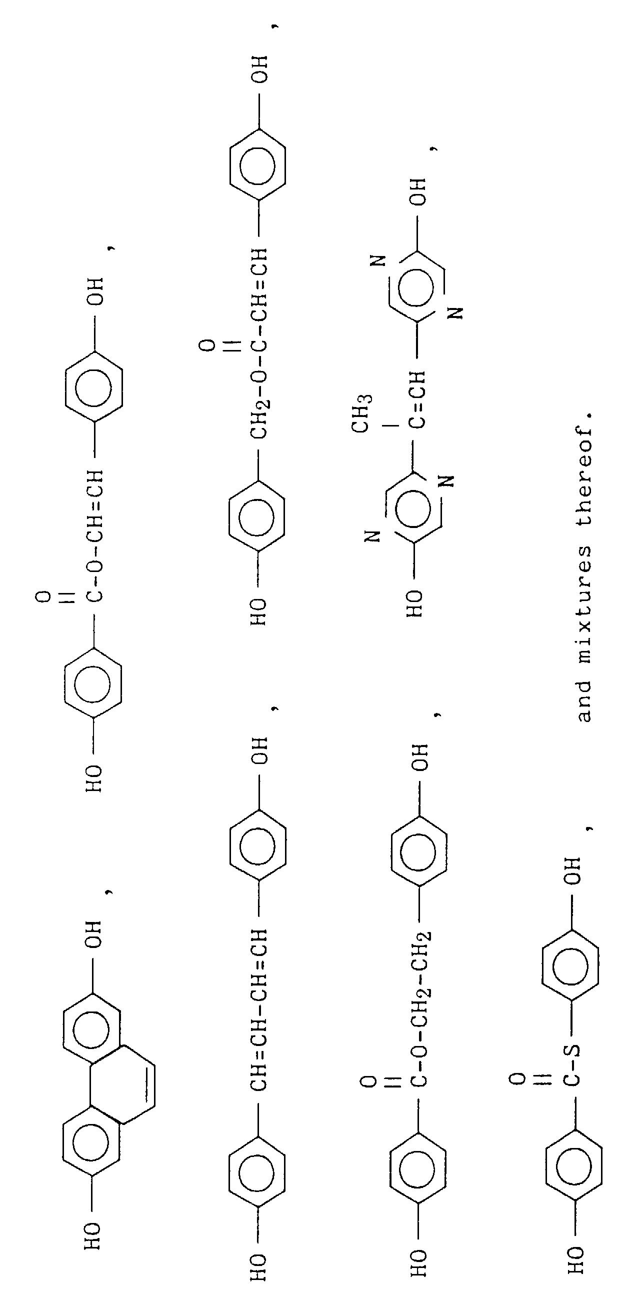

Particularly suitable polyamines are, for example, 4,4'-diaminostilbene, 4,4'-diamino-alpha-methyl-stilbene, 4,4'-diaminodiphenylacetylene, 4,4'-diamino-alpha-cyanostilbene, 4,4'-bis-(4-aminophenoxy)diphenyl, 4,4'-diaminodiphenylazomethine, 4,4'-diamino-3,3'-dichlorobenzanilide, 4,4'-diamino-3,3'-dichloroazobenzene,

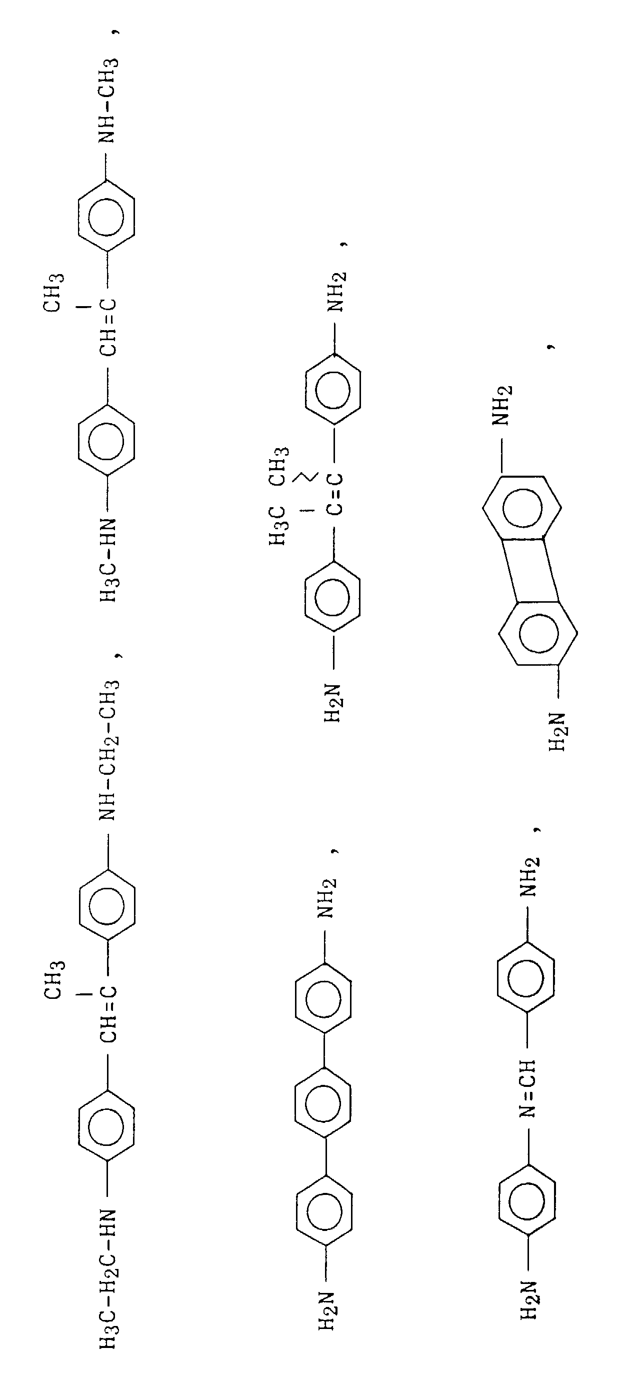

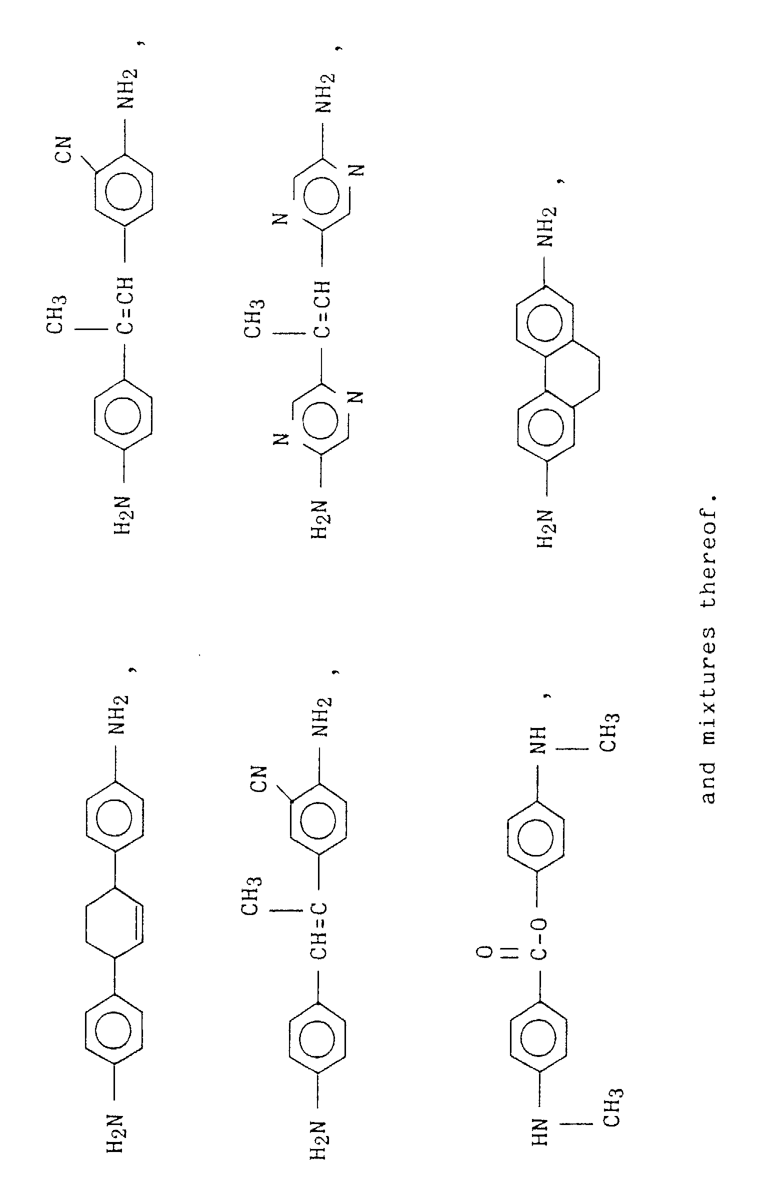

Particularly suitable aminophenols are, for example, 4-amino-4'-hydroxystilbene, 4-amino-4'-hydroxy-alpha-methylstilbene, 4-amino-4'-hydroxy-alpha-cyanostilbene, 4-amino-4'-hydroxybenzanilide and mixtures thereof.

Suitable cyanogen halides include cyanogen chloride and cyanogen bromide. Alternately, the method of Martin and Bauer described in Organic Synthesis, volume 61, pages 35-68 (1983) published by John Wiley and Sons can be used to generate the required cyanogen halide in situ from sodium cyanide and a halogen such as chlorine or bromine.

Suitable base compounds include both inorganic bases and tertiary amines such as sodium hydroxide, potassium hydroxide, trimethylamine, triethylamine and mixtures thereof. Triethylamine is most preferred as the base.

Suitable solvents for the cyanation reaction include, for example, water, aliphatic ketones, chlorinated hydrocarbons, aliphatic and cycloaliphatic ethers and diethers, aromatic hydrocarbons and mixtures thereof. Acetone, methylethylketone, methylene chloride or chloroform are particularly suitable as the solvent.

### CURING OF THE POLYCYANATES OR POLYCYANAMIDES CONTAINING ONE OR MORE MESOGENIC OR RODLIKE MOIETIES

The polycyanates or polycyanamides containing one or more mesogenic or rodlike structure(s) are cured (thermoset) by heating from 50°C to 400°C, preferrably by heating from 100°C to 250°C, optionally in the presence of a suitable catalyst. Suitable catalysts include, for example, acids, bases, salts, nitrogen and phosphorus compounds, such as Lewis acids such as AlCl₃, BF₃, FeCl₃, TiCl₄, ZnCl₂, SnCl₄; protonic acids such as HCl, H₃PO₄; aromatic hydroxy componds such as phenol, p-nitrophenol, pyrocatechol, dihydroxynaphthalene; sodium hydroxide, sodium methylate, sodium phenolate, trimethylamine, triethylamine, tributylamine, diazabicyclo-(2.2.2)-octane, quinoline, isoquinoline, tetrahydroisoquinoline, tetraethylammonium chloride, pyridine-N-oxide, tributyl phosphine, zinc octoate, tin octoate, zinc naphthenate, cobalt naphthenate, cobalt octoate and cobalt acetylacetonate. Also suitable as catalysts are the metal chelates such as, the chelates of transition metals and bidentate or tridentate ligands, particularly the chelates of iron, cobalt, zinc, copper, manganese, zirconium, titanium, vanadium, aluminum and magnesium. These and other operable catalysts are disclosed in U.S. Patent Nos. 3,694,410 and 4,094,852. Cobalt naphthenate, cobalt octoate and cobalt acetylacetonate are most preferred as the catalysts. The quantity of catalyst used, if any, depends on, for example, the structure of the particular catalyst, the structure of the polycyanate or polycyanamide being cured, the cure temperature and the cure time. Generally, catalyst concentrations of from 0.001 to 2 percent by weight are preferred.

B-staging or prepolymerization of the compositions of the polycyanates or polycyanamides of the present invention can be accomplished by using lower temperatures and/or shorter curing times. Curing of the thus formed B-staged (prepolymerized) resin can then be accomplished at a later time or immediately following B-staging (prepolymerization) by increasing the temperature and/or curing time.

The cured (thermoset) products prepared from the polycyanates or polycyanamides containing mesogenic or rodlike structure(s) possess the cyanate group homopolymerization structure 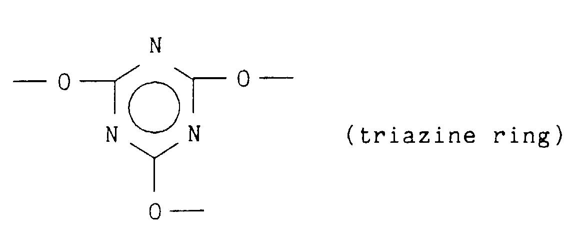 or the cyanamide group homopolymerization structure unless other functionalities are present in the polycyanate or polycyanamide that participate in the curing process. Such a case occurs in the curing of 4,4'-dicyanatobenzanilide where participation of the secondary amide hydrogen in the curing process leads to the formation of additional curing structure(s).

### POLYCYANATES OR POLYCYANAMIDES WHICH DO NOT CONTAIN MESOGENIC OR RODLIKE MOIETIES AND WHICH CAN BE EMPLOYED IN THE CURABLE AND CURED COMPOSITIONS

Suitable polycyanates or polycyanamides which do not contain mesogenic or rodlike structures and which can be employed to prepare the polymerizable mixtures of the present invention include, for example, those represented by the following formulas IX, X, XI and XII: 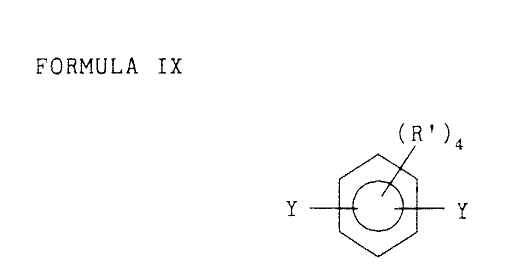 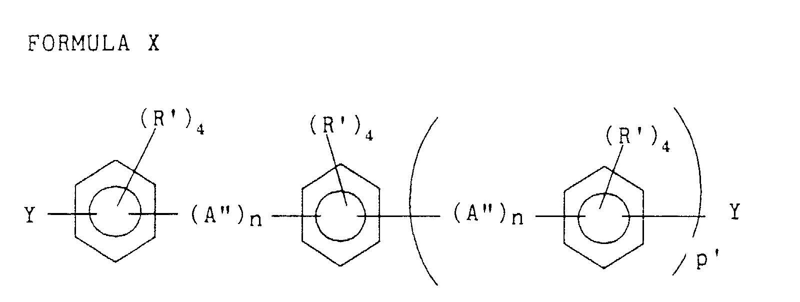 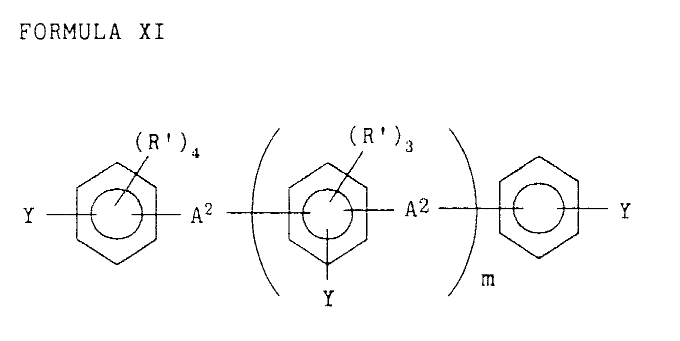 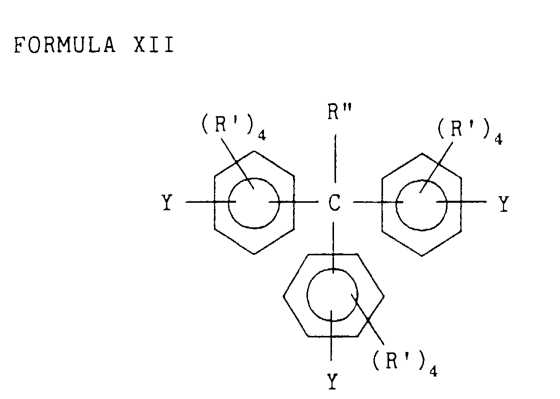 wherein Y, A" and n are as hereinbefore defined; each A² is independently an alkylene group having from 1 to 10, preferably from 1 to 4 carbon atoms or a 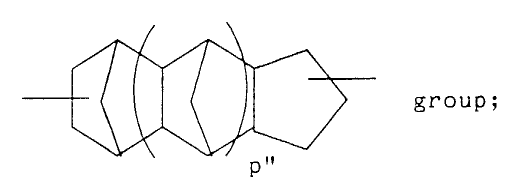 each R' is independently hydrogen, a hydrocarbyl or hydrocarbyloxy group having from 1 to 10, preferably 1 to 4 carbon atoms, a halogen, preferably chlorine or bromine, a phenyl group, a -O-C≡N group, or a -N-R¹-C≡N group; each R" is independently hydrogen, a hydrocarbyl or hydrocarbyloxy group having from 1 to 10, preferably 1 to 4 carbon atoms, a halogen, preferably chlorine or bromine, or a phenyl group; p' has a value from zero to 100, preferably from zero to 30; p" has a value of from zero to 10, preferably from zero to 3 and m has a value of from 0.001 to 6, preferably from 0.01 to 3. The aromatic rings can also contain one or more heteroatoms selected from, for example, N, O or S.

Suitable polycyanates or polycyanamides which do not contain mesogenic or rodlike structures represented by formulas IX, X, XI and XII include, for example, bisphenol A dicyanate, the dicyanates of 4,4'-dihydroxydiphenyl, 4,4'-dihydroxydiphenyl oxide, resorcinol, hydroquinone, 4,4'-thiodiphenol, 4,4'-sulfonyldiphenol, 3,3',5,5'-tetrabromobisphenol A, 2,2',6,6'-tetrabromobisphenol A, 2,2'-dihydroxydiphenyl, 3,3'-dimethoxybisphenol A, 4,4'-dihydroxydiphenylcarbonate, dicyclopentadiene diphenol, 4,4'-dihydroxybenzophenone, 4,4'-dihydroxydiphenyl methane, tricyclopentadiene diphenol, the tricyanate of tris(hydroxyphenyl)methane, the tetracyanate of 2,2',4,4'-tetrahydroxydiphenyl methane, the polycyanate of a phenolformaldehyde condensation product (novolac), the polycyanate of a dicyclopentadiene and phenol condensation product, the dicyanamide of 4,4'-diaminodiphenyl methane and the cyanate/cyanamide of p-aminophenol.

The polycyanates or polycyanamides which do not contain mesogenic or rodlike structures are prepared using the corresponding polyphenol, polyamine or aminophenol precursor and the previously described cyanation (cyanamidation) chemistry. As a specific process unique to the present invention, mixtures of one or more polyphenols, polyamines or aminophenols which do not contain mesogenic or rodlike structures with one or more polyphenols, polyamines or aminophenols which contain one or more mesogenic or rodlike structure(s) may be cyanated (cyanamidated) to provide a polymerizable mixture of the present invention.

### EPOXY RESINS WHICH CAN BE EMPLOYED IN THE CURABLE AND CURED COMPOSITIONS

Suitable epoxy resins which can be employed to prepare the polymerizable mixtures of the present invention include materials having an average of more than one vicinal epoxide group per molecule for example, the epoxy resins represented by the following formulas XIII, XIV, XV, XVI, XVII, XVIII, XIX, XX, XXI, XXII: 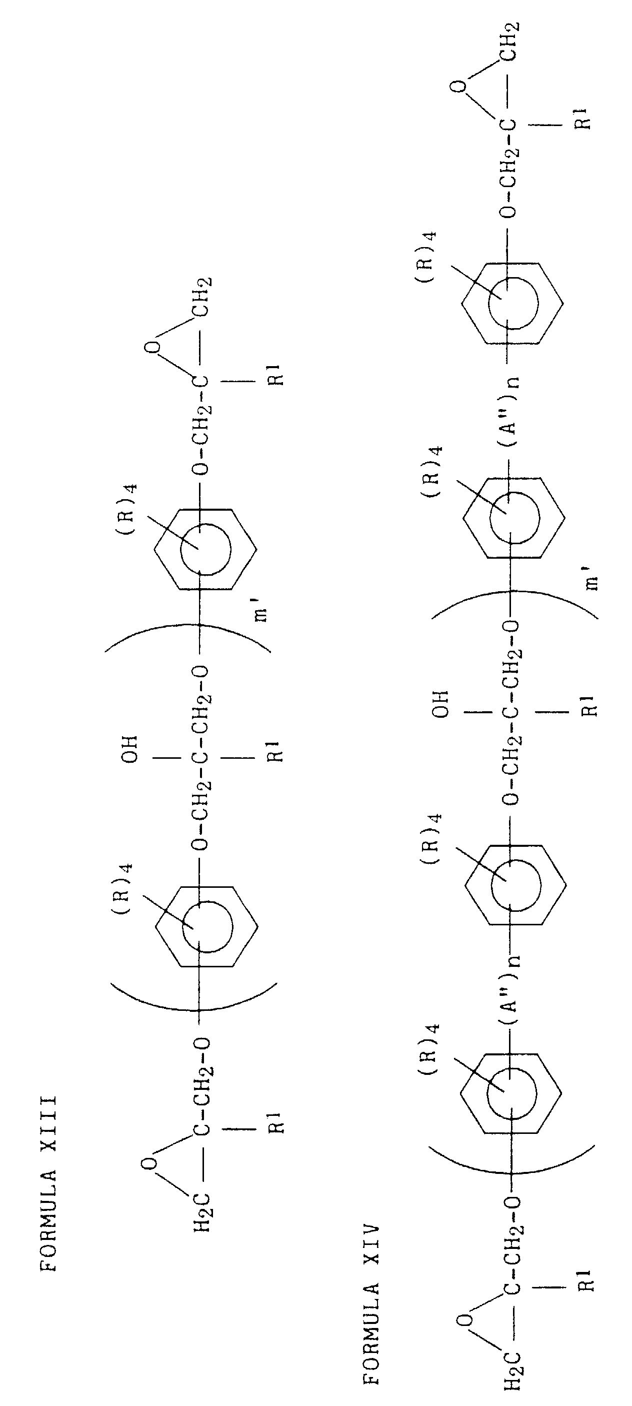 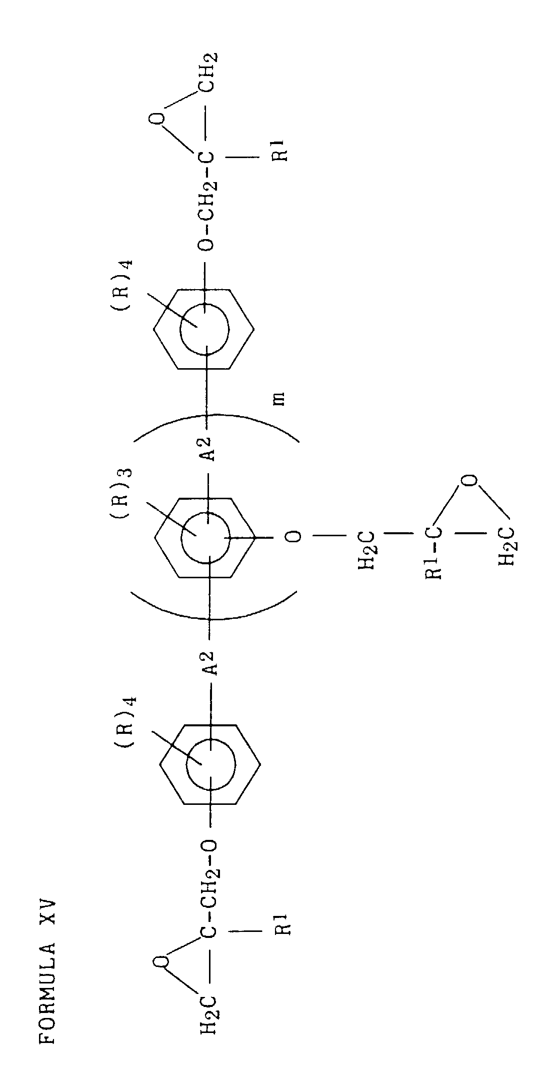 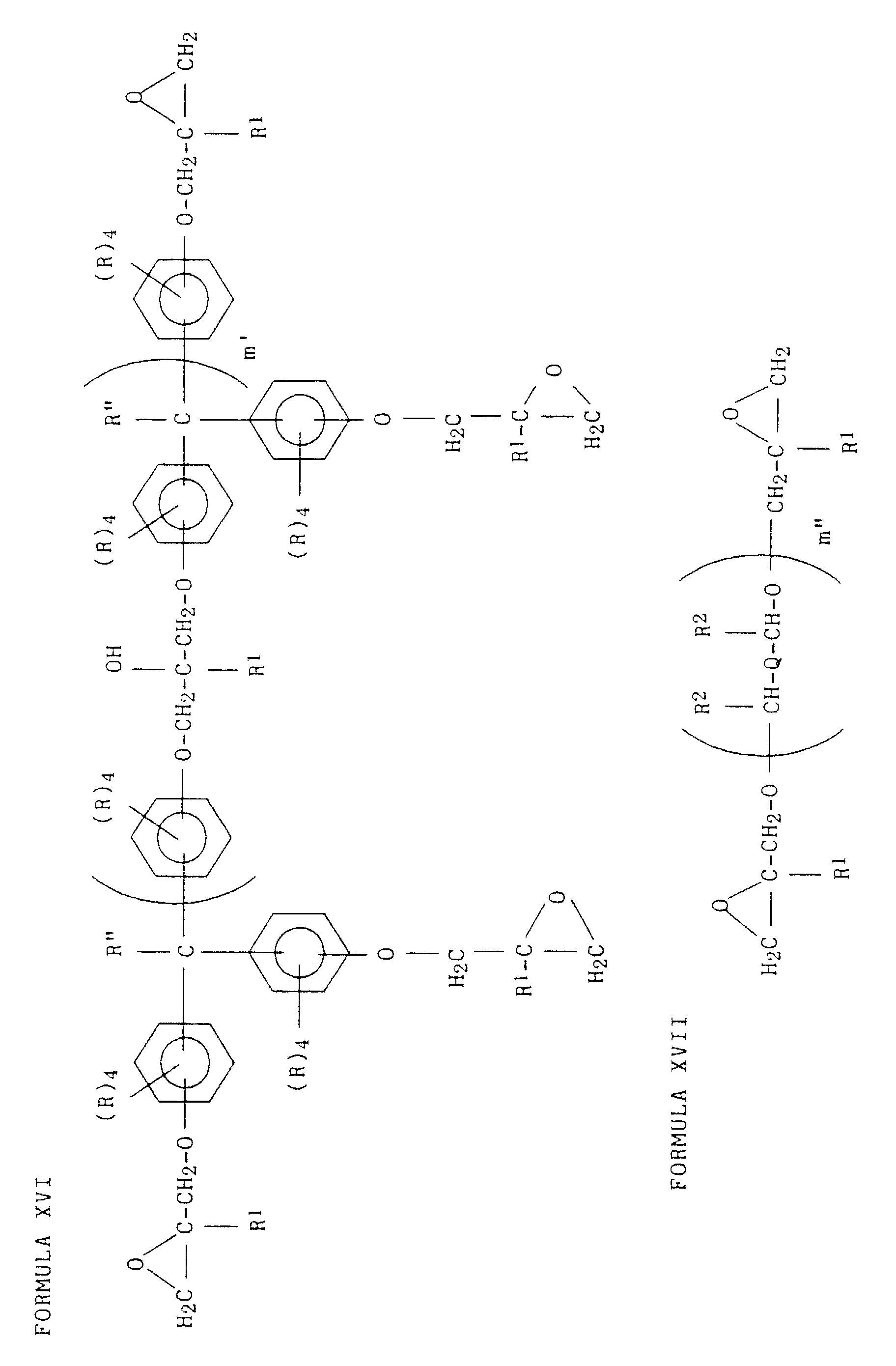 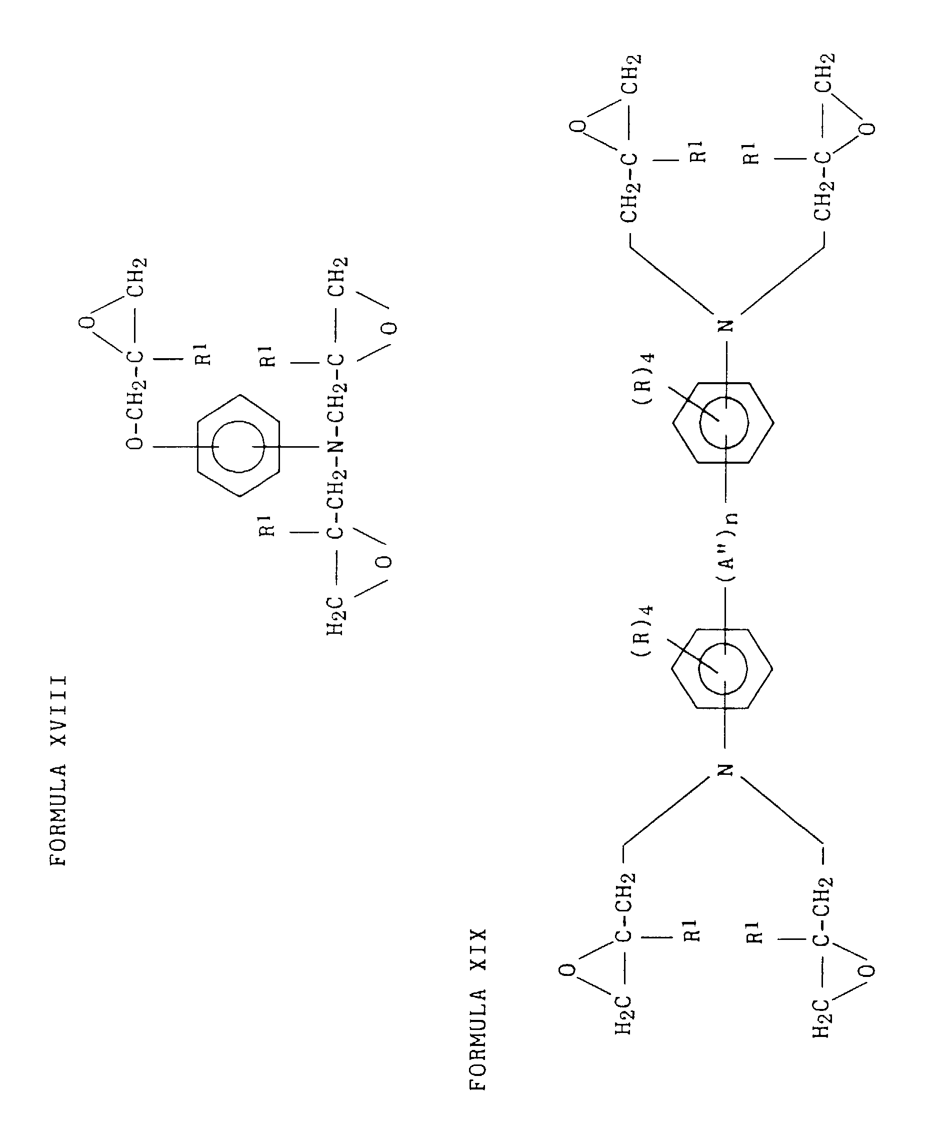 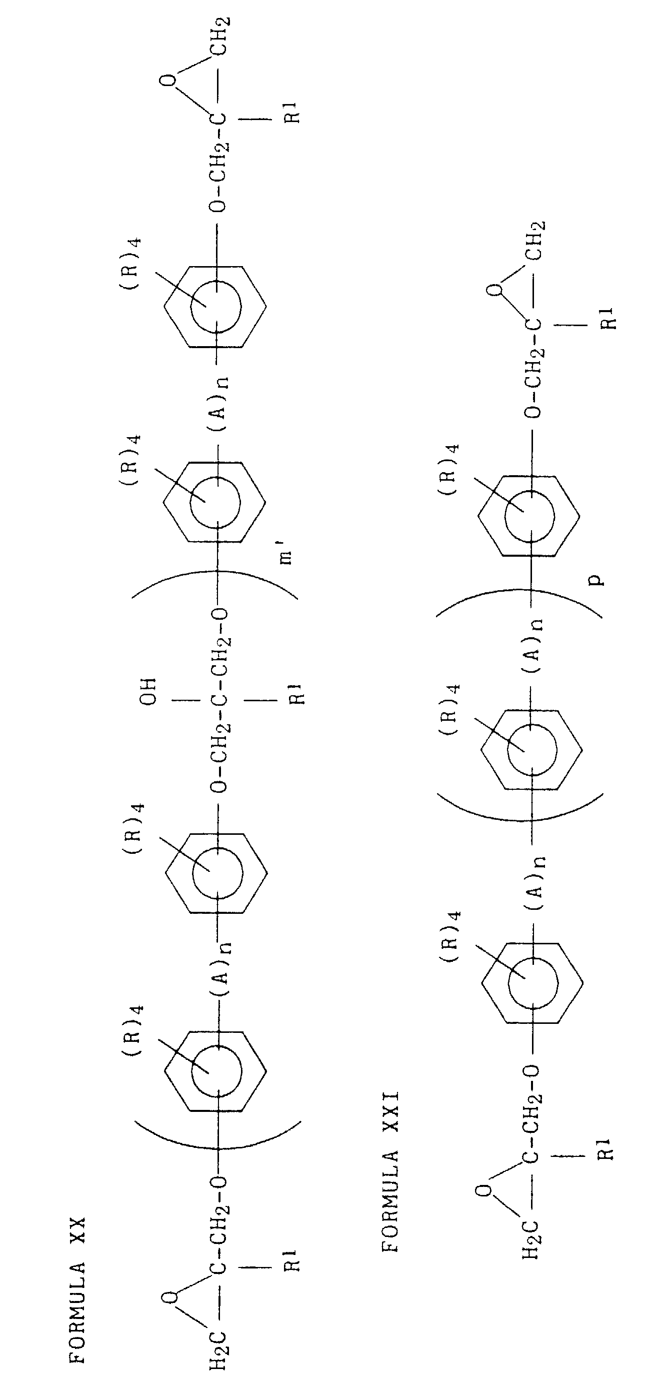 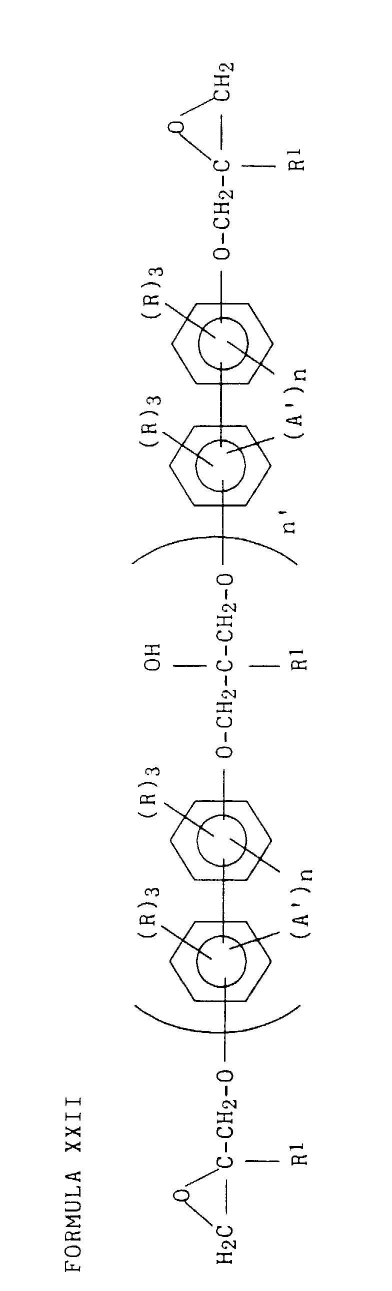 wherein A, A², A', A", R, R¹, R², R", m, n, n' and p are as hereinbefore defined; each R³ is independently hydrogen, or a hydrocarbyl or halohydrocarbyl group having from 1 to 6, preferably 1 to 2 carbon atoms; Q is a direct bond, -CH₂-S-CH₂-, -(CH₂)_{n"}-, or 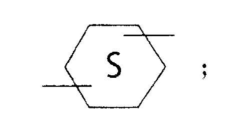 m' has a value of from zero to 30, preferably from zero to 5; m" has a value from 1 to 10, preferably from 1 to 4 and n" has an average value from 1 to 10. The aromatic rings can also contain one or more heteroatoms selected from, for example, N, O or S.

Particularly suitable epoxy resins represented by formulas XIII, XIV, XV, XVI, XVII, XVIII, XIX, XX, XXI and XXII are, for example, the diglycidyl ethers of resorcinol, hydroquinone, dihydroxydiphenyl methane, bisphenol A, 3,3',5,5'-tetrabromobisphenol A, 4,4'-sulfonyldiphenol, 4,4'-thiodiphenol, 4,4'-dihydroxydiphenyl oxide, 4,4'-dihydroxybenzophenone, 2,2'-dihydroxydiphenyl, dicyclopentadiene diphenol, tricyclopentadiene diphenol, 4,4'-dihydroxydiphenyl, 4,4'-dihydroxystilbene, 4,4'-dihydroxy-alpha-methylstilbene, 4,4'-dihydroxy-alpha-cyanostilbene, 4,4'-dihydroxychalcone, 4,4'-dihydroxydiphenylacetylene, 4,4'-dihydroxy-diphenylazomethine, 4,4'-dihydroxyazobenzene, 4,4'-bis(4-hydroxyphenoxy)diphenyl, 4,4'-dihydroxybenzanilide, ethylene glycol, thiodiglycol, diethylene glycol, dipropylene glycol, polypropylene glycol, polyethylene glycol, 1,4-cyclohexanediol, dibutylene glycol, the advancement reaction product of the diglycidyl ether of bisphenol A and bisphenol A, the advancement reaction product of the diglycidyl ether of 4,4'-dihydroxy-alpha-methylstilbene and 4,4'-dihydroxy-alpha-methylstilbene, the triepoxide of p-aminophenol, the tetraepoxide of 4,4'-diaminodiphenyl methane, the triglycidyl ether of tris-(hydroxyphenyl)methane, the tetraglycidyl ether of 2,2',4,4'-tetrahydroxydiphenyl methane, the polyglycidyl ether of a phenolformaldehyde condensation product (novolac) and the polyglycidyl ether of a dicyclopentadiene or oligomer thereof and phenol or halogen or alkyl substituted phenol condensation product.

The aforementioned epoxy resins can be prepared by reaction of a polyphenol (polyamine, aminophenol, polyalkylene glycol) with an epihalohydrin and a basic acting material. Said reaction generally involves two distinct steps: coupling reaction of the epihalohydrin and polyphenol to provide a halohydrin intermediate and dehydrohalogenation reaction of the halohydrin intermediate to provide the glycidyl ether product. Suitable catalysts and reaction conditions for preparing epoxy resins are described in the Handbook of Epoxy Resins by Lee and Neville, McGraw-Hill (1967).

### POLYMALEIMIDES FOR USE IN THE CURABLE AND CURED COMPOSITIONS

Suitable polymaleimides which can be employed to prepare the polymerizable mixtures of the present invention include, for example, those represented by the following formulas XXIII, XXIV, XXV, XXVI, XXVII, XXVIII, XXVIX, XXX: 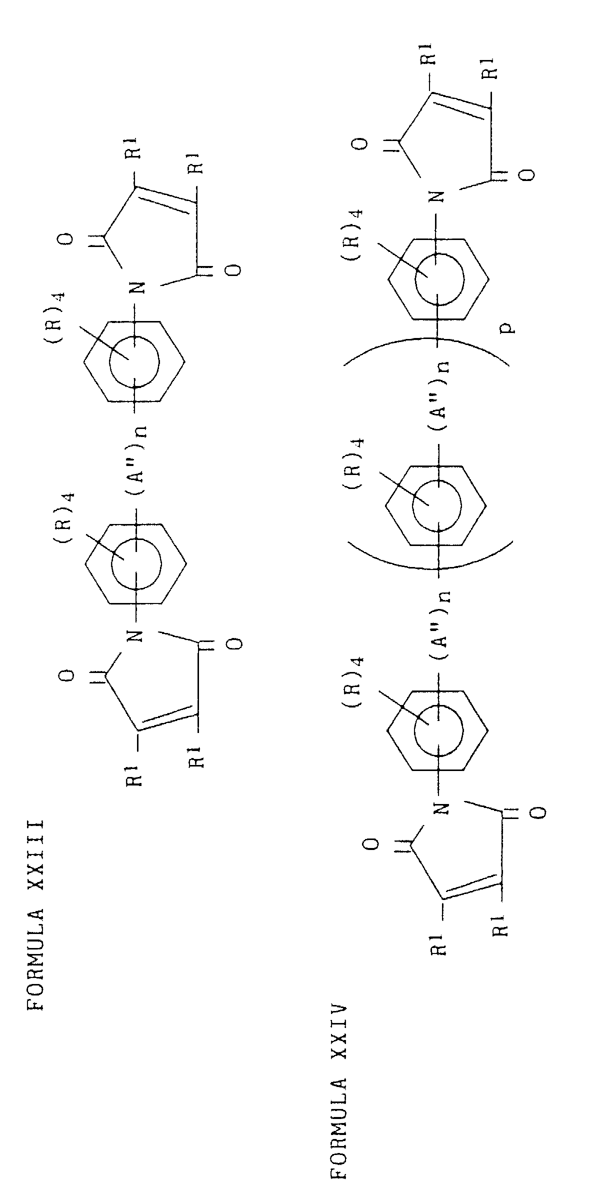 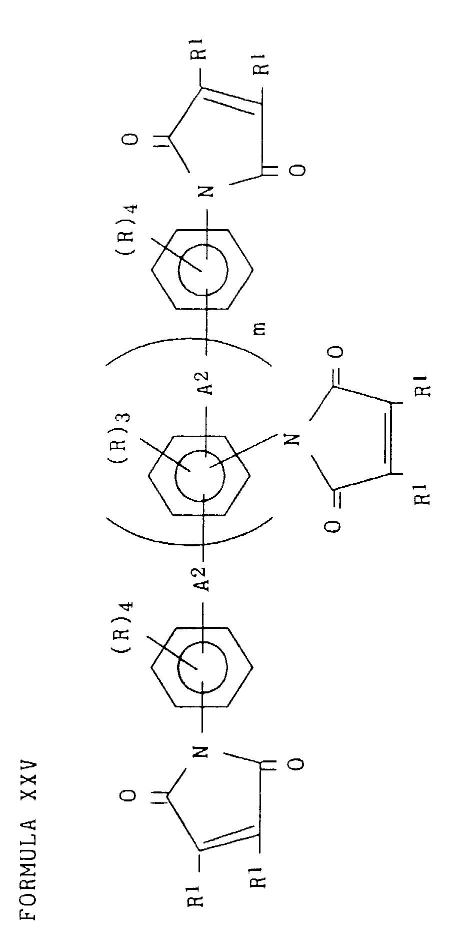 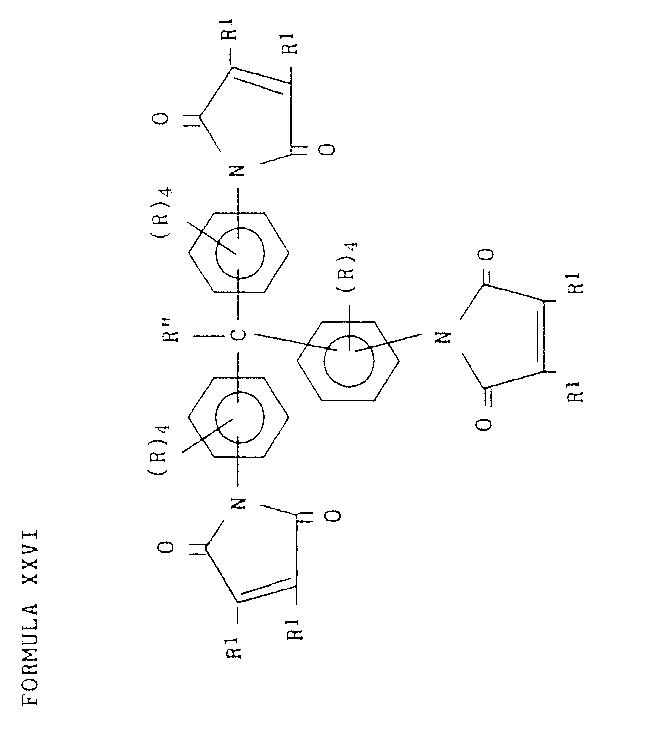 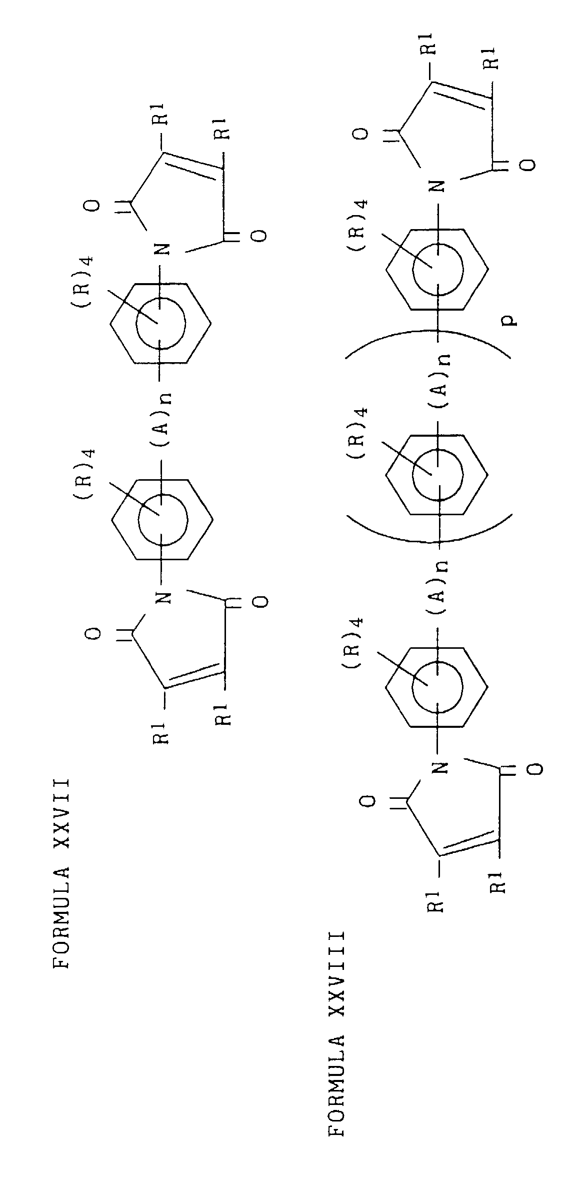 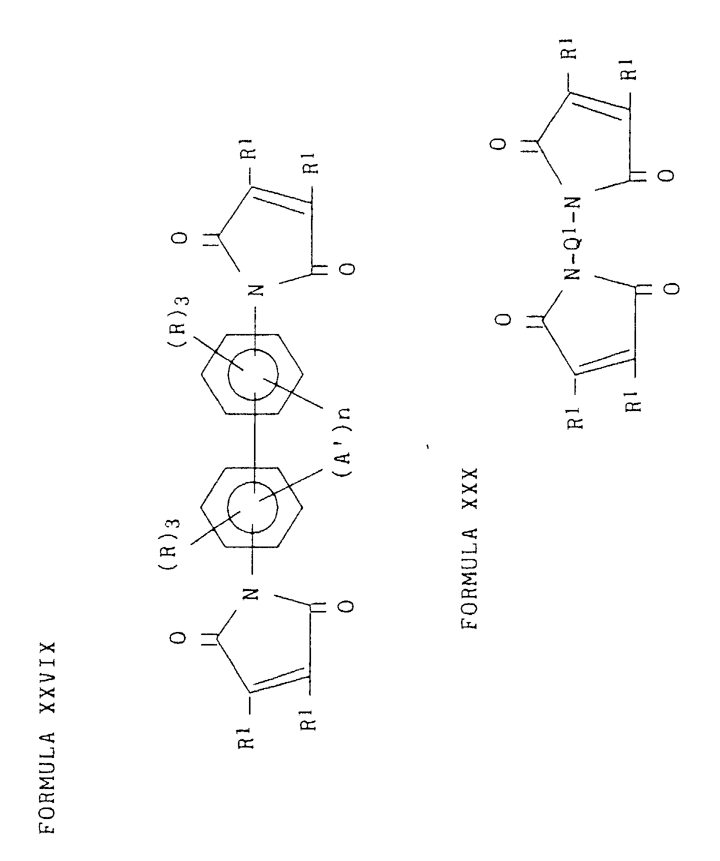 wherein A, A², A', A", R, R¹, R", m, n and p are as hereinbefore defined and Q¹ is a divalent hydrocarbyl group having from 2 to 12 carbon atoms and may be linear or branched aliphatic, cycloaliphatic or polycycloaliphatic. The aromatic rings can also contain one or more heteroatoms selected from, for example, N, O or S.

Particularly suitable polymaleimides represented by formulas XXIII, XXIV, XXV, XXVI, XXVII, XXVIII, XXIX and XXX are, for example, N,N'-ethylenebismaleimide, N,N'-ethylenebis(2-methylmaleimide), N,N'-hexamethyl-enebismaleimide, N,N'-(oxydi-p-phenylene)bismaleimide, N,N'-(methylenedi-p-phenylene)maleimide, N,N'-(methyl-enedi-p-phenylene)bis(2-methylmaleimide), N,N'-(thio-di-p-phenylene)bismaleimide, N,N'-(sulfonyldi-m-phenylene)bismaleimide, N,N'-(isopropylidenedi-p-phenylene)-bismaleimide, polymethylene polyphenylene polymaleimides, the bismaleimide of 4,4'-diaminostilbene and the bismaleimide of 4,4'-diaminobenzanilide.

The polymaleimides can be prepared by reacting a stoichiometric quantity of a maleic anhydride per amine group with a polyamine in the presence of a suitable solvent, for example, aromatic hydrocarbons, chlorinated hydrocarbons or N,N-dimethylformamide. The polymaleamic acid resulting from reaction of a maleic anhydride and a polyamine may be isolated and dehydrated to the desired polymaleimide. Alternately, the reaction may be performed in a single continous step. Detailed procedures for preparing polymaleimides can be found in U.S. Patent Nos. 2,444,536; 2,462,835; and Journal of Polymer Science: Part A: Polymer Chemistry, Vol. 27, pages 375-388 (1989).

### POLYAMINES SUITABLE FOR USE IN THE CURABLE AND CURED COMPOSITIONS

Suitable polyamines which can be employed to prepare the polymerizable mixtures of the present invention, include those containing one or more of the mesogenic or rodlike structure(s) already described herein, as well as any of the other known polyamines which do not contain mesogenic or rodlike structures. Typical representatives of said polyamines free of mesogenic or rodlike structures include 1,4-diaminobutane, 1,6-hexanediamine, 1,12-diaminododecane, 2-methyl-4-ethyl-1,8-diaminooctane, 1,4-diaminocyclohexane, 4,4'-diaminodiphenyl methane and 1,4-diaminobenzene, tris(aminophenyl)methane, anilineformaldehyde condensation products.

### POLYPHENOLS SUITABLE FOR USE IN THE CURABLE AND CURED COMPOSITIONS

Suitable polyphenols which can be employed to prepare the polymerizable mixtures of the present invention, include those containing one or more of the mesogenic or rodlike structure(s) already described herein as well as any of the other known polyphenols which do not contain mesogenic or rodlike structures. Typical representatives of said polyphenols free of mesogenic or rodlike structures include resorcinol, 4,4'-sulfonyldiphenol, 4,4'-dihydroxydiphenyl oxide, tris(hydroxyphenyl)methane, and phenolformaldehyde condensation products.

### POLYMERIZABLE UNSATURATED MONOMERS SUITABLE FOR USE IN THE CURABLE AND CURED COMPOSITIONS

Suitable compounds containing one or more polymerizable ethylenically unsaturated group(s) which can be employed to prepare the polymerizable mixtures of the present invention include both those containing one or more mesogenic or rodlike structure(s) and those free of said structures.

Suitable polymerizable ethylenically unsaturated monomers containing one or more mesogenic or rodlike moieties are cataloged by Alexandre Blumstein in Liquid Crystalline Order in Polymers, published by Academic Press, New York (1978) on pages 105-140; Mesomorphic Order in Polymers and Polymerization in Liquid Crystalline Media published by American Chemical Society (ACS Symposium Series 74), Washington, D.C. (1978) on pages 56-70; and N. A. Plate and V. P. Shibaev in Comb-Shaped Polymers and Liquid Crystals published by Plenum Press, New York (1987) on pages 1-415; V. Percec, et. al., Polymer Bulletin, 17, pages 347-352 (1987); R. Duran and P. Gramain, Makromol. Chem., 188, pages 2001 - 2009 (1987); A.M. Mousa, et. al., Polymer Bulletin, 6, pages 485 - 492 (1982); H. Finkelmann, et. al., Makromol. Chem., 179, pages 829 - 832 (1978); M. Portugall, et. al., Makromol. Chem., 183, pages 2311-2321 (1982) and U.S. Patent Nos. 4,637,896 and 4,614,619. Suitable polymerizable ethylenically unsaturated monomers containing one or more mesogenic or rodlike moieties per molecule are represented by the formulas XXXI or XXXII:

FORMULA XXXI M-Q²

FORMULA XXXII M-(Q³)ₙ-R³-Q²

wherein n is as hereinbefore defined, M is a group containing two or more aromatic rings bridged by a rigid central linkage, R³ is a divalent hydrocarbon group having from one to 12 carbon atoms and may be linear, branched, cyclic, aromatic or a combination thereof and may be substituted with one or more inert groups for example, a methoxy group, or may contain one or more inert heteroatom containing linkages, such as an ether linkage; Q³ is -O-, -NR¹-, -S-, -O-CO-, -CO-O-, -NR¹-CO-, -CO-NR¹-, -CO-, -O-CO-O-, -S-CO-, -CO-S-, -NR¹-CO-O-, -O-CO-NR¹-, -NR¹-CO-NR¹- wherein R¹ is as hereinbefore defined ; and Q₂ is a polymerizable ethylenically unsaturated group. As a class, these monomers generally contain a -CH=CH₂, allyl, methallyl, propenyl, isopropenyl, acrylate or methacrylate group as the polymerizable ethylenically unsaturated group and a linear divalent aliphatic, aliphatic ether, aliphatic polyether, aliphatic thioether or cycloaliphatic flexible spacer connecting the polymerizable ethylenically unsaturated group and the mesogenic or rodlike group(s) through a heteroatom linkage. Typical mesogenic or rodlike groups include those wherein two or more aromatic rings are bridged by a rigid central linkage wherein said rigid central linkage is required to bridge the aromatic rings to provide at least about 80 percent para substitution. The aromatic rings can be inertly substituted, however, unsubstituted aromatic rings which maximize the molecular aspect ratio are preferred. Also preferred is a single inert substituent in the para position on the ring not connected to the polymerizable ethylenically unsaturated group (either directly or via a flexible spacer). This type of substituent can be used to enhance the molecular aspect ratio. Typical of these inert substituents are CH₃O-, Cl-, NO₂- and -C≡N. The aromatic rings can also contain one or more heteroatoms selected from, for example, N, O or S. Typical rigid central linkage groups for bridging the aromatic rings include, for example, a direct bond, -CR¹=CR¹-, -C≡C-, -N=N-, -CR¹=N-, -CR¹=N-N=CR¹-, -CR¹=CR¹-CO-, -O-CO-, -NR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 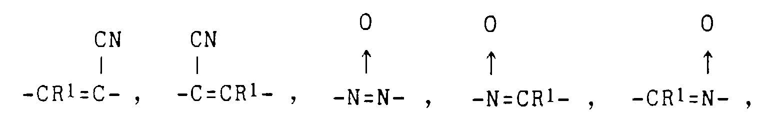 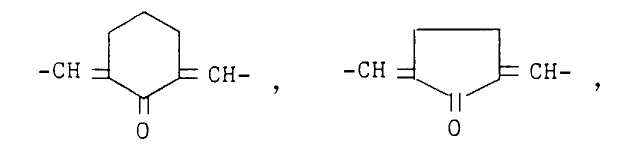 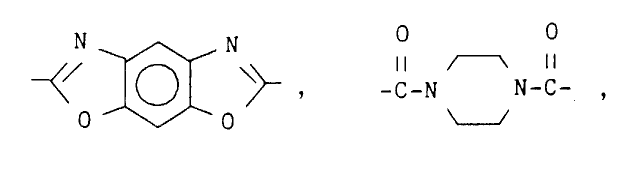 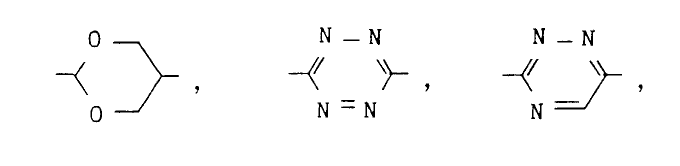 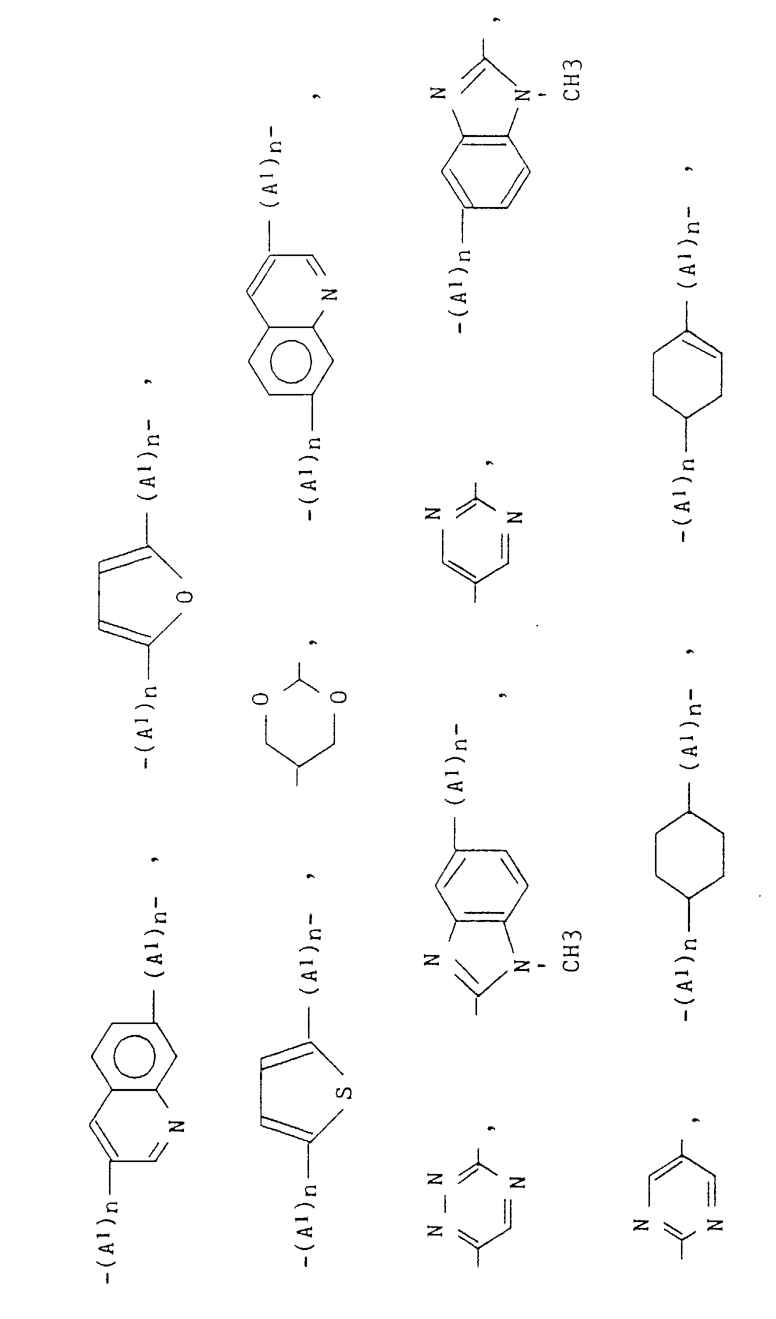 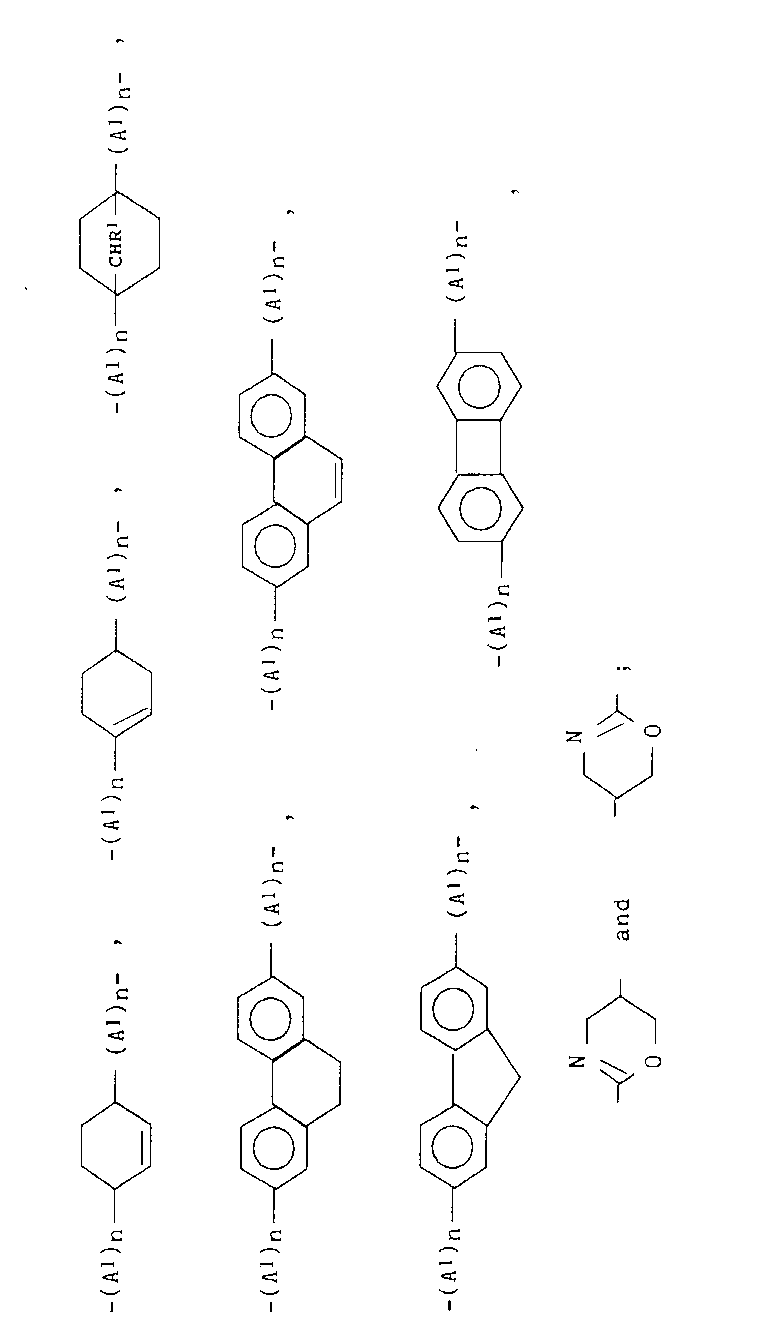 wherein R¹, A¹, n and n' are as hereinbefore defined. As is well known in the prior art, all or a part of the aromatic rings can be replaced with other promesogenic structures, for example, the trans-cyclohexane ring or a cholesterol group. Additionally, it is has been demonstrated in the prior art that efficacious mesogenic or rodlike containing polymerizable ethylenically unsaturated monomers can be prepared with omission of the flexible spacer between the polymerizable ethylenically unsaturated group and the mesogenic or rodlike group(s).

Generally, the ethylenically unsaturated monomers containing -CH=CH₂, acrylate, allyl, methallyl, propenyl, isopropenyl or methacrylate as the polymerizable vinyl group and a linear divalent hydrocarbon group connecting the vinyl group and the mesogenic or rodlike group through heteroatom containing functional groups between the hydrocarbon spacer and the mesogenic group are most preferred. Thus, a mesogenic group ether linked to a -CH₂-CH₂- which is in turn linked to provide a methacrylate ester, that is, 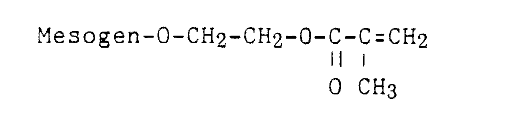 or a mesogenic group linked to a vinyl group, that is, 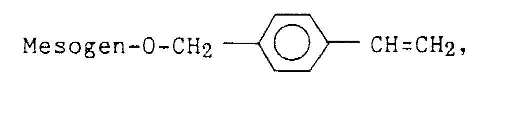 are examples of those species preferred as the ethylenically unsaturated monomer containing one or more mesogenic or rodlike moieties.

Particularly suitable ethylenically unsaturated monomers containing a mesogenic or rodlike moiety include, for example, 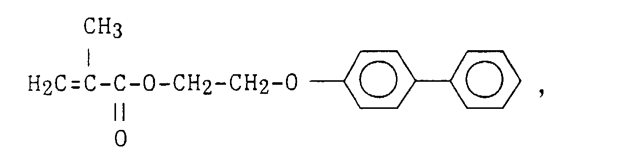 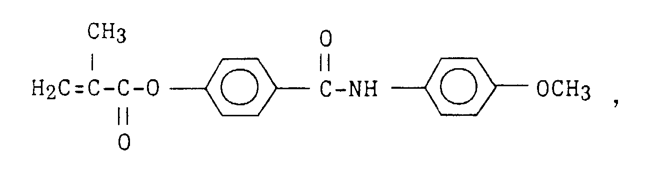 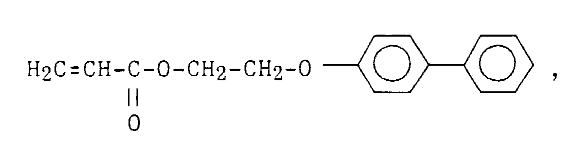 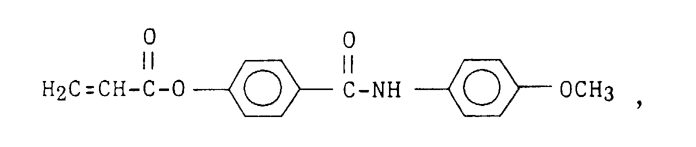 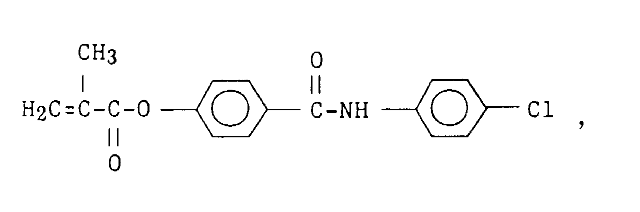 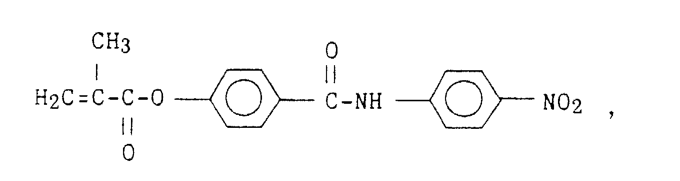 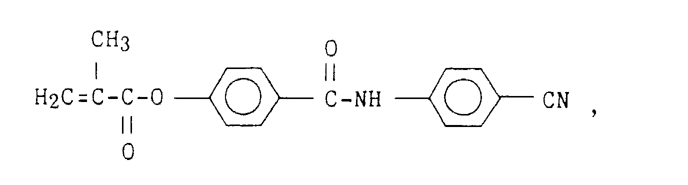 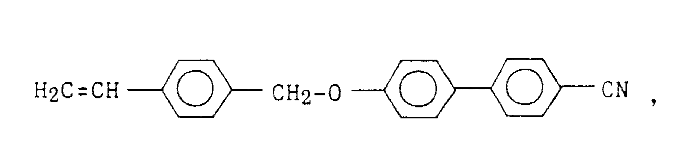 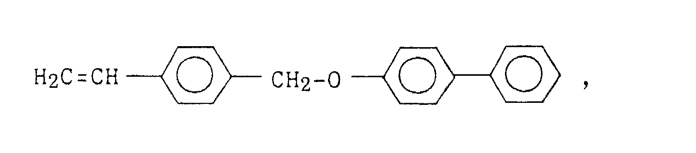 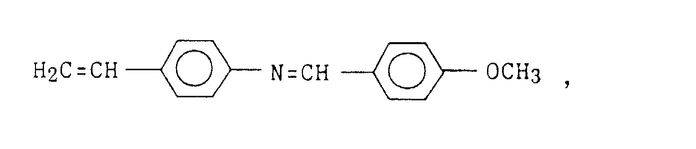 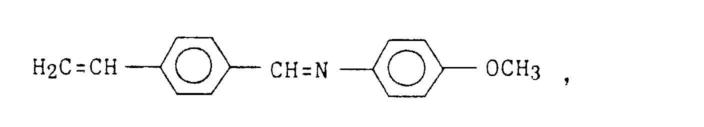 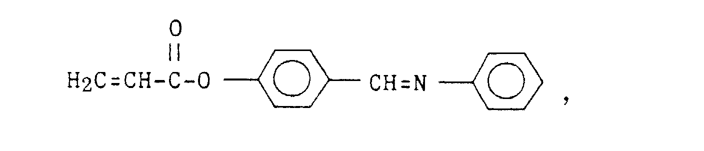 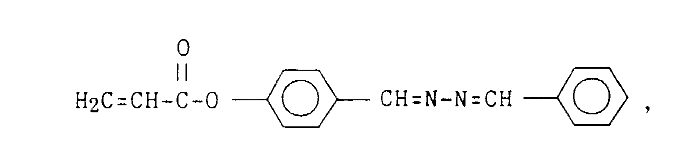 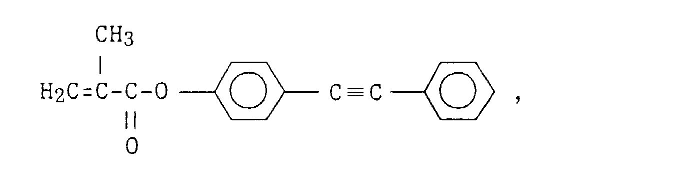 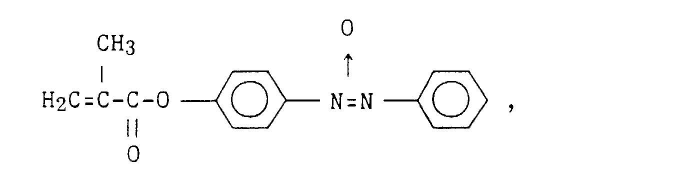 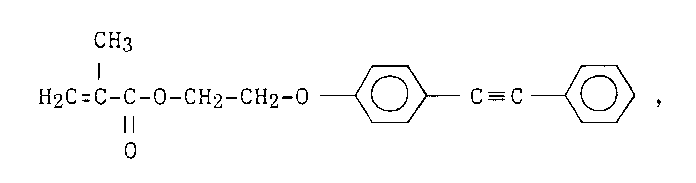 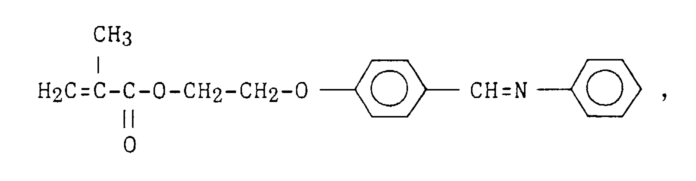 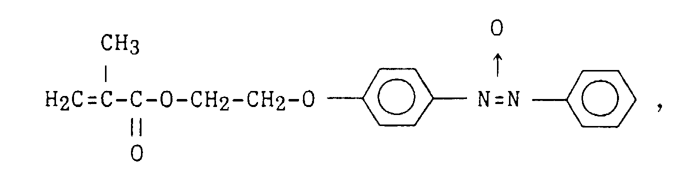 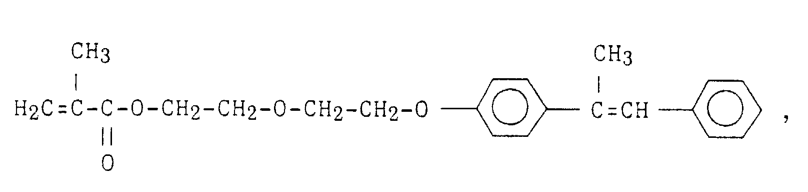 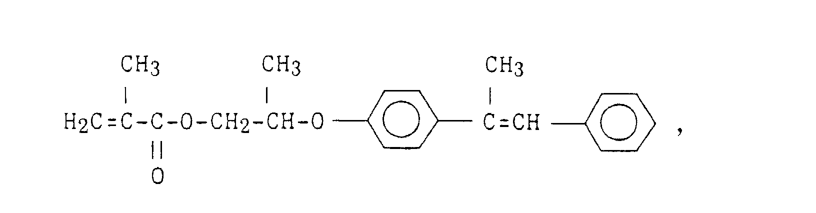 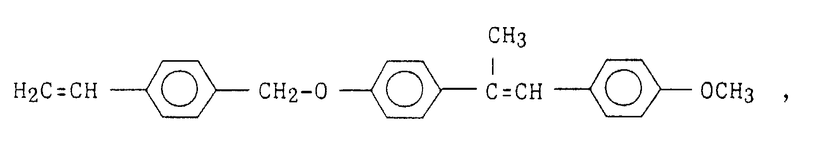 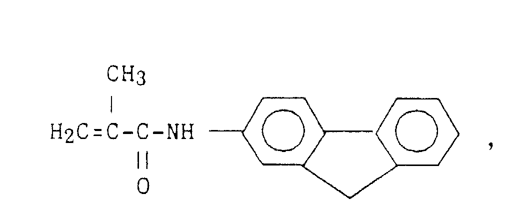 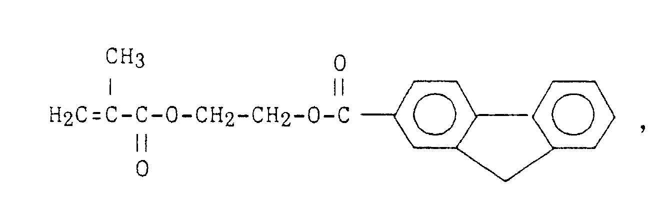 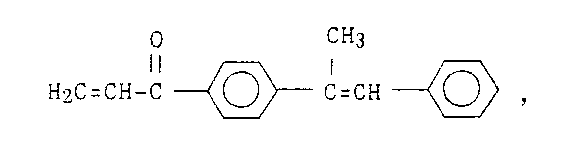 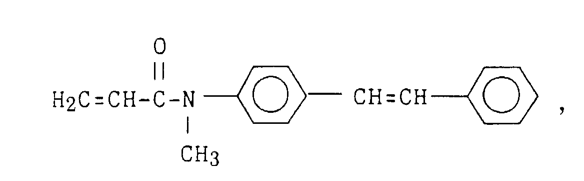 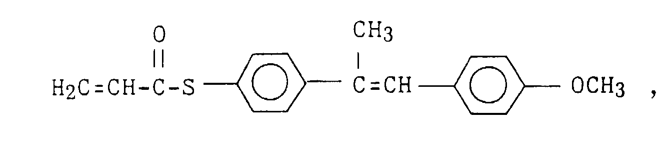 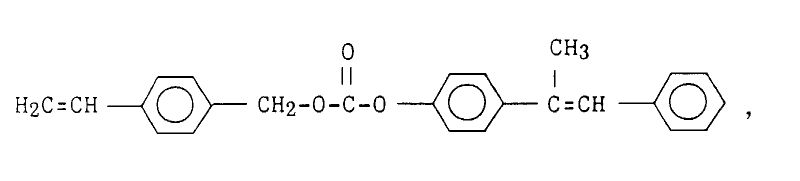 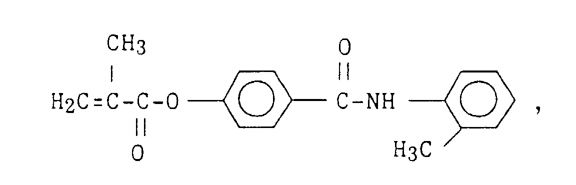 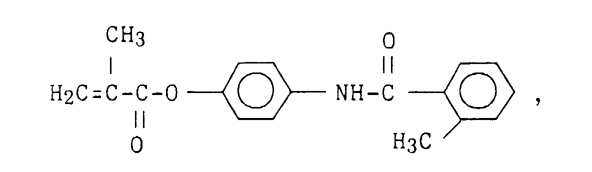 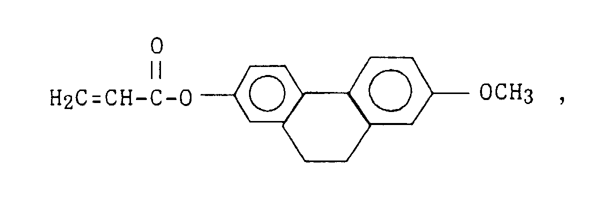 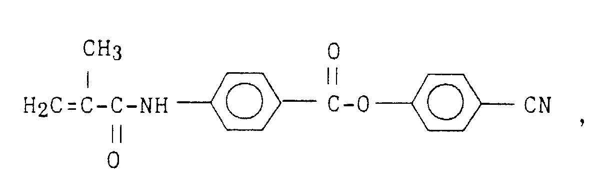 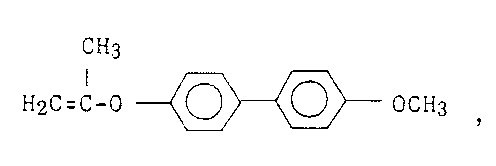 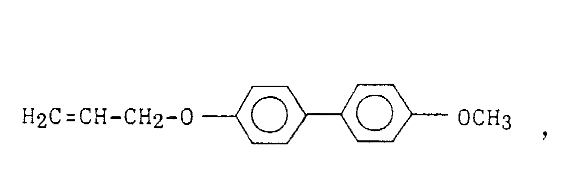 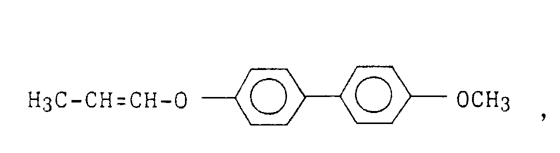 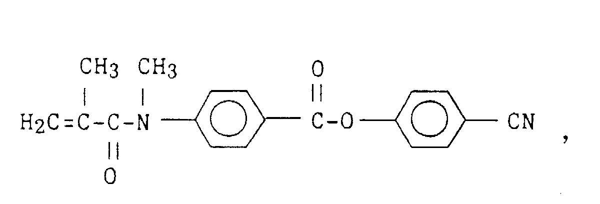 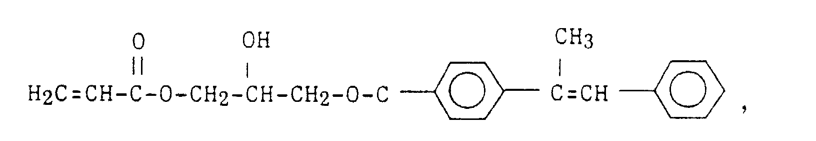 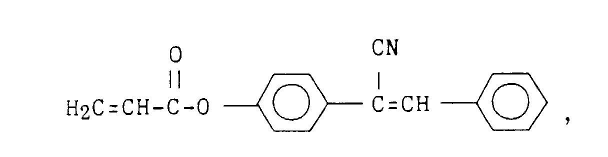 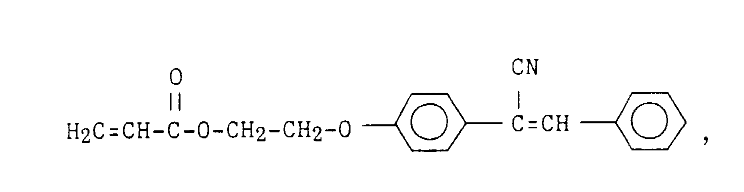 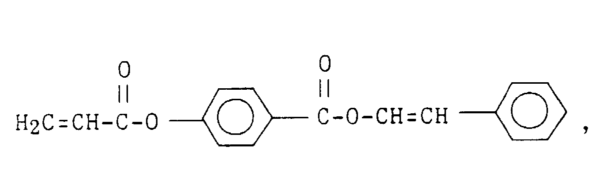 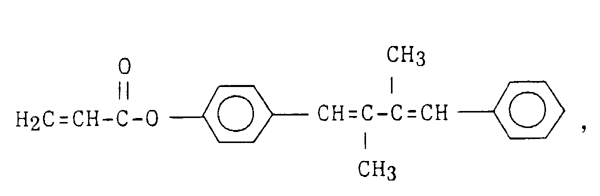 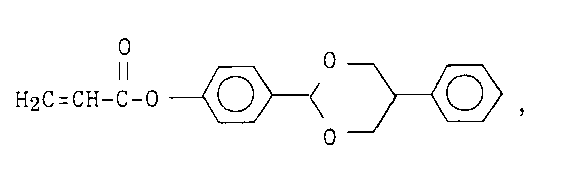 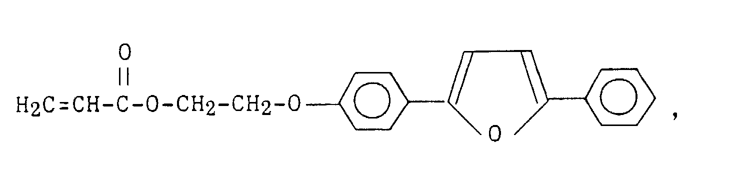 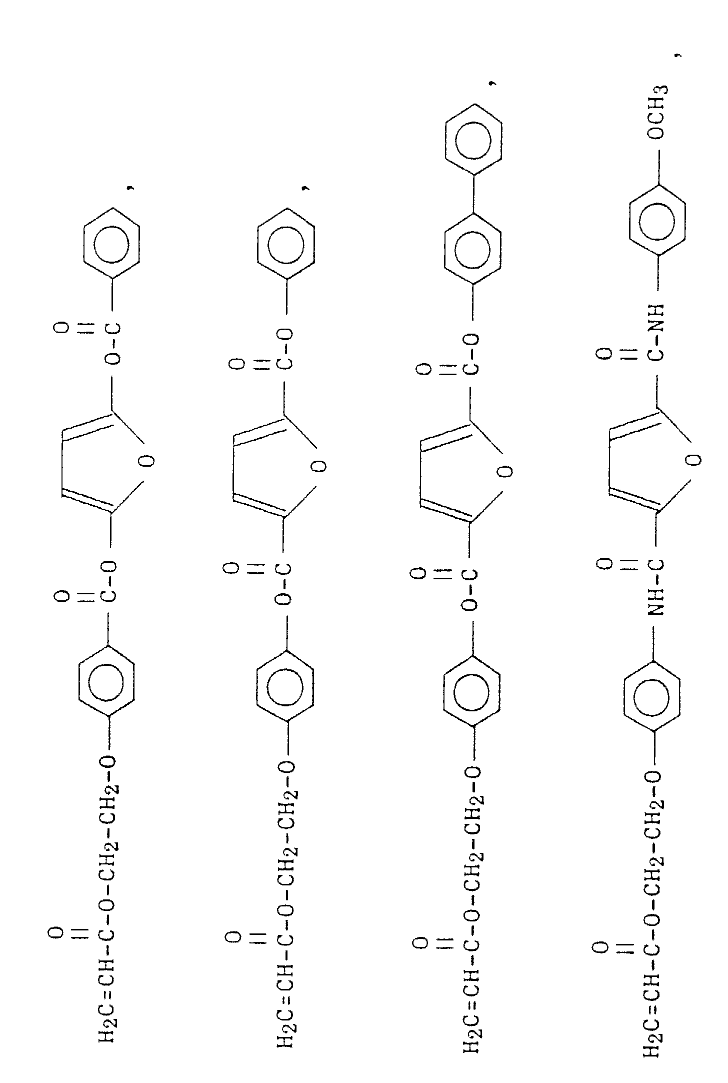 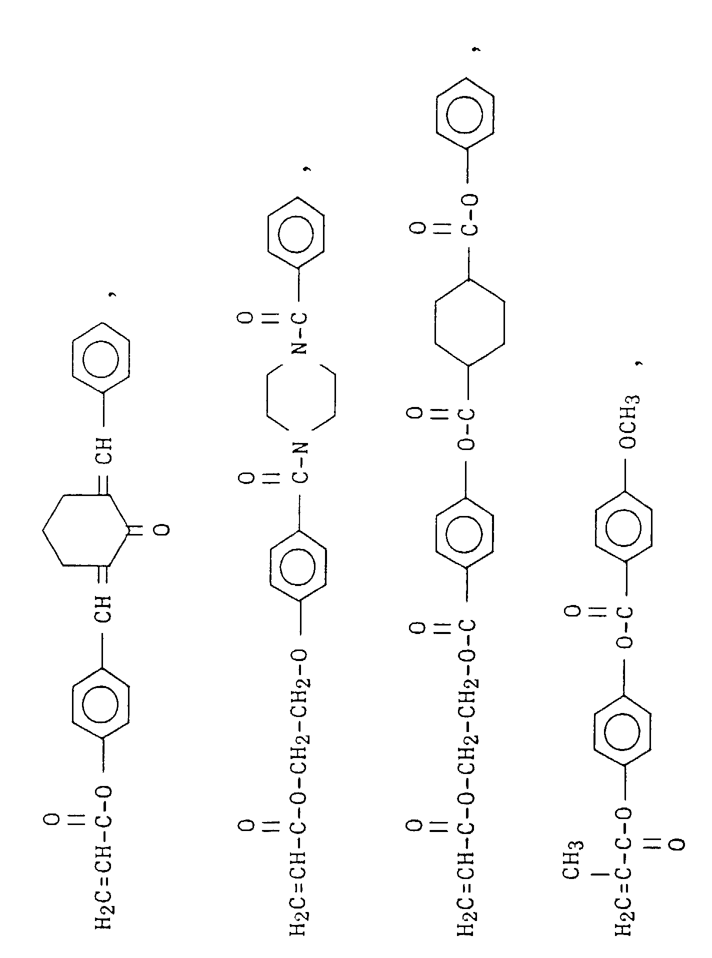 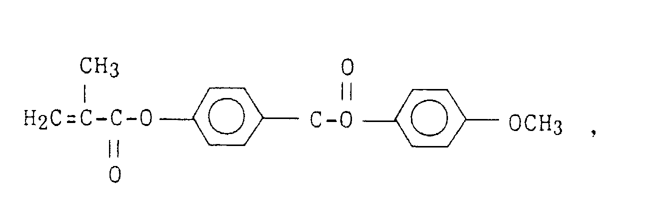 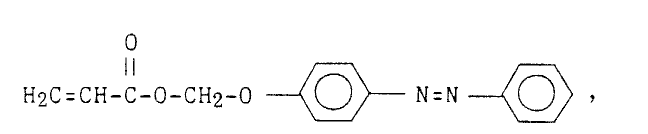 and any combination thereof.

Suitable polymerizable ethylenically unsaturated monomers which do not contain mesogenic or rodlike structures can be selected from the many known classes of polymerizable vinyl monomers. Suitable such monomers include, for example, the vinyl aromatic compounds represented by the following formula XXXIII: 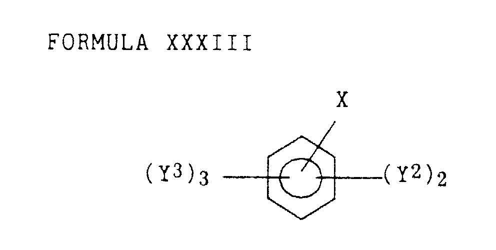 wherein Y² is independently hydrogen, a hydrocarbyl or hydrocarbyloxy group having from 1 to 5 carbon atoms, a vinyl group, an allyl group, a methallyl group, a propenyl group, a isopropenyl group, a nitro group, a nitrile group, a halogen, such as chlorine or bromine or fluorine, or a -CO-R¹ group; each Y³ is independently hydrogen, a hydrocarbyl or hydrocarbyloxy group having from 1 to 5 carbon atoms, or a halogen, such as chlorine or bromine or fluorine and X is 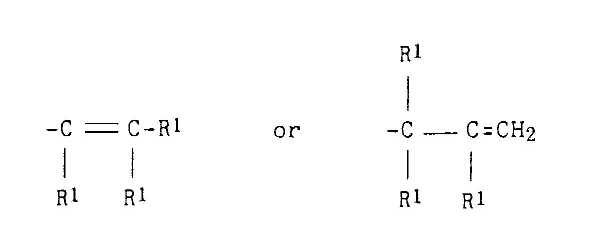 wherein R¹ is as hereinbefore defined;
or the acrylate (methacrylate) compounds represented by the following formula XXXIV: 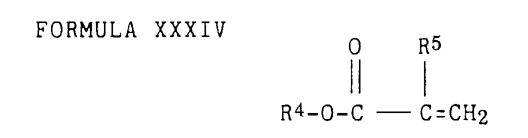 wherein R⁴ is a hydrocarbyl group having from 2 to 25 carbon atoms and may be branched, cyclic, polycyclic, saturated or unsaturated and R⁵ is hydrogen or a methyl group.

Typical polymerizable unsaturated monomers represented by formula XXXIII include, for example, styrene, alpha-methylstyrene, o-, m-, p-chlorostyrene; o-, m-, p-bromostyrene; o-, m-, p-tert-butylstyrene; o-, m-, p-methylstyrene; o-, m-, p-methoxystyrene; divinylbenzenes, trivinylbenzenes, o-, m-, p-isopropenylstyrene; o-, m-, p-allylstyrene; o-, m-, p-methallylstyrene; and allylbenzene, methallylbenzene, and diallylbenzenes.

Typical acrylate (methacrylate) esters represented by formula XXXIV include, for example, ethyl acrylate, n-butyl acrylate, n-butyl methacrylate, sec-butyl acrylate, 2-ethylhexyl acrylate, 2-ethylhexyl methacrylate, n-dodecyl acrylate, cyclohexyl acrylate, methylcyclohexyl acrylate, norbornyl acrylate, dicyclopentadiene acrylate and methyldicyclopentadiene acrylate.

Other suitable monomers include the acidic monomers, such as acrylic and methacrylic acid; the amide monomers, such as acrylamide and N-methylacrylamide; the allyl monomers, such as diallylphthalate, triallylisocyanurate, diallylmaleate and dimethallylfumarate; the vinyl halides, such as vinyl chloride and vinyl bromide; the vinyl esters, such as vinyl acetate; the vinyl di and polycyclic aromatics, such as vinyl naphthalene; the vinyl nitriles, such as acrylonitrile; and the hydroxyalkyl acrylates and methacrylates, such as 2-hydroxyethyl acrylate.

### COMPOUNDS CONTAINING BOTH A CYANATE OR CYANAMIDE GROUP AND A POLYMERIZABLE ETHYLENICALLY UNSATURATED GROUP FOR USE IN THE CURABLE AND CURED COMPOSITIONS

Suitable compounds which contain both a cyanate or cyanamide group and a polymerizable ethylenically unsaturated group in the same molecule that can be used to prepare the polymerizable mixtures of the present invention include, for example, those represented by the following formula XXXV: 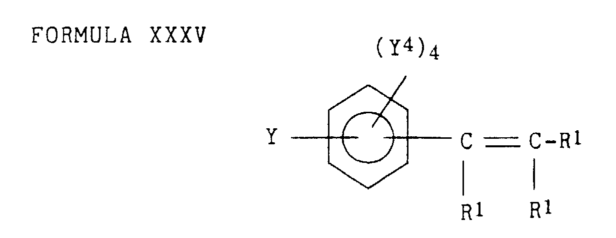 wherein each Y and R¹ are as hereinbefore defined, Y⁴ is independently hydrogen, a hydrocarbyl or hydrocarbyloxy group having from 1 to 12 carbon atoms, a nitro group, a nitrile group, a halogen, such as chlorine or bromine or fluorine, or a -CO-R¹ group; or a compound represented by the following formula XXXVI: 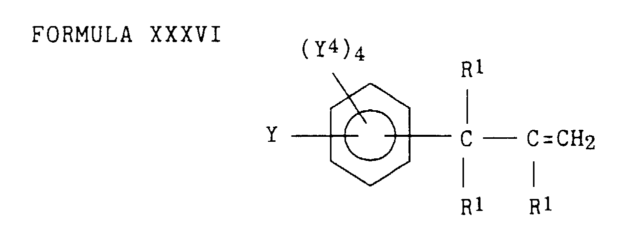 wherein each Y, Y⁴ and R¹ are as hereinbefore defined.

Suitable compounds which contain a cyanate or cyanamide group and a polymerizable ethylenically unsaturated group in the same molecule represented by formulas XXXV and XXXVI include, for example, o-, m-, p-isopropenylphenyl cyanate; o-, m-, p-vinylphenyl cyanate; methyl-p-isopropenylphenyl cyanates; 3-chloro-4-isopropenylphenyl cyanate; o-, m-, p-propenylphenyl cyanate; o-, m-, p-allylphenyl cyanate; and o-, m-, p-methallylphenyl cyanate. Some of the alkenylphenol precursors to the alkenylphenyl cyanates represented by formula XXXV, notably the vinylphenols, have a tendency to dimerize or oligomerize thus leading to poly(alkenylphenyl)cyanates. It is most preferred that the alkenylphenyl cyanate be substantially free of dimeric and/or oligomeric components, although it is operable to use an alkenylphenyl cyanate containing substantial (up to 90 percent by weight) dimeric and/or oligomeric components. A specific preparation of p-isopropenylphenyl cyanate is taught in Example 1 of U.S. Patent No. 4,559,399.

### COMPOUNDS CONTAINING BOTH A 1,2-EPOXIDE GROUP AND A POLYMERIZABLE ETHYLENICALLY UNSATURATED GROUP FOR USE IN THE CURABLE AND CURED COMPOSITIONS

Suitable compounds which contain both a 1,2-epoxide group and a polymerizable ethylenically unsaturated group in the same molecule that can be used to prepare the polymerizable mixtures of the present invention include, for example, those represented by the following formulas XXXVII or XXXVIII: 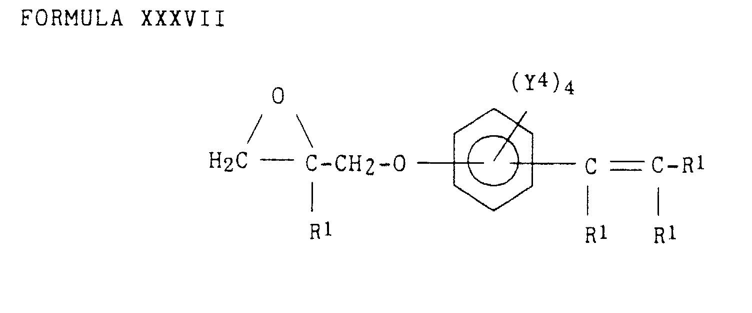 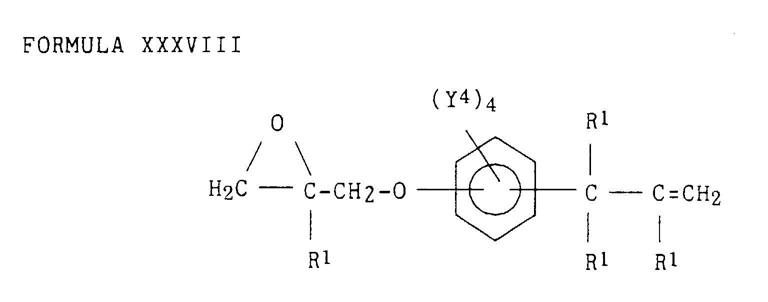 wherein each Y⁴ and R¹ are as hereinbefore defined.

Suitable compounds which contain a 1,2-epoxide group and a polymerizable ethylenically unsaturated group in the same molecule represented by formulas XXXVII and XXXVIII include, for example, o-, m-, p-isopropenylphenyl glycidyl ether; o-, m-, p-vinylphenyl glycidyl ether; methyl-p-isopropenylphenyl glycidyl ethers; 3-chloro-4-isopropenylphenyl glycidyl ether; o-, m-, p-propenylphenyl glycidyl ether; o-, m-, p-allylphenyl glycidyl ether; and o-, m-, p-methallyphenyl glycidyl ether. Some of the alkenylphenol precursors to the alkenylphenyl glycidyl ethers represented by formula XXXVII, notably the vinylphenols, have a tendency to dimerize or oligomerize thus leading to poly(alkenylphenyl)glycidyl ethers. It is most preferred that the alkenylphenyl glycidyl ether be substantially free of dimeric and/or oligomeric components, although it is operable to use an alkenylphenyl glycidyl ether containing substantial (up to 90 percent by weight) dimeric and/or oligomeric components. The compounds which contain a 1,2-epoxide group and a polymerizable ethylenically unsaturated group in the same molecule are prepared using the corresponding phenol containing a polymerizable ethylenically unsaturated group and the hereinbefore described chemistry used in the preparation of epoxy resins.

### COMPOUNDS CONTAINING BOTH A MALEIMIDE GROUP AND A CYANATE GROUP SUITABLE FOR USE IN THE CURABLE AND CURED COMPOSITIONS

Suitable compounds which contain both a maleimide group and a cyanate group in the same molecule that can be used to prepare the polymerizable mixtures of the present invention include, for example, those represented by the following formulas XXXIX, XXXX or XXXXI: 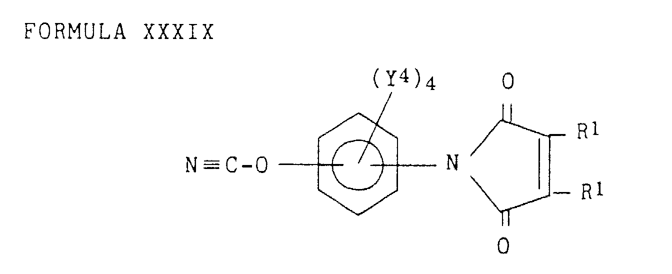 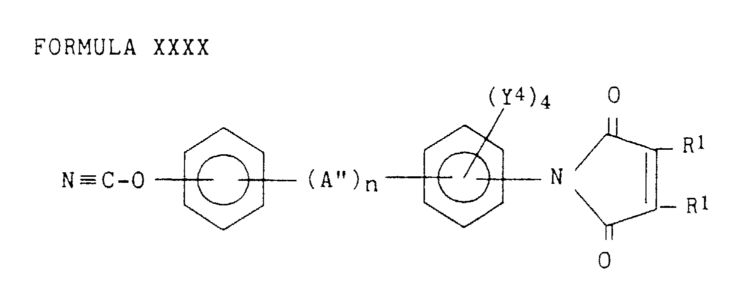 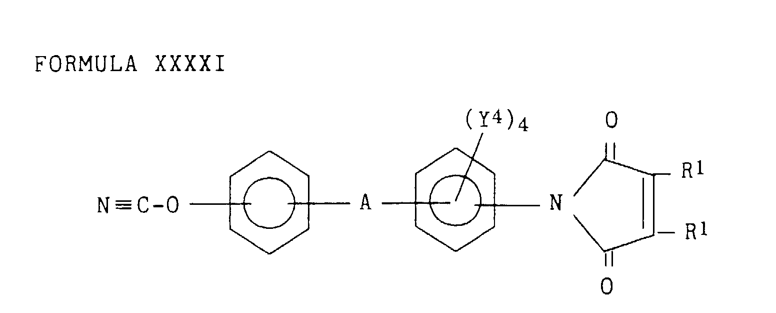 wherein each Y⁴, R¹, A, A" and n are as hereinbefore defined.

Suitable compounds which contain a maleimide group and a cyanate group in the same molecule represented by formulas XXXIX, XXXX and XXXXI include, for example, 4-(1-(3-(2,5-dihydro-2,5-dioxo-1H-pyrrol-1-yl)phenyl)-1-methylethyl)phenyl cyanate; 4-(1-(4-(2,5-dihydro-2,5-dioxo-1H-pyrrol-1-yl)phenyl)-1-methylethyl)phenyl cyanate; 4-(1-(4-(2,5-dihydro-2,5-dioxo-1H-pyrrol-1-yl)phenyl)ethyl)phenyl cyanate; 4-(4-(2,5-dihydro-2,5-dioxo-1H-pyrrol-1-yl)phenoxy)phenyl cyanate; 4-((4-(2,5-dihydro-2,5-dioxo-1H-pyrrol-1-yl)phenyl)thio)phenyl cyanate; 4-(4-(2,5-dihydro-2,5-dioxo-1H-pyrrol-1-yl)benzoyl)phenyl cyanate; 4-((4-(2,5-dihydro-2,5-dioxo-1H-pyrrol-1-yl)phenyl)sulfonyl)phenyl cyanate; 4-(1-(4-(2,5-dihydro-3-methyl-2,5-dioxo-1H-pyrrol-1-yl)phenyl)-1-methylethyl)phenyl cyanate; 2,6-dibromo-4-(1-(3,5-dibromo-4-(2,5-dihydro-2,5-dioxo-1H-pyrrol-1-yl)phenyl)-1-methylethyl)phenylcyanate; 4-(2,5-dihydro-2,5-dioxo-1H-pyrrol-1-yl)phenyl cyanate; and 3-(2,5-dihydro-2,5-dioxo-1H-pyrrol-1-yl)phenyl cyanate. Preparation of compounds which contain a maleimide group and a cyanate group in the same molecule is taught in U.S. Patent No. 4,683,276.

### COMPOUNDS CONTAINING ONE CYANATE OR CYANAMIDE GROUP PER MOLECULE AND ONE OR MORE MESOGENIC OR RODLIKE MOIETIES WHICH CAN BE EMPLOYED IN THE CURABLE AND CURED COMPOSITIONS

Suitable compounds which contain one or more mesogenic or rodlike structure(s) and an average of one cyanate or cyanamide group per molecule that can be used to prepare the polymerizable mixtures of the present invention include, for example, those represented by the following formulas XXXXII, XXXXIII, XXXXIV, or XXXXV: 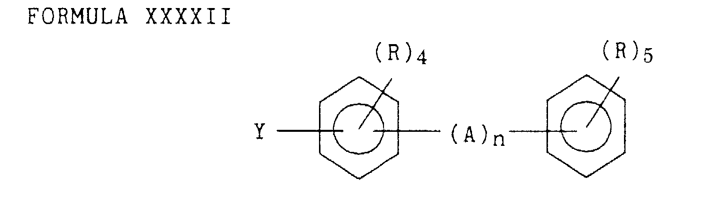 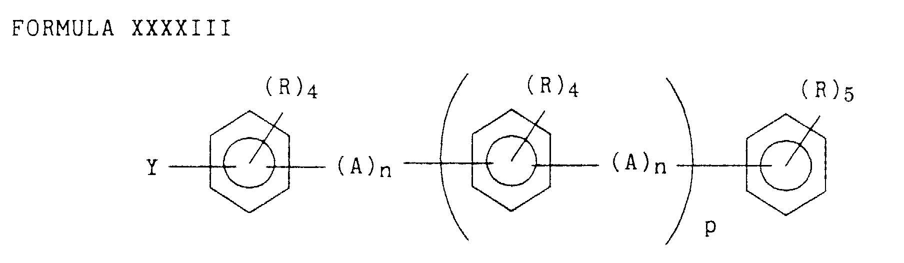 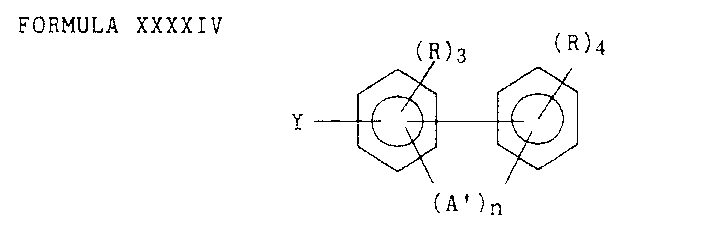 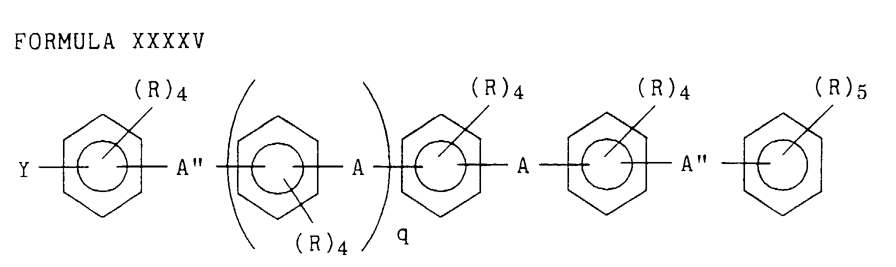 wherein at least 80 percent of the -A- linkages, the direct bond in Formula XXXXIV and the Y groups are in the para position with respect to each other and each A, A', A", R, Y and p are as hereinbefore defined and q has a value from zero to 6, preferably zero to 3.

Suitable compounds which contain one or more mesogenic or rodlike structure(s) and an average of one cyanate or cyanamide group per molecule represented by formulas XXXXII, XXXXIII, XXXXIV and XXXXV include, for example, the cyanates of 4-hydroxystilbene, 4-hydroxy-4'-methoxystilbene, 4-hydroxy-4'-chlorostilbene, 4-hydroxy-4'-nitrostilbene, 4-hydroxy-4'-cyanostilbene, 4-hydroxy-alpha-methylstilbene, 4-hydroxychalcone, 1-(4-hydroxyphenyl)-2-phenylacetylene, 1-(4-hydroxyphenyl)-2-phenylazomethine, 4-hydroxyphenylazobenzene, 4-hydroxyphenylazoxybenzene, 4-(4-hydroxyphenoxy)-diphenyl, 4-hydroxydiphenyl, 4-hydroxy-alpha-cyanostilbene, 4-hydroxy-alpha-ethylstilbene, 4-hydroxybenzanilide, 4-hydroxy-4'-methoxybenzanilide, 4-hydroxy-3,3',5,5'-tetramethyl-alpha-methylstilbene, N-methyl-4-hydroxybenzamide, N-phenyl-4-hydroxy-benzamide, 4-hydroxy-3,3',5,5'-tetrabromo-alpha-methylstilbene, 4-hydroxyphenylbenzoate, phenyl-4-hydroxybenzoate, the cyanamides of 4-aminostilbene, 4-amino-alpha-methylstilbene and 4-aminobenzanilide. The compounds which contain one or more mesogenic or rodlike structure(s) and an average of one cyanate or cyanamide group per molecule are prepared using the corresponding monophenol (monoamine) containing one or more mesogenic or rodlike structure(s) and the hereinbefore described chemistry used in the preparation of polycyanates (polycyanamides).

### METHOD FOR FORMING THE MIXTURES OF THE PRESENT INVENTION

The mixtures of the present invention can be prepared by directly combining one or more of the desired component(s) with one or more polycyanates or polycyanamides containing one or more mesogenic or rodlike structures or by addition of one or more of the desired components to the polycyanates or polycyanamides containing one or more mesogenic or rodlike structures in increments or stages. When a single component is to be added to the polycyanates or polycyanamides containing one or more mesogenic or rodlike structures, said component may be prepolymerized (B-staged) or fully homopolymerized, prior to the addition. Additionally, certain of said single components may be homopolymerized (interpolymerized) while dispersed in or mixed in with one or more polycyanates or polycyanamides containing one or more mesogenic or rodlike structures. When two or more components are to be added to the polycyanates or polycyanamides containing one or more mesogenic or rodlike structures, said components may be partially or totally copolymerized or reacted together, prior to the addition. Additionally, when two or more components are to be added to the polycyanates or polycyanamides containing one or more mesogenic or rodlike structures, one component may be prepolymerized or fully homopolymerized in the presence of the other components, prior to the addition. It is understood that one or more catalysts or accelerators may be included where desired to facilitate the aforementioned copolymerization, interpolymerization, prepolymerization, homopolymerization or reaction of one or more specific components.

The mixtures of the thermosettable polycyanate or polycyanamide containing one or more mesogenic or rodlike moieties (component A) and the components B-1 to B-12 can contain any amounts of components A and B. Suitably, the components are employed in amounts such that the mixture contains from 1 to 99, preferably from 25 to 95, more preferably from 50 to 90 percent by weight of component A based on the combined weight of components A and B; and from 99 to 1, preferably from 75 to 5, more preferably from 50 to 10 percent by weight of component B based on the combined weight of components A and B.

### POLYMERIZATION OF THE POLYMERIZABLE MIXTURES

The mixtures of the present invention may be polymerized by heating from 50°C to 400°C, preferrably by heating from 100°C to 250°C, optionally in the presence of one or more suitable catalysts. In addition to the catalysts previously delineated for the polymerization of polycyanates or polycyanamides, whenever one or more polymaleimides, compounds containing one or more polymerizable ethylenically unsaturated group(s), compounds which simultaneously contain both a cyanate or cyanamide group and a polymerizable ethylenically unsaturated group, compounds which simultaneously contain both a 1,2-epoxide group and a polymerizable ethylenically unsaturated group or compounds which simultaneously contain both a maleimide group and a cyanate group are present, it is often desireable to utilize one or more free radical forming catalysts for the purpose of polymerizing all or a part of said unsaturated groups. Said free radical forming catalysts include the organic peroxides and hydroperoxides as well as the azo and diazo compounds. Preferred free radical forming catalysts include, for example, benzoylperoxide, t-butylhydroperoxide, t-butylperoxybenzoate, azobisisobutyronitrile, dicumylperoxide, di-tert-butylperoxide and cumene hydroperoxide. The quantity of catalyst used, if any, depends on, for example, the structure of the particular catalyst, the structure of the components used in the polymerizable mixture, the cure structure desired, the cure time and the cure temperature. Generally, catalyst concentrations of from 0.001 to 2 percent by weight are preferred. B-staging or prepolymerization of the mixtures of the present invention can be accomplished by using lower temperatures and/or shorter curing times. Curing of the thus formed B-staged (prepolymerized) mixture can then be accomplished at a later time or immediately following B-staging (prepolymerization) by increasing the temperature and/or curing time.

The polymerized mixtures possess a variety of curing structures which depend, in part, upon, for example, the amounts and types of individual components used to prepare said mixture, the sequence of component addition and procedure used to prepare said mixture, the amounts and types of catalysts, if any, employed, the reaction times and temperatures.

Mixtures of (A) one or more polycyanates containing one or more mesogenic or rodlike structures and no other moieties reactive with the cyanate group with (B-1) one or more polycyanates which do not contain mesogenic or rodlike structures and/or prepolymers of either of the aforementioned types of polycyanates cure via cyclotrimerization of the cyanate moieties to provide the polytriazine thermoset. As a preferred embodiment of the present invention, addition of about 10 percent or more of a polycyanate containing one or more mesogenic or rodlike structures to a polycyanate which does not contain mesogenic or rodlike structures followed by polymerizing or curing provides a polytriazine with improved mechanical properties over those of the polytriazine obtained from curing or polymerizing of only the polycyanate which does not contain mesogenic or rodlike structures.

Mixtures of (A) one or more polycyanates containing one or more mesogenic or rodlike structures and no other moieties reactive with the cyanate group with (B-2) one or more epoxy resins using a 1 to 1 mole ratio of cyanate groups to epoxide groups polymerize to produce a complex structure. Increasing the mole ratio of cyanate to epoxide groups can be done to increase the relative amount of triazine groups in the cured product. A preferred embodiment of the present invention is the polymerization product of a polycyanate containing one or more mesogenic or rodlike structures with an epoxy resin containing one or more mesogenic or rodlike structures. The aforementioned polymerized product provides improved properties, notably increased glass transition temperature, relative to the polymerization product of a polycyanate containing one or more mesogenic or rodlike structures with an epoxy resin which does not contain mesogenic or rodlike structures.

Mixtures of (A) one or more polycyanates containing one or more mesogenic or rodlike structures and no other moieties reactive with the cyanate group with (B-3) one or more polymaleimides can polymerize to produce a complex variety of structures including the triazine group resulting from cyclotrimerization of cyanate moieties 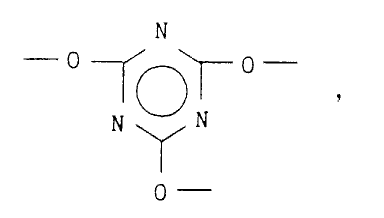 the maleimide group homopolymerization structure, 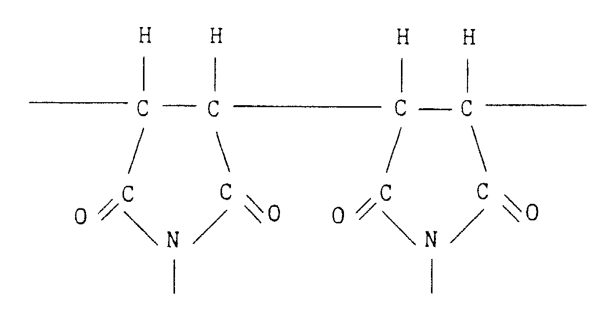 and cyanate group and maleimide group copolymerization structures for example, 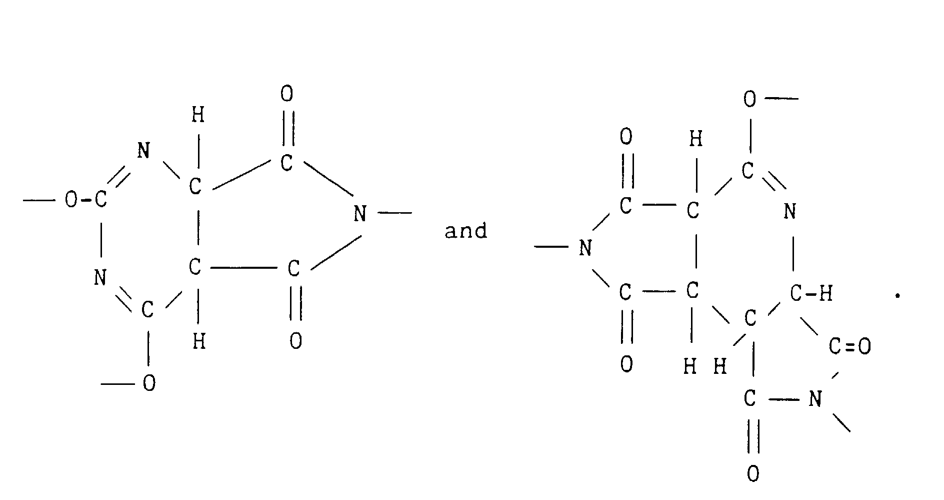 Changes in the mole ratio of cyanate groups and maleimide groups can be made to influence the composition of the cured product. Increasing the mole ratio of cyanate groups to maleimide groups to above 1 to 1 can be done to increase the relative amount of triazine groups in the polymerized product. A decrease in the mole ratio of cyanate groups to maleimide groups to below 1 to 1 favors an increase in the amount of maleimide group homopolymerization structure in the cured product. A preferred embodiment of the present invention is the polymerization product of a polycyanate containing one or more mesogenic or rodlike structures with a polymaleimide containing one or more mesogenic or rodlike structures. The aforementioned polymerized product provides improved mechanical properties relative to the polymerization product of a polycyanate containing one or more mesogenic or rodlike structures with a polymaleimide which does not contain mesogenic or rodlike structures. Methods for the copolymerization of polycyanates which do not contain mesogenic or rodlike structures with polymaleimides are taught by U.S. Patent Nos. 4,469,859; 4,404,330; 4,396,745; 4,383,903; 4,373,086; 4,371,689; 4,369,304; 4,287,014 and 4,110,364.

Mixtures of (A) one or more polycyanates containing one or more mesogenic or rodlike structures and no other moieties reactive with the cyanate group with (B-4) one or more polyamines using a 1 to 1 mole ratio of cyanate groups to amine groups polymerize to producepoly(iminocarbamic acid esters). Increasing the mole ratio of cyanate to amine groups can be done to induce formation of triazine groups in the polymerized or cured product. Methods for the reaction of polyamino compounds with polycyanate compounds to produce poly(iminocarbamic acid esters) are taught by U.S. Patent No. 3,502,617.

Mixtures of (A) one or more polycyanates containing one or more mesogenic or rodlike structures and no other moieties reactive with the cyanate group with (B-5) one or more polyphenols using a 1 to 1 mole ratio of cyanate groups to phenolic hydroxyl groups polymerize to produce poly(iminocarbonic esters). Increasing the mole ratio of cyanate to phenolic hydroxyl groups can be done to induce formation of triazine groups in the polymerized or cured product. Methods for the reaction of polyphenol compounds with polycyanate compounds to produce poly(iminocarbonic acid esters) are taught by U.S. Patent No. 3,491,060.

Mixtures of (A) one or more polycyanates containing one or more mesogenic or rodlike structure(s) and no other moieties reactive with the cyanate group with (B-6) one or more polymerizable ethylenically unsaturated compounds can polymerize to produce a complex variety of structures including the triazine group resulting from cyclotrimerization of cyanate moieties, structure from the polymerization of the polymerizable ethylenically unsaturated compound(s) and cyanate group and polymerizable ethylenically unsaturated group copolymerization structures. A specific example of a structure arising from the copolymerization of cyanate groups with a vinyl aromatic group (styrene) is as follows: 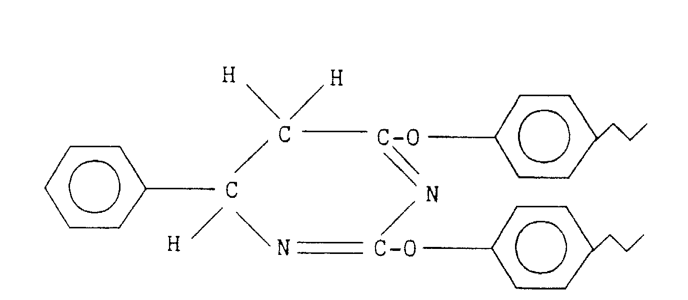 Changes in the mole ratio of cyanate groups and polymerizable ethylenically unsaturated groups can be made to influence the composition of the polymerized product. Increasing the mole ratio of cyanate groups to polymerizable ethylenically unsaturated groups to above 1 to 1 can be done to increase the relative amount of triazine groups in the polymerized product. A decrease in the mole ratio of cyanate groups to polymerizable ethylenically unsaturated groups to below 1 to 1 favors an increase in the amount of polymerizable ethylenically unsaturated group homopolymerization structure in the polymerized product. A preferred embodiment of the present invention is the polymerization product of a polycyanate containing one or more mesogenic or rodlike structures with a polymerizable ethylenically unsaturated compound containing one or more mesogenic or rodlike structures. The aforementioned polymerized product provides improved mechanical properties relative to the polymerization product of a polycyanate containing one or more mesogenic or rodlike structures with a polymerizable ethylenically unsaturated compound which does not contain mesogenic or rodlike structures. Methods for the copolymerization of a specific class of polycyanates which do not contain mesogenic or rodlike structures with vinyl aromatic monomers are taught by U.S. Patent No. 4,746,727.

Mixtures of (A) one or more polycyanates containing one or more mesogenic or rodlike structures and no other moieties reactive with the cyanate group with (B-7) one or more compounds which simultaneously contain both a cyanate group and a polymerizable ethylenically unsaturated group or (B-8) one or more compounds which simultaneously contain both a 1,2-epoxide group and a polymerizable ethylenically unsaturated group or (B-9) one or more compounds which simultaneously contain both a maleimide group and a cyanate group can polymerize to produce a complex variety of structures, including those previously mentioned for the various respective functional groups. As a specific example, a preferred mixture of the present invention consists of the polymerization product of (B-7) one or more compounds which simultaneously contain both a cyanate group and a polymerizable ethylenically unsaturated group with (B-6) one or more compounds containing one or more ethylenically unsaturated groups. This copolymer is either prepared in situ by free radical initiated polymerization of the (B-6) and (B-7) components in a molten or solvent solution of (A) one or more polycyanates containing one or more mesogenic or rodlike structures or it may be prepared separately then added to component (A). The resultant mixture is a polymer modified polycyanate which can be homopolymerized to provide the corresponding polymer modified polytriazine. In a further preferred embodiment of the present invention, if component (B-6) consists of one or more compounds containing ethylenically unsaturated groups and one or more mesogenic or rodlike structures, liquid crystal polymer modified polycyanates and polytriazines thereof can be produced via the aforementioned technique. Preparation of polymer modified cyanates which do not contain mesogenic or rodlike structures is taught by U.S. Patent No. 4,559,399.

Mixtures of (A) one or more polycyanates containing one or more mesogenic or rodlike structures with (B-10) one or more compounds which contain one or more mesogenic or rodlike structures per and only one cyanate (cyanamide) group per molecule can be cured via cyclotrimerization of the cyanate moieties to provide the polytriazine thermoset, providing that no other moieties reactive with cyanate groups are present in (A) or (B-10). Increasing the amount of the aforementioned cyanate compound containing an average of one cyanate group per molecule with respect to the amount of polycyanate can be used as a convenient method for lowering the crosslink density of the polytriazine product thereof.

### ORIENTATION OF THE POLYMERIZED PRODUCT CONTAINING MESOGENIC OR RODLIKE STRUCTURES

During processing and/or curing of the polycyanates or polycyanamides containing one or more mesogenic or rodlike structures or the mixtures containing said polycyanates or polycyanamides, electric or magnetic fields or shear stresses can be applied for the purpose of orienting the mesogenic or rodlike moieties contained or developed therein. As specific examples of these methods, Finkelmann, et. al., Macromol. Chem., 180, 803-806 (March, 1979), induced orientation in an electric field, of thermotropic methacrylate copolymers containing mesogenic side chain groups decoupled from the main chain via flexible spacers. Orientation in a magnetic field of mesogenic side chain groups decoupled from the main chain via flexible spacers has been demonstrated by Roth and Kruecke, Macromol. Chem., 187, 2655-2662 (November, 1986). Magnetic field induced orientation of mesogenic main chain containing polymers has been demonstrated by Moore, et. al., ACS Polymeric Material Sciences and Engineering, 52, 84-86 (April-May, 1985). Magnetic and electric field induced orientation of low molecular weight mesogenic compounds is discussed by W. Krigbaum in Polymer Liquid Crystals, pages 275-309 (1982), published by Academic Press, Inc. The use of shear to induce orientation is also discussed therein. When the curing is to be performed in an electric or magnetic field, it is frequently of value to conduct simple preliminary experiments that allow for balancing of cure kinetics versus induction of orientation under the particular experimental conditions being employed (i.e. catalyst(s) level being used, temperature used, inherent dielectric (diamagnetic) susceptibility of the specific mesogenic or rodlike structure(s) used, etc.). This is done recognizing the relatively greater ease of inducing orientation in low molecular weight materials versus polymeric materials containing mesogenic moieties.

In addition to orientation by electric or magnetic fields, the polycyanates or polycyanamides containing one or more mesogenic or rodlike structures or mixtures containing said polycyanates or polycyanamides can be oriented by shear forces which are induced by flow through dies, orifices and mold gates. A general discussion of orientation of thermotropic liquid crystalline polymers by this method is given by S.K. Garg and S. Kenig in High Modulus Polymers, pages 71-103 (1988) published by Marcel Dekker, Inc. For the mesomorphic polycyanates or mixtures containing said polycyanates, this shear orientation can conveniently be produced by or during processing methods such as injection molding, extrusion, pultrusion, filament winding, filming and prepreging.

### OTHER COMPONENTS WHICH CAN BE EMPLOYED

The polycyanates or polycyanamides containing one or more mesogenic or rodlike structures or mixtures containing said polycyanates or polycyanamides can be blended with other materials such as solvents or diluents, fillers including those comprising a liquid crystalline polymer, pigments, dyes, flow modifiers, thickeners, reinforcing agents, mold release agents, wetting agents, stabilizers, fire retardant agents, surfactants, low profile additives, shrinkage control agents, other resinous products and combinations thereof.

These additives are added in functionally equivalent amounts, for example, the pigments and/or dyes are added in quantities which will provide the composition with the desired color; however, they are suitably employed in amounts of from zero to 20, more suitably from 0.5 to 5, most suitably from 0.5 to 3 percent by weight based on the total weight of the composition.

Solvents or diluents which can be employed herein include, for example, hydrocarbons, ketones, aliphatic ethers, cyclic ethers, esters, chlorinated hydrocarbons and combinations thereof. Particularly suitable solvents or diluents include, for example, toluene, xylenes, methylethyl ketone, methylisobutyl ketone, methylamyl ketone, chloroform, acetone, perchloroethylene, methylene chloride, tetrahydrofuran, 1,4-dioxane, ethyl acetate, butyl acetate and combinations thereof.

The modifiers such as thickeners, flow modifiers, shrinkage control agents, low profile additives can be suitably employed in amounts from 0.05 to 15, more suitably from 0.1 to 10, most suitably from 0.1 to 5 percent by weight based on the total weight of the composition.

Reinforcing materials which can be employed herein include, for example, natural and synthetic fibers in the form of woven fabric, mats, monofilament, multifilament, unidirectional fibers, rovings, random fibers or filaments, inorganic fillers or whiskers, and hollow spheres. Suitable reinforceing materials include, for example, glass, ceramics, nylon, rayon, cotton, aramid, graphite, polyalkylene terephthlates, polyethylene, polypropylene, polyesters, carbon, boron, asbestos, combinations and hybrids thereof.

Suitable fillers which can be employed herein include, for example, inorganic oxides, ceramic microspheres, plastic microspheres, glass microspheres, inorganic whiskers, calcium carbonate, graphite powder, sand, metal powders and combinations thereof. The fillers can be employed in amounts from 0.1 to 95, more suitably from 5 to 80, most suitably from 10 to 50 percent by weight of the total composition.

### USES FOR THE COMPOSITIONS

The compositions of the present invention can be employed, for example, in the preparation of laminates, prepregs, composites, coatings, castings, pultruded products, filament wound products, films, molding and potting formulations and injection molded products.

The following examples are illustrative of the invention but are not to be construed as to limiting the scope thereof in any manner.

### Example 1

### A. Synthesis of 4,4'-Dihydroxy-alpha-methylstilbene

Phenol (376.44 grams, 4.0 moles), chloroacetone (205.62 grams, 2.0 moles as chloroacetone) and methylene chloride (300 grams) were added to a reactor and cooled to -10°C with stirring. The chloroacetone used was a technical grade containing 90 percent chloroacetone, 2.5 percent acetone, 6.5 percent 1,1-dichloroacetone and 1.0 percent 1,3-dichloroacetone. Concentrated sulfuric acid (196.16 grams, 2.0 mole) was added dropwise to the stirred solution over a one hour period and so as to maintain the reaction temperature at -10°C. After two hours of post reaction at the -10°C temperature, the viscous orange oil solution was mixed with 500 milliliters of iced deionized water. The oil solution was separated then washed with a second 500 milliliter portion of iced deionized water. After separation, the recovered oil solution was added to a 2 liter beaker along with 250 milliliters of ethanol and stirred to provide a solution. Deionized water (250 milliliters) was added to the stirred solution and heating commenced. As the temperature of the mixture increased, the stirred mixture began to clear. Each time clearing was observed, sufficient deionized water was added to induce cloudiness, followed by continuation of the mixing and heating. Once the temperature reached 70°C, a massive precipitation of white crystalline plates occurred and was followed by immediate coalesence of the precipitated product to an oil. The oil layer was recovered by decantation of the water layer and 250 milliliters of ethanol was added. Deionized water was again added to the stirred solution as heating commenced, in an amount sufficient to induce cloudiness each time clearing was observed. Once the temperature reached 90°C, a massive precipitation of white crystalline plates again occurred. At this time, stirring was stopped and the crystalline slurry, as well as the decanted water layer were both chilled to 5°C and held therein for 12 hours. The crystalline product was recovered by filtration, combined with 250 milliliters of deionized water then stirred with heating to 90°C. After cooling to 5°C, the crystalline product was recovered by filtration then dried in a vacuum oven at 100°C and 5mm Hg (665 Pa) to a constant weight of 243.3 grams. Proton magnetic resonance spectroscopy and infrared spectrophotometric analysis confirmed the product structure.

### B. Preparation of Dicyanate of 4,4'-Dihydroxy-alpha-methylstilbene

4,4'-Dihydroxy-alpha-methylstilbene (226.26 grams, 2.0 hydroxyl equivalents) prepared using the method of A above, cyanogen bromide (222.45 grams, 2.10 moles) and acetone (1200 milliliters) were added to a reactor and maintained under a nitrogen atmosphere with stirring. The stirred solution was cooled to -5°C, then triethylamine (203.39 grams, 2.01 moles) was added to the reactor over a 34 minute period and so as to maintain the reaction temperature at -5°C to -3°C. After completion of the triethylamine addition, the reactor was maintained at -5°C to -3°C for an additional 45 minutes followed by addition of the reactor contents to 1 gallon of deionized water. After 5 minutes, the water and product mixture was multiply extracted with three 400 milliliter volumes of methylene chloride. The combined methylene chloride extract was washed with 250 milliliters of 0.05 percent by weight aqueous hydrochloric acid followed by washing with two 500 milliliter portions of deionized water, then drying over anhydrous sodium sulfate. The dry methylene chloride extract was filtered and solvent removed by rotary evaporation under a vacuum for 60 minutes at 100°C. The dicyanate of 4,4'-dihydroxy-alpha-methylstilbene (272.5 grams) was recovered in 98.64 percent yield as a mass of light tan colored crystalline needles. Infrared spectrophotometric analysis of a film sample of the product confirmed the product structure (disappearance of phenolic hydroxyl absorbance, appearance of cyanate absorbance (2236 and 2270 cm⁻¹, sharp).

### C. Characterization of the Dicyanate of 4,4'-Dihydroxy-alpha-methylstilbene for Liquid Crystallinity

A portion (10.28 milligrams) of the dicyanate 4,4'-dihydroxy-alphamethylstilbene from B above was analyzed by differential scanning calorimetry using a heating rate of 10°C per minute and the indicated temperature ranges. The results are given in Table I.

**TABLE I**

| CYCLE DESIGNATION | OBSERVED TRANSITION TEMPERATURES (°C) MIDPOINT/RANGE | ENTHALPY (J/G) | COMMENTS |
|---|---|---|---|
| First heating (30 to 200°C) | 81/52-90 | --- | Single peak |
| First cooling (200 to 0°C) | 42/56-28 | --- | Single peak |
| Second heating (0 to 200°C) | 70.8/50-74 | 26.52 | Two resolved peaks |
| | 79.4/74-88 | 17.57 | |
| Second cooling (200 to -50°C) | 42/56-26 | 53.58 | Single peak |
| Third heating (-50 to 150°C) | unchanged from second heating | unchanged from second heating | unchanged from second heating |

Analysis of the dicyanate via cross polarized light microscopy was completed using a microscope equipped with a programmable hot stage using a heating rate of 10°C per minute and 35X magnification. The results are given in Table II.

**TABLE II**

| CYCLE DESIGNATION | OBSERVED TRANSITION TEMPERATURES (°C) | COMMENTS |
|---|---|---|
| First Heating | 25 | Immobile crystals. |
| | 67 | First fluidity noted. |
| | 76 | Crystals moving in fluid, crystals are restructuring. |
| | 82 | Isotropization complete. |
| First Cooling | 61 | First crystallization noted. |
| | 58 | Immobile crystalline mass. |
| Second Heating | 25 | Immobile crystals. |
| | 67 | First fluidity noted. |
| | 76 | Crystals moving in fluid, crystals are restructuring. |
| | 82 | Isotropization complete. |

### D. Preparation of a Cured Polytriazine Casting from the Dicyanate of 4,4'-Dihydroxy-alpha-methylstilbene

A 250.0 gram portion of the dicyanate of 4,4'-dihydroxy-alpha-methylstilbene from B above was heated to 100°C to form a solution, cooled to 50°C, then 0.25 gram of cobalt naphthenate (6.0 percent active) was added and mixed in. This solution was reheated to 100°C, filtered, poured into a 1/8 inch (3.17 mm) mold, then placed in an oven and maintained at 125°C for 2 hours, 177°C for 4 hours, 200°C for 4 hours then 250°C for 2 hours. The transparent, light amber colored casting was demolded and used to prepare test pieces for tensile and flexural strength and modulus, tensile elongation and average Barcol hardness (934-1 scale). Mechanical properties of the tensile and flexural test pieces were determined using an Instron machine with standard test methods (ASTM D 638 and D 790). The results are reported in Table III.

### COMPARATIVE EXPERIMENT A

### A. Preparation of Bisphenol A Dicyanate

4,4'-Isopropylidene diphenol (456.60 grams, 4.0 hydroxyl equivalents), cyanogen bromide (444.91 grams, 4.20 moles) and acetone (1100 milliliters) were added to a reactor and maintained under a nitrogen atmosphere with stirring. The stirred solution was cooled to -5°C, then triethylamine (406.82 grams, 4.02 moles) was added over a 60 minute period and so as to maintain the reaction temperature at -5°C to -3°C. After completion of the triethylamine addition, the reactor was maintained at -5°C to -3°C for an additional 25 minutes followed by addition of the reactor contents to 1.5 gallons of deionized water. After 5 minutes, the water and product mixture was multiply extracted with three 500 milliliter volumes of methylene chloride. The combined methylene chloride extract was washed with 500 milliliters of 0.05 percent by weight aqueous hydrochloric acid followed by washing with 500 milliliters of deionized water, then drying over anhydrous sodium sulfate. The dry methylene chloride extract was filtered and solvent removed by rotary evaporation under vacuum for 60 minutes at 100°C. The bisphenol A dicyanate (545.8 grams) was recovered in 98.1 percent yield as a tan colored crystalline solid. Infrared spectrophotometric analysis of a film sample of the product confirmed the product structure (disappearance of phenolic hydroxyl absorbance, appearance of cyanate absorbance).

### B. Preparation of a Cured Polytriazine Casting from Bisphenol A Dicyanate

A 200.0 gram portion of bisphenol A dicyanate prepared using the method of A above was heated to 100°C to form a solution, cooled to 50°C, then 0.20 gram of cobalt naphthenate (6.0 percent active) was added and mixed in. This solution was reheated to 100°C, filtered, poured into a 1/8 inch (3.17 mm) mold, then placed in an oven and maintained at 125°C for 2 hours, 177°C for 4 hours, 200°C for 4 hours, then 250°C for 2 hours. The transparent, light amber colored casting was demolded and used to prepare test pieces which were tested using the method of Example 1-D. The results are reported in Table III.

**TABLE III**

| MECHANICAL PROPERTY | EXAMPLE 1-D | COMPARATIVE EXPERIMENT A-B |
|---|---|---|
| Barcol Hardness | 51 | 48 |
| Tensile Strength (psi) | 15,010 | 13,080 |
| Tensile Modulus (psi) | 549,301 | 510,336 |
| Elongation (%) | 3.87 | 3.26 |
| Flexural Strength (psi) | 24,442 | 19,176 |
| Flexural Modulus (psi) | 568,556 | 555,138 |

Examination of a portion of the polytriazine casting of Example 1-D using cross polarized light microscopy revealed birefringence. By way of contrast, no birefringence was observed in the casting of Comparative Experiment A-B.

### Example 2

### Differential Scanning Calorimetry

A portion (8.0 milligrams) of the dicyanate of 4,4'-dihydroxy-alpha-methylstilbene catalyzed with cobalt naphthenate from Example 1-D above was analyzed by differential scanning calorimetry (DSC). A first heating from 25 to 325°C was completed at a rate of 10°C per minute under a stream of nitrogen flowing at 35 cubic centimeters per minute resulting in the exothermic curing of the dicyanate. Similarly, a second heating was completed, with the glass transition temperature for the cured polytriazine determined from the data of this heating. The results are reported in Table IV.

### COMPARATIVE EXPERIMENT B

The method of Example 2 was repeated using a portion (7.7 milligrams) of bisphenol A dicyanate catalyzed with cobalt naphthenate from Comparative Experiment A-B. The results are reported in Table IV.

**TABLE IV**

| SAMPLE DESIGNATION | GLASS TRANSITION TEMPERATURE | | |
|---|---|---|---|
| | ONSET (°C) | MIDPOINT (°C) | END (°C) |
| Example 1-D | 263.1 | 288.2 | 308.1 |
| Comparative Experiment A-B | 235.9 | 251.1 | 256.9 |

### Example 3

Sets of four flexural strength test pieces prepared from the castings of Example 1-D and Comparative Experiment A-B were weighed, then immersed in deionized water contained in individual jars and maintained at 92°C. The test pieces were weighed at the indicated intervals and the percent weight gain calculated as follows: 100[(exposed weight - initial weight)/initial weight]. An average of the percent weight gain was then calculated with the results reported in Table V.

**TABLE V**

| SAMPLE DESIGNATION | PERCENT WEIGHT GAIN (hours of exposure) | | | |
|---|---|---|---|---|
| | 24 | 48 | 72 | 94 |
| Example 1-D | 1.31 | 1.81 | 2.12 | 2.30 |
| Comparative Experiment A-B | 1.93 | 2.37 | 2.57 | 2.69 |

### Example 4

### A. Synthesis of bis(4'-Hydroxyphenyl)-1,4-diiminomethylbenzene

p-Aminophenol (327.4 grams, 3.00 moles) and methanol (1300 milliliters) were added to a 2 liter round bottom flask with stirring. Terephthaldehyde (201.2 grams, 1.50 moles) was added to the reactor followed by heating to 50°C. After 3 hours at 50°C, the reaction mixture was vacuum filtered and the solids thus obtained were washed with methanol (500 milliliters). The washed solids were added back to the reactor along with additional methanol (1300 milliliters) and then heated with stirring to reflux. After refluxing for one hour, the mixture was again vacuum filtered with the recovered solids again added back to the reactor along with fresh methanol (950 milliliters). After refluxing for one hour, the mixture was vacuum filtered and the solids thus recovered were dried at 80°C for four hours in a vacuum oven to a constant weight. The product was recovered (423.4 grams) as a light yellow colored powder in 89.2 percent yield. Differential scanning calorimetry of a portion of the product revealed a sharp melting point endotherm at 270°C. Infrared spectrophotometric analysis of a film of a nujol mull of a portion of the product confirmed the structure [phenolic hydroxyl absorbance (3370 cm⁻¹, broad), -CH=N-stretching absorbance (1618 cm⁻¹, sharp)].

### B. Preparation of Dicyanate of bis(4'-Hydroxyphenyl)--1,4-diiminomethylbenzene

bis(4'-Hydroxyphenyl)-1,4-diiminomethylbenzene (137.85 grams, 0.8716 hydroxyl equivalent) from A above, cyanogen bromide (96.94 grams, 0.915 mole) and acetone (1000 milliliters) were added to a reactor and maintained under a nitrogen atmosphere with stirring. The stirred solution was cooled to -5°C, then triethylamine (89.08 grams, 0.8803 moles) was added to the reactor over a 33 minute period and so as to maintain the reaction temperature at -5°C to -3°C. After completion of the triethylamine addition, the reactor was maintained at -5°C to -3°C for an additional 45 minutes followed by addition of the reactor contents to 1 gallon of deionized water. After 5 minutes, the water and product mixture was filtered through a fritted glass funnel and the resultant cake of yellow powder was washed by slurrying into 250 milliliters of deionized water. A second filtration recovered the yellow powder which was washed by slurring into 250 milliliters of methylene chloride. A third filtration recovered the yellow powder which was washed for a final time by slurring into 500 milliliters of deionized water followed by filtration to recover the yellow powder product. The dicyanate of bis(4'-hydroxyphenyl)-1,4-diiminomethylbenzene (155.1 grams) was recovered in 97.16 percent yield as a light yellow colored powder after drying at 100°C in a vacuum oven to constant weight. Infrared spectrophotometric analysis of a nujol mull of a portion of the product confirmed the product structure [disappearance of phenolic hydroxyl absorbance (3370 cm⁻¹, broad), appearance of cyanate absorbance (2234 and 2272 cm⁻¹, sharp) retention of the -CH=N- stretching absorbance (1620 cm⁻¹, sharp)].

### C. Evaluation of the Curing Behavior of the Dicyanate of bis(4'-Hydroxyphenyl)-1,4-diiminomethylbenzene Using Differential Scanning Calorimetry and Infrared Spectrophotometric Analysis

A portion (8.75 milligrams) of the dicyanate of bis(4'-hydroxyphenyl)-1,4-diiminomethylbenzene (uncatalyzed) from B above was analyzed by differential scanning calorimetry (DSC). Heating from 25°C to 330°C was completed at a rate of 10°C per minute under a stream of nitrogen flowing at 35 cubic centimeters per minute resulting in the exothermic curing of the dicyanate. The results are reported in Table VI.

**TABLE VI**

| DESCRIPTION OF TRANSITION | ONSET (°C) | MIDPOINT (°C) | END (°C) |
|---|---|---|---|
| Exotherm (minor) | 126 | 191 | 206¹ |
| Exotherm (major) | 206 | 213 | 238 |

| | | | |
|---|---|---|---|
| ¹End of first exotherm merges into the second exotherm. | | | |

The cured product was recovered from the differential scanning calorimetry as a yellow powder and was used to prepare a nujol mull. Infrared spectrophotometric analysis of a film of the nujol mull on a sodium chloride plate revealed that complete disappearance of the cyanate absorbance had occurred with retention of -CH=N- stretching absorbance and appearance of a new absorbance at 1560 cm⁻¹ attributed to the presence of the triazine ring.

### D. Evaluation of the Curing Behavior of the Dicyanate of bis(4'-Hydroxyphenyl)-1,4-diiminomethylbenzene Using Optical Microscopy

A portion of the dicyanate of bis(4'-hydroxyphenyl)-1,4-diiminomethylbenzene from B above was placed between two glass slides and heated on a hot stage. During this heating, observations relating to changes in the morphology of the dicyanate were made at 35X magnification using a cross polarized light source. At a heating rate of 20°C per minute, no melt for the dicyanate was observed up to 280°C. However, when a second sample of the dicyanate between glass slides was introduced on to the hot stage preheated to 250°C, melt flow was observed. After melt occurred, a birefringent morphology was produced. Curing followed the melt within 30 seconds with retention of birefringence in the solid. Another sample of the dicyanate between glass slides was introduced on to the hot stage preheated to 250°C. After melt had occurred, shear was applied to the sample by moving the top glass slide back and forth. With the application of shear, a shimmering fluorescence was produced. After the sample had solidified, birefringent striations were also observed which were oriented in the same direction that the shear was applied.

### E. Preparation of Cured Dicyanate of bis(4'-Hydroxyphenyl)-1,4-diiminomethylbenzene for Thermal Mechanical Analysis

A portion (0.71 gram) of the dicyanate of bis(4'-hydroxyphenyl)-1,4-diiminomethylbenzene from B above was placed in an aluminum pan which had been coated with a mold release. The aluminum pan was then put in a forced air convection type oven preheated to 250°C. Within the first minute in the oven, melt flow followed by thermosetting was observed. After 4 hours at 250°C, the oven was allowed to slowly cool to room temperature (25°C) then the cured layer was removed from the pan. Optical microscopy of the cured product at 70X magnification using a cross polarized light source revealed extensive birefringence. Glass transition temperature and the mean linear thermal coefficient of expansion over the range from 35°C to Tg were evaluated using a portion of the cured product. In this analysis, a constant probe force of 0.1 Newtons and a heating rate of 10°C per minute were used over a range of 25°C to 325°C. These results are reported in Table IX for both the initial and a second heating of the same sample.

### Example 5

### Preparation of a Blend of the Dicyanate of bis(4'-Hydroxyphenyl)-1,4-diiminomethylbenzene and the Polycyanate of a Dicyclopentadiene Phenolic Novolac: Evaluation of Curing Behavior Using Differential Scanning Calorimetry and Thermal Mechanical Analysis of the Cured Blend

A portion (0.88 gram) of the dicyanate of bis(4'-hydroxyphenyl)-1,4-diiminomethylbenzene from Example 4-B and 1.77 grams of a 2.2 cyanate functional polycyanate of a dicyclopentadiene phenolic novolac (uncatalyzed) from the same lot as Comparative Experiment C were mixed in an aluminum pan in a 100°C oven. A paste was formed, a portion (15.0 milligrams) was analyzed by differential scanning calorimetry (DSC). Heating from 25°C to 330°C was completed at a rate of 10°C per minute under a stream of nitrogen flowing at 35 cubic centimeters per minute resulting in exothermic curing. The results are reported in Table VII.

**TABLE VII**

| DESCRIPTION OF TRANSITION | ONSET (°C) | MIDPOINT (°C) | END (°C) |
|---|---|---|---|
| Exotherm (enthalpy = -375 J/g) | 165 | 217 | 280 |

The differential scanning calorimetry results may be compared with those shown for the neat dicyanate of bis(4'-hydroxyphenyl)-1,4-diiminomethylbenzene in Example 4-C (Table VI) and the neat polycyanate of the dicyclopentadiene phenolic novolac in Comparative Experiment C (Table VIII). When compared to the neat polycyanate of the dicyclopentadiene phenolic novolac in Comparative Experiment C, the addition of the dicyanate of bis(4'-hydroxyphenyl)-1,4-diiminomethylbenzene to form the present blend has reduced the onset temperature to the curing exotherm by 35°C and the curing exotherm peak temperature by 79°C.

Immediately after removal of the sample for differential scanning calorimetry, the aluminum pan was placed in a forced air, convection type oven preheated to 250°C. Within two minutes in the oven, melt flow followed by thermosetting was observed. After 4 hours at 250°C, the oven was allowed to slowly cool to room temperature (25°C) then the cured layer removed from the pan. Glass transition temperature and the mean linear thermal coefficient of expansion over the range from 35°C to Tg were evaluated using a portion of the cured product. In this analysis, a constant probe force of 0.1 Newtons and a heating rate of 10°C per minute were used over a range of 25°C to 325°C. These results are reported in Table IX for both the initial and a second heating of the same sample.

### COMPARATIVE EXPERIMENT C

### Evaluation of the Curing Behavior of the Polycyanate of a Dicyclopentadiene Phenolic Novolac and Thermal Mechanical Analysis of the Cured Polytriazine

15.0 milligrams of a commercial grade 2.2 cyanate functional polycyanate of a dicyclopentadiene phenolic novolac (uncatalyzed) was analyzed by differential scanning calorimetry (DSC). Heating from 25 to 330°C was completed at a rate of 10°C per minute under a stream of nitrogen flowing at 35 cubic centimeters per minute resulting in exothermic curing. The results are reported in Table VIII.

**TABLE VIII**

| DESCRIPTION OF TRANSITION | ONSET (°C) | MIDPOINT (°C) | END (°C) |
|---|---|---|---|
| Exotherm (enthalpy = -354 J/g) | 200 | 296 | 315 |

1.93 grams of the polycyanate of a dicyclopentadiene phenolic novolac was placed in an aluminum pan. The aluminum pan was then put in a forced air convection type oven preheated to 250°C. After twenty minutes in the oven thermosetting was observed. After 4 hours at 250°C, the oven was allowed to slowly cool to room temperature (25°C) then the cured layer removed from the pan. Optical microscopy of the cured product at 70X magnification using a cross polarized light source revealed a minor amount of birefringence. Glass transition temperature and the mean linear thermal coefficient of expansion over the range from 35°C to Tg were evaluated using a portion of the cured product. In this analysis, a constant probe force of 0.1 Newtons and a heating rate of 10°C per minute were used over a range of 25°C to 325°C. These results are reported in Table IX for both the initial and a second heating of the same sample.

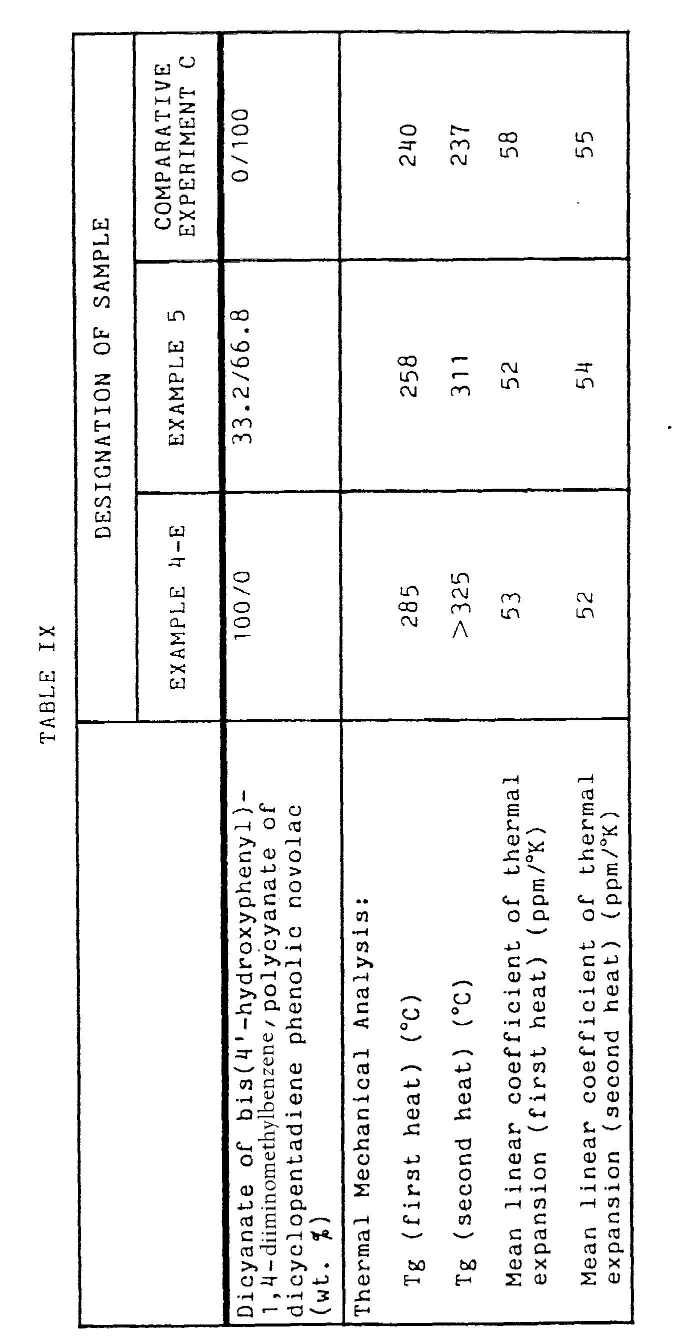

### Example 6

### Preparation of a Cured Polytriazine Casting from a Blend of Bisphenol A Dicyanate and the Dicyanate of bis(4'-Hydroxyphenyl)-1,4-diiminomethylbenzene

A 175.0 gram portion of bisphenol A dicyanate prepared using the method of Comparative Experiment A-A and a 25.0 gram portion of the dicyanate of bis(4'-hydroxyphenyl)-1,4-diiminomethylbenzene from Example 4-B were heated to 145°C to form a solution, then further heated to 150°C and held therein for 10 minutes. The resulting B-staged resin solution was filtered while hot through a heated fritted glass funnel. This solution was then poured into a 1/8 inch (31.7 mm) mold, then placed in an oven and maintained at 125°C for 2 hours, 177°C for 4 hours, 200°C for 4 hours, then 250°C for 2 hours. The transparent, light amber colored casting was demolded and used to prepare test pieces which were tested using the method of Example 1-D. The results are reported in Table X and may be compared with those obtained for Comparative Experiment A-B reported in Table III.

**TABLE X**

| MECHANICAL PROPERTY | EXAMPLE 6 |
|---|---|
| Barcol Hardness | 51 |
| Tensile Strength (psi) | 12,753 |
| Tensile Modulus (psi) | 564,869 |
| Elongation (%) | 3.09 |
| Flexural Strength (psi) | 23,475 |
| Flexural Modulus (psi) | 588,060 |

### Example 7

### Differential Scanning Calorimetry

The method of Example 2 was repeated using a portion (8.6 milligrams) of the bisphenol A dicyanate and dicyanate of bis(4'-hydroxyphenyl)-1,4-diiminomethylbenzene blend from Example 6. The results from the second heating are reported in Table XI and may be compared with those reported for Comparative Experiment A-B reported in Table IV.

**TABLE XI**

| SAMPLE DESIGNATION | GLASS TRANSITION TEMPERATURE | | |
|---|---|---|---|
| | ONSET (°C) | MIDPOINT (°C) | END (°C) |
| Example 4-D | 255.5 | 263.4 | 277.6 |

### Example 8

### Differential Scanning Calorimetry of Blends of the Dicyanate of 4,4'-Dihydroxy-alpha-methylstilbene and Various Epoxy Resins

### Blend A:

A portion (0.5525 gram, 0.004 cyanate equivalent) of the dicyanate of 4,4'-dihydroxy-alpha-methylstilbene from Example 1-D above, a commercial grade diglycidyl ether of bisphenol A (179.95 epoxide equivalent weight) (0.7198 gram, 0.004 epoxide equivalent) and cobalt naphthenate (0.0013 gram, 0.1 percent by weight) were blended then gently heated to form a homogeneous solution.

### Blend B:

A portion (0.4144 gram, 0.003 cyanate equivalent) of the dicyanate of 4,4'-dihydroxy-alpha-methylstilbene from Example 1-D above, the diglycidyl ether of 4,4'-dihydroxy-alpha-methylstilbene (177.61 epoxide equivalent weight) (0.5328 gram, 0.003 epoxide equivalent) and cobalt naphthenate (0.001 gram, 0.1 percent by weight) were blended then gently heated to form a homogeneous solution.

Portions (9.4 and 8.8 milligrams, respectively) of the aforementioned blends were analyzed by differential scanning calorimetry using the method of Example 2. The results from the second heating are reported in Table XII.

**TABLE XII**

| SAMPLE DESIGNATION | GLASS TRANSITION TEMPERATURE | | |
|---|---|---|---|
| | ONSET (°C) | MIDPOINT (°C) | END (°C) |
| Example 8, Blend A | 172.0 | 188.3 | 204.3 |
| Example 8, Blend B | 208.5 | 215.7 | 221.1 |

### Example 9

### Differential Scanning Calorimetry of A Blend of the Dicyanate of 4,4'-Dihydroxy-alpha-methylstilbene and p-Vinyltoluene

A portion (0.70 gram, 70.0 percent by weight) of the dicyanate of 4,4'-dihydroxy-alpha-methylstilbene from Example 1-D above, p-vinyltoluene (0.30 gram, 30 percent by weight) and cobalt naphthenate (0.001 gram, 0.1 percent by weight) were blended then gently heated to form a homogeneous solution. A portion (9.5 milligrams) of the aforementioned blend was analyzed by differential scanning calorimetry using the method of Example 2. The results from the second heating are reported in Table XIII.

**TABLE XIII**

| SAMPLE DESIGNATION | GLASS TRANSITION TEMPERATURE | | |
|---|---|---|---|
| | ONSET (°C) | MIDPOINT (°C) | END (°C) |
| Example 9 | 264.4 | 289.2 | 309.2 |

### Example 10

### A. Synthesis of 4,4'-Dihydroxybenzanilide

4,4'-dihydroxybenzophenone (100.0 grams, 0.467 mole) was added to ethanol (300 milliliters) in a 1 liter Erlenmeyer flask and stirred. After dissolution of the 4,4'-dihydroxybenzophenone, a solution of hydroxylamine hydrochloride (48.6 grams, 0.699 mole) and sodium acetate (57.4 grams, 0.700 mole) in deionized water (70 milliliters) was added followed by an additional 100 milliliters of ethanol. This mixture was stirred and heated on a hot plate to a gentle reflux (75°C). After heating for 4 hours, the solution was allowed to cool to room temperature (25°C) with stirring and then filtered. The filter cake was washed with ethanol (100 milliliters); then the total filtrate obtained (600.4 grams) was concentrated to a weight of 219.2 grams by evaporation of the ethanol and water. The concentrated solution was placed into a 1 liter Erlenmeyer flask then stirred as 600 milliliters of deionized water was added. The addition of the deionized water induced formation of a white precipitate. After thirty minutes of stirring, the mixture was filtered. The solid 4,4'-dihydroxybenzophenone oxime thus obtained weighed 98.22 grams after drying. 4,4'-Dihydroxybenzophenone oxime (66.0 grams, 0.288 mole) and glacial acetic acid (330 milliliters) were added to a 500 milliliter round bottom flask equipped with a stirrer, water cooled condensor, nitrogen purge and heating mantle. A catalytic amount of p-toluenesulfonic acid (1.85 grams, 0.027 mole) was added and the reaction mixture then heated to 83°C. After heating for approximately two hours, a precipitate formed and the mixture was stirred for an additional two hours at 87°C. After this time, 25 milliliters of deionized water was added to the reactor and after thirty minutes, the contents of the reactor was transferred to a 1 liter Erlenmeyer flask and maintained therein with stirring. Immediately following the transfer, 400 milliliters of deionized water was added to the flask. The mixture was stirred for forty five minutes and then filtered. The filter cake thus obtained was washed with 800 milliliters of deionized water and then dried. The resultant light beige colored solid weighed 54.82 grams. Fourier transform infrared spectrophotometric analysis of a portion of the product confirmed the product structure as that of 4,4'-dihydroxybenzanilide. Differential scanning calorimetry demonstrated a sharp melting point endotherm at 273°C for the 4,4'-dihydroxybenzanilide product.

### B. Preparation of Dicyanate of 4,4'-Dihydroxybenzanilide

4,4'-Dihydroxybenzanilide (32.0 grams, 0.2792 hydroxyl equivalent) from A above, cyanogen bromide (32.53 grams, 0.3071 mole) and acetone (500 milliliters) were added to a reactor and maintained under a nitrogen atmosphere with stirring. The stirred solution was cooled to -5°C, then triethylamine (28.54 grams, 0.2820 mole) was added to the reactor over a 20 minute period and so as to maintain the reaction temperature at -5°C to -3°C. After completion of the triethylamine addition, the reactor was maintained at -5°C to -3°C for an additional 45 minutes followed by addition of the reactor contents to 1 gallon of deionized water. After 2 minutes of agitation the water and product mixture was filtered through a fritted glass funnel to remove a cake of white crystalline product. The filter cake was washed with 500 milliliters of deionized water, recovered by filtration, and again washed with 500 milliliters of deionized water. The cake recovered from a final filtration was dried at 80°C in a vacuum oven to a constant weight of 37.55 grams (96.33 percent isolated yield). Infrared spectrophotometric analysis of a film sample of the product confirmed the product structure (presence of amide N-H stretching at 3403 cm⁻¹, sharp, presence of amide I carbonyl stretching in solid state at 1681 cm⁻¹ disappearance of phenolic hydroxyl absorbance, appearance of cyanate absorbance (2273 and 2232 cm⁻¹, sharp).

### C. Evaluation of the Curing Behavior of the Dicyanate of 4,4'-Dihydroxybenzanilide Using Differential Scanning Calorimetry and Infrared Spectrophotometric Analysis

A portion (11.40 milligrams) of the dicyanate of 4,4'-dihydroxybenzanilide (uncatalyzed) from B above was analyzed by differential scanning calorimetry (DSC). Heating from 25°C to 250°C was completed at a rate of 10°C per minute under a steam of nitrogen flowing at 35 cubic centimeters per minute resulting in exothermic curing of the dicyanate. The results are reported in Table XIV.

**TABLE XIV**

| DESCRIPTION OF TRANSITION | ONSET (°C) | MIDPOINT (°C) | END (°C) |
|---|---|---|---|
| Endotherm (minor) | 179 | 187 | 191 |
| Exotherm (enthalpy = -359 J/g) | 191 | 199 | 209 |

The cured product was recovered from the differential scanning calorimetry as a light amber colored solid and was used to prepare a nujol mull. Infrared spectrophotometric analysis of a film sample of the nujol mull on a sodium chloride plate revealed that complete disappearance of the cyanate absorbance, the amide carbonyl stretch and the amide N-H stretch had occurred with appearance of new absorbances at 1743, 1654 and 1560 cm⁻¹ (1560 cm⁻¹ was attributed to the presence of the triazine ring).

### D. Evaluation of the Curing Behavior of the Dicyanate of 4,4'-Dihydroxybenzanilide Using Optical Microscopy

A portion of the dicyanate of 4,4'-dihydroxybenzanilide from B above was placed between two glass slides and heated on a hot stage. During this heating, observations relating to changes in the morphology of the dicyanate were made at 35X magnification using a cross polarized light source. At a heating rate of 10°C per minute melt flow was observed at 184°C and resulted in a birefringent texture. Curing followed the melt once the temperature reached 187°C. On cooling to room temperature (25°C) extensive birefringence was observed in the thermoset product. Another sample of the dicyanate between glass slides was introduced on to the hot stage preheated to 190°C. Within one minute, melt had occurred then shear was applied to the sample by moving the top glass slide back and forth. After less than an additional 30 seconds, the sample had solidified and the hot stage was cooled to room temperature at a rate of 10°C per minute. At room temperature, birefringent striations were also observed which were oriented in the same direction that the shear was applied. Upon reheating of the thermoset at a rate of 10°C per minute, the birefringent striations remained up to 250°C at which temperature heating was discontinued.

### E. Preparation of Cured Dicyanate of 4,4'-Dihydroxybenzanilide for Thermal Mechanical Analysis

A portion (0.50 gram) of the dicyanate of 4,4'-dihydroxybenzanilide from B above was placed in an aluminum pan. The aluminum pan was then put in a forced air convection type oven preheated to 190°C. Within the first five minutes in the oven, melt flow followed by thermosetting was observed. After 5 minutes at 190°C, the oven temperature was raised to 250°C and maintained therein for four hours before the oven was allowed to slowly cool to room temperature (25°C); then the cured layer removed from the pan. Optical microscopy of the cured product at 70X magnification using a cross polarized light source revealed extensive birefringence. Glass transition temperature and the mean linear thermal coefficient of expansion over the range from 35°C to Tg were evaluated using a portion of the cured product. In this analysis, a constant probe force of 0.1 Newtons and a heating rate of 10°C per minute were used over a range of 25 to 325°C. These results are reported in Table XVI.

### Example 11

### Preparation of a Blend of the Dicyanate of 4,4'-Dihydroxybenzanilide and the Polycyanate of a Dicyclopentadiene Phenolic Novolac: Evaluation of Curing Behavior Using Differential Scanning Calorimetry and Thermal Mechanical Analysis of the Cured Blend

A portion (0.88 gram) of the dicyanate of 4,4'-dihydroxybenzanilide from Example 10-B and 1.72 grams of a polycyanate of a dicyclopentadiene phenolic novolac (uncatalyzed) from the same lot as Comparative Experiment C were mixed in an aluminum pan contained in a 100°C oven. From the paste formed, a portion (15.0 milligrams) was analyzed by differential scanning calorimetry (DSC). Heating from 25°C to 330°C was completed at a rate of 10°C per minute under a stream of nitrogen flowing at 35 cubic centimeters per minute resulting in exothermic curing. The results are reported in Table XV.

**TABLE XV**

| DESCRIPTION OF TRANSITION | ONSET (°C) | MIDPOINT (°C) | END (°C) |
|---|---|---|---|
| Exotherm (enthalpy = -459 J/g) | 165 | 212 | 240 |

The differential scanning calorimetry results may be compared with those shown for the neat dicyanate of 4,4'-dihydroxybenzanilide in Example 10-C (Table XIV) and the neat polycyanate of the dicyclopentadiene phenolic novolac in Comparative Experiment C (Table VIII). When compared to the neat polycyanate of the dicyclopentadiene phenolic novolac in Comparative Experiment C, the addition of the dicyanate of 4,4'-dihydroxybenzanilide to form the present blend has reduced the onset temperature of the curing exotherm by 35°C and the curing exotherm peak temperature by 84°C.

Immediately after removal of the sample for differential scanning calorimetry, the aluminum pan was placed in a forced air, convection type oven preheated to 190°C. Within fifteen minutes in the oven, melt flow followed by thermosetting was observed. After 3 hours at 190°C, the oven temperature was raised to 232°C and maintained therein for two hours before the oven was allowed to slowly cool to room temperature (25°C) then the cured layer removed from the pan. Glass transition temperature and the mean linear thermal coefficient of expansion over the range from 35°C to Tg were evaluated using a portion of the cured product. In this analysis, a constant probe force of 0.1 Newtons and a heating rate of 10°C per minute were used over a range of 25°C to 325°C. These results are reported in Table XVI.

### COMPARATIVE EXPERIMENT D

### Thermal Mechanical Analysis of the Cured Polytriazine of a Polycyanate of a Dicyclopentadiene Phenolic Novolac: Use of a Different Cure Schedule

2.57 grams of the polycyanate of a dicyclopentadiene phenolic novolac from the same lot as Comparative Experiment C was placed in an aluminum pan. The aluminum pan was then put in a forced air convection type oven preheated to 190°C. After three hours at 190°C, the oven temperature was raised to 232°C and maintained therein for two hours before the oven was allowed to slowly cool to room temperature (25°C); then the cured layer was removed from the pan. Optical microscopy of the cured product at 70X magnification using a cross polarized light source revealed a minor amount of birefringence. Glass transition temperature and the mean linear thermal coefficient of expansion over the range from 35°C to Tg were evaluated using a portion of the cured product. In this analysis, a constant probe force of 0.1 Newtons and a heating rate of 10°C per minute were used over a range of 25°C to 325°C. These results are reported in Table XVI.

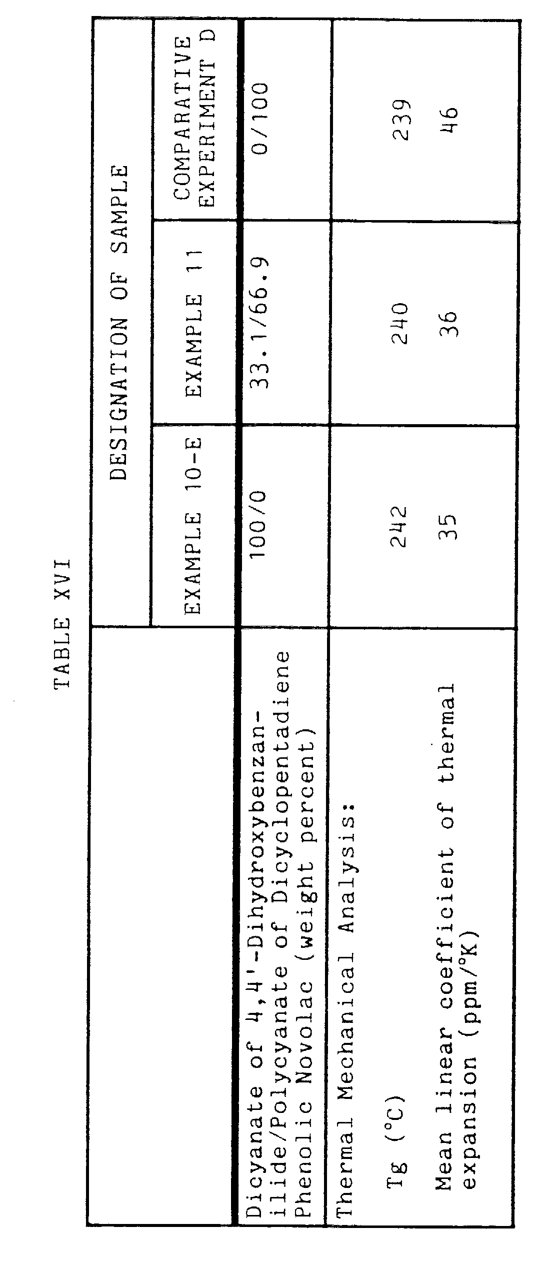

## Claims

1. A polycyanate or polycyanamide composition containing one or more mesogenic or rodlike moieties represented by the following formulas I, II, III or IV: 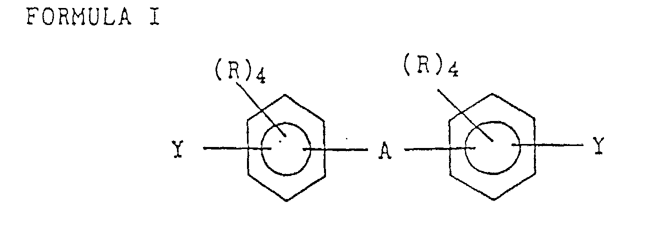 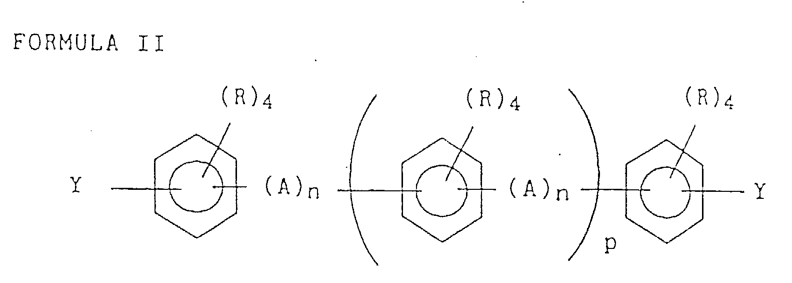 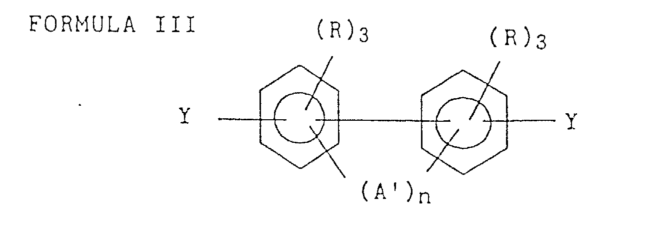 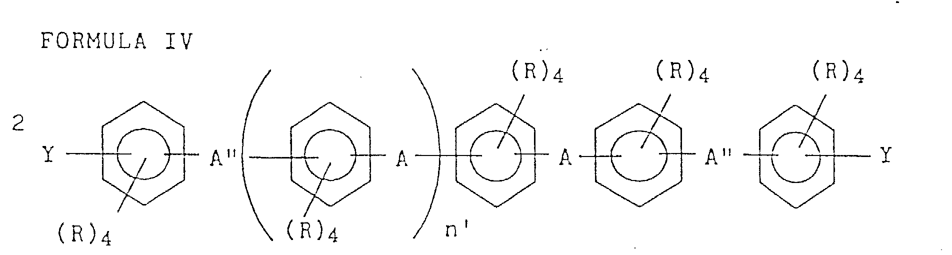 wherein at least about 80 percent of the -A- linkages, the direct bond in Formula III and the Y groups are in the para position with respect to each other; each Y is independently a -O-C≡N or a -NR¹-C≡N group; each A is independently -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹, -CR¹=CR¹-O-CO-, -CO-O-CR¹=CR¹-, -CO-O-N=CR¹-, -CR¹=N-O-CO-, -CR¹=CR¹-CO-O-, -CO-S-,--O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 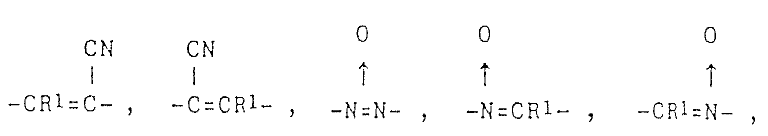 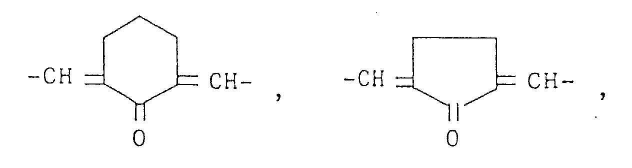 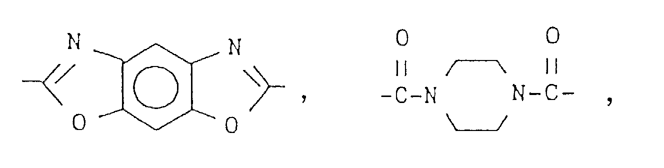 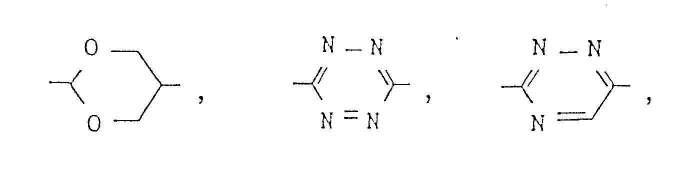 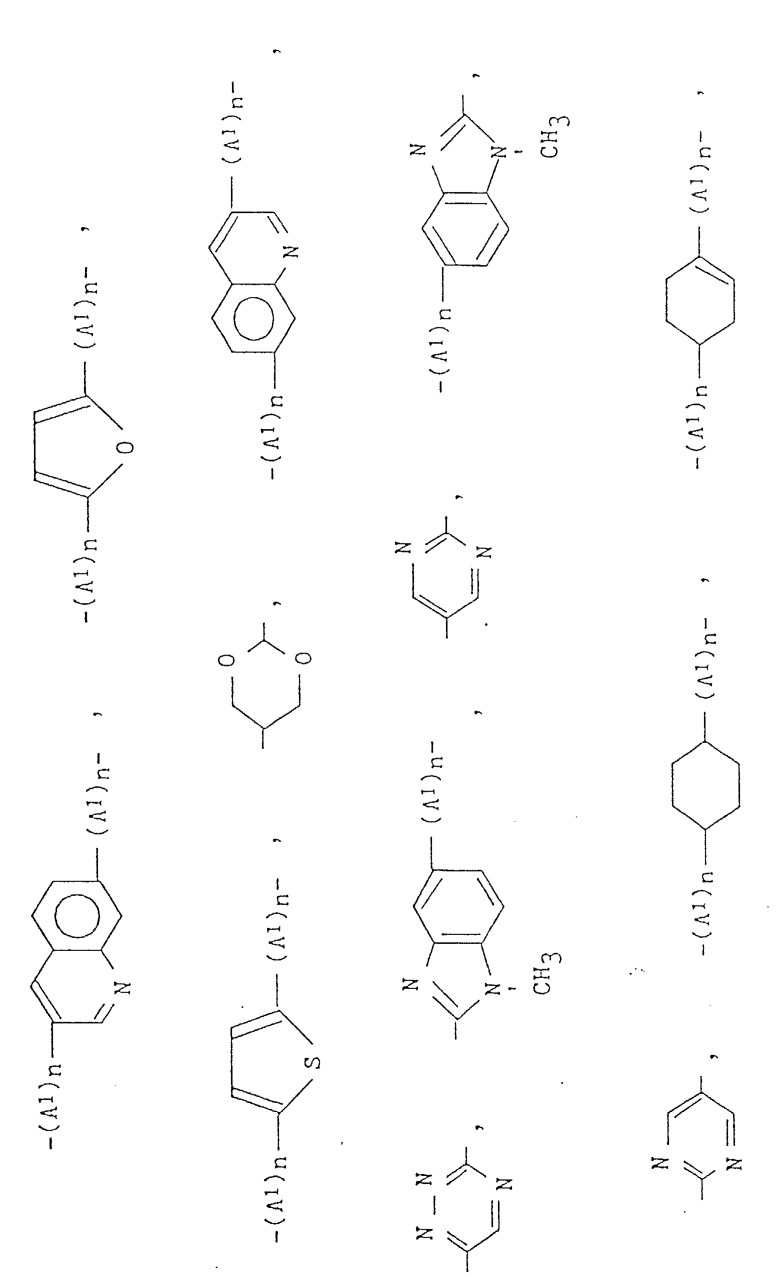 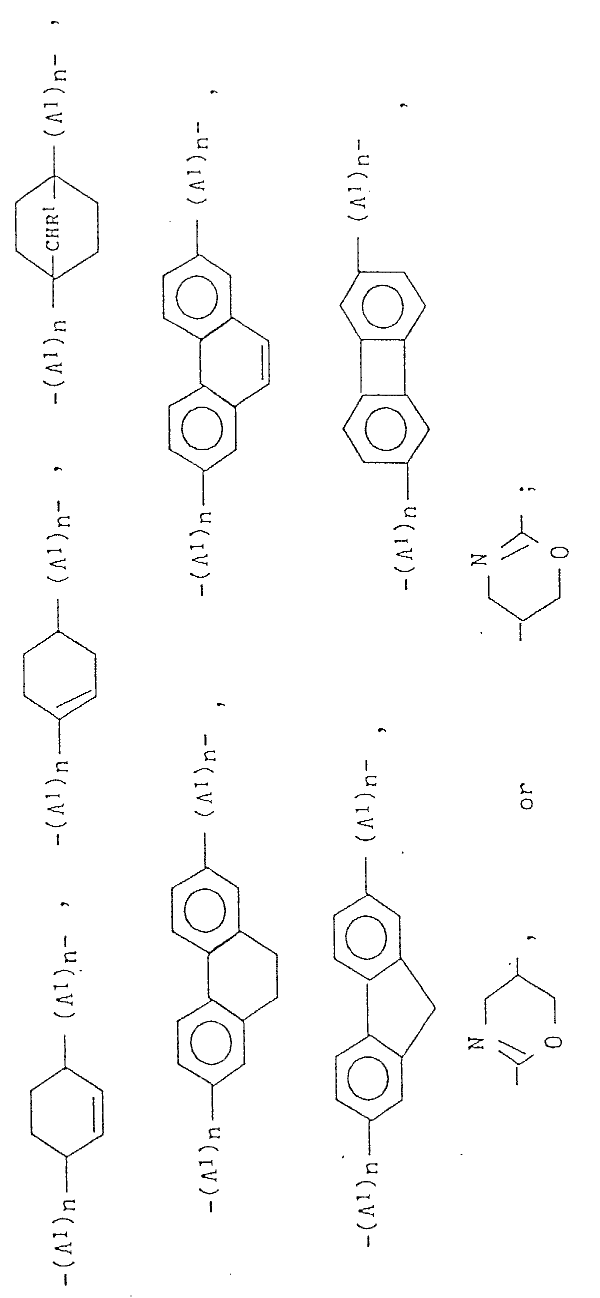 each A' is independently a divalent hydrocarbyl group having from 1 to 10 carbon atoms; each A" is independently an alkylene group having from 1 to 10 carbon atoms, a direct bond, -O-, -CO-, -S-, -S-S-, -SO-, -SO₂- or -O-CO-O-; each A¹ is independently a -CO-, -O-CO-, -CO-O-, -CO-NR¹-, or -NR¹-CO- group; each R is independently hydrogen or a hydrocarbyl or hydrocarbyloxy group having from 1 to 10 carbon atoms, a halogen atom, a nitro group, a nitrile group, a phenyl group or a -CO-R¹ group; each R¹ is independently hydrogen or a hydrocarbyl group having 1 to 3 carbon atoms; n has a value of zero or one; n' has a value from 1 to 6; p has a value from 1 to 30; and wherein the aromatic rings can also contain one or more heteroatoms selected from N, O, or S; with the proviso that in formula I, where R = H and Y = -O-C≡N, A may not be -CH=CH- and with the proviso that in formula I, where R = H and Y = -NH-C≡N, A may not be -N=N-, -NH-CO- or -CO-O- and with the proviso that in formula IV where R is -H or CH₃, n = 1, Y = -O-C≡N and A" is as hereinbefore defined, both A groups may not simultaneously be -O-CO-, the dicyanate of bis(4'-hydroxyphenyl)-1,4-diiminomethylbenzene being excluded.

2. A polycyanate or polycyanamide composition of Claim 1 wherein said polycyanate or polycyanamide is the dicyanate of 4,4'-dihydroxy-α-methylstilbene, the dicyanate of 4,4'-dihydroxybenzanilide or any combination thereof.

3. A curable composition comprising a polycyanate or polycyanamide composition of Claim 1 or 2 and one or more suitable curing agents or curing catalysts therefor.

4. A polymerizable composition comprising a mixture of
(A) at least one thermosettable polycyanate or polycyanamide containing one or more mesogenic or rodlike moities as defined in claim 1
(B) at least one component selected from the group consisting of
(1) at least one polycyanate or polycyanamide which does not contain mesogenic or rodlike structures;
(2) at least one epoxy resin;
(3) at least one polymaleimide;
(4) at least one polyamine;
(5) at least one polyphenol;
(6) at least one compound containing one or more polymerizable ethylenically unsaturated group(s);
(7) at least one compound which contains in the same molecule both a cyanate or cyanamide group and a polymerizable ethylenically unsaturated group;
(8) at least one compound which contains in the same molecule both a 1,2-epoxide group and a polymerizable ethylenically unsaturated group;
(9) at least one compound which contains in the same molecule both a maleimide group and a cyanate group;
(10) at least one compound which contains one or more mesogenic or rodlike moieties and only one cyanate or cyanamide group per 4,4'-molecule;
(11) at least one prepolymer of any of the aforesaid components (1) through (10) or any combination of any two or more of said components; or
(12) a mixture of any two or more of components (1) through (11) in any proportion and any combination with the proviso that component (A) may include 4,4'-dicyanatostilbene (formula I, R = H, Y = -O-C≡N, A = -CH=CH-) if components (B-4) or (B-5) are not used and with the proviso that component (A) may include 4,4'-dicyanamidoazobenzene (formula I, R = H, Y = -NH-C≡N, A = -N=N-), 4,4'-dicyanamidobenzanilide (formula I, R = H, Y = -NH-C≡N, A = -NH-CO-) or 4,4'-dicyanamidophenylbenzoate (formula I, R = H, Y = -NH-C≡N, A = -CO-O-) if components (B-3) or (B-4) are not used and with the proviso that component (A) may include the compositions of formula IV where R is -H or -CH₃, n = 1, Y = -O-C≡N, A" is as hereinbefore defined, where both A groups may simultaneously be -O-CO-.

5. A polymerizable composition of Claim 4 wherein components (A) and (B) are present in amounts of from 50 to 90 parts by weight of component (A) and from 50 to 10 parts by weight of component (B) and said polycyanate or polycyanamide is the dicyanate of 4,4'-dihydroxy-α-methylstilbene, the dicyanate of 4,4'-dihydroxybenzanilide or any combination thereof.

6. A curable composition comprising a composition of Claim 4, 5 and one or more suitable curing agents or curing catalysts therefor.

7. A curable composition of Claim 6 wherein said curing agent or curing catalyst is a Lewis acid, a protonic acid, an aromatic hydroxy compound, a base including alkali metal hydroxides, alkoxides and phenates, a tertiary amine, a quaternary ammonium salt, pyridine-N-oxides, trialkylphosphines, metal salts and metal chelates or an organic peroxide or hydroperoxide, or an azo or diazo compound or any combination thereof.

8. A curable composition of Claim 3 or 6 wherein said curing agent or curing catalyst is cobalt naphthenate, cobalt octoate, cobalt acetylacetonate or any combination thereof.

9. The product resulting from polymerizing the composition of Claim 1 or 4.

10. The product of Claim 9 wherein said composition is subjected to orientation during polymerization.

11. The product resulting from curing the curable composition of Claim 3 or 6.

12. The product of Claim 11 wherein said curable composition is subjected to orientation during curing.

13. The product of Claim 10 or 12 wherein said orientation is accomplished by means of an electric field, a magnetic field or shear stresses.

## Patentansprüche

1. Polycyanat- oder Polycyanamidzusammensetzung, enthaltend eine oder mehrere mesogene oder stabförmige Einheiten, wiedergegeben durch die folgenden Formeln I, II, III oder IV: 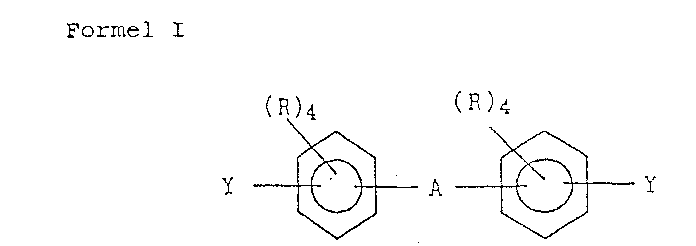 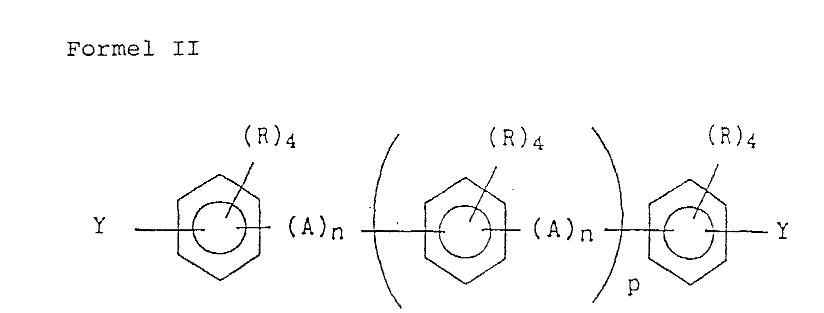 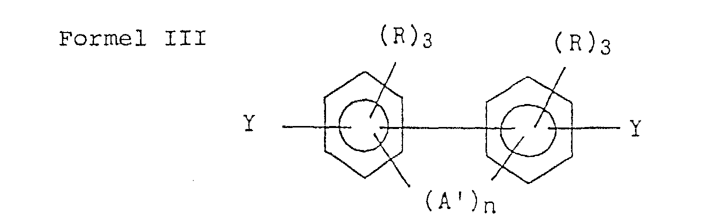 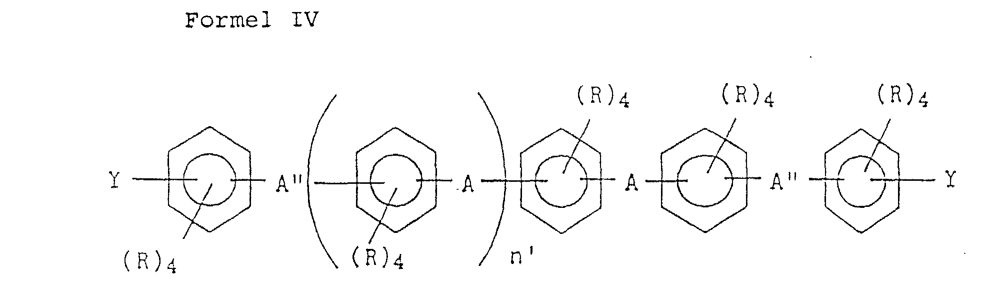 worin wenigstens etwa 80 % der -A-Bindungen, die direkte Bindung in Formel III und die Y-Gruppen in para-Stellung zueinander stehen; jedes Y unabhängig eine Gruppe -O-C≡N oder -NR¹-C≡N ist; jedes A unabhängig ist: -CR¹=CR¹-, -C≡C-, -N=N-, -CR¹=N-, -O-CO-, -NR¹-CO-, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹-, -CR¹=CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 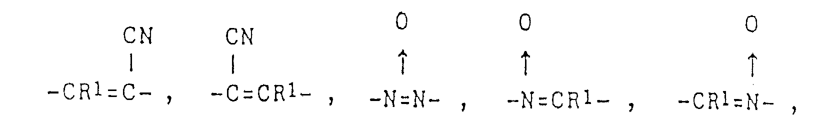 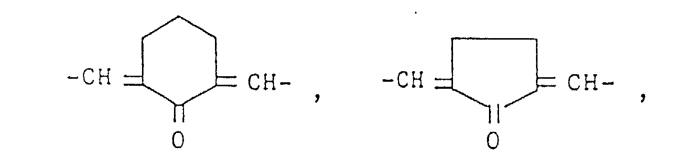 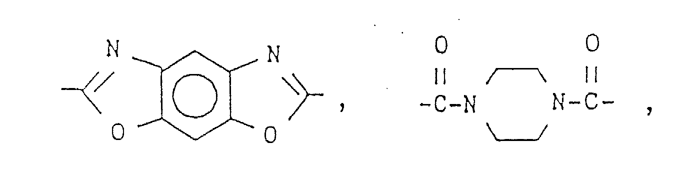 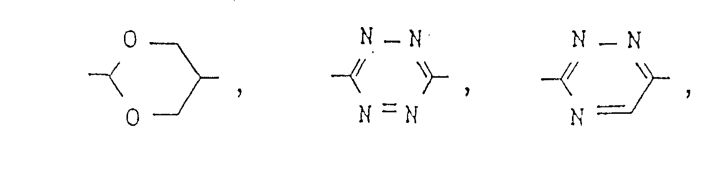 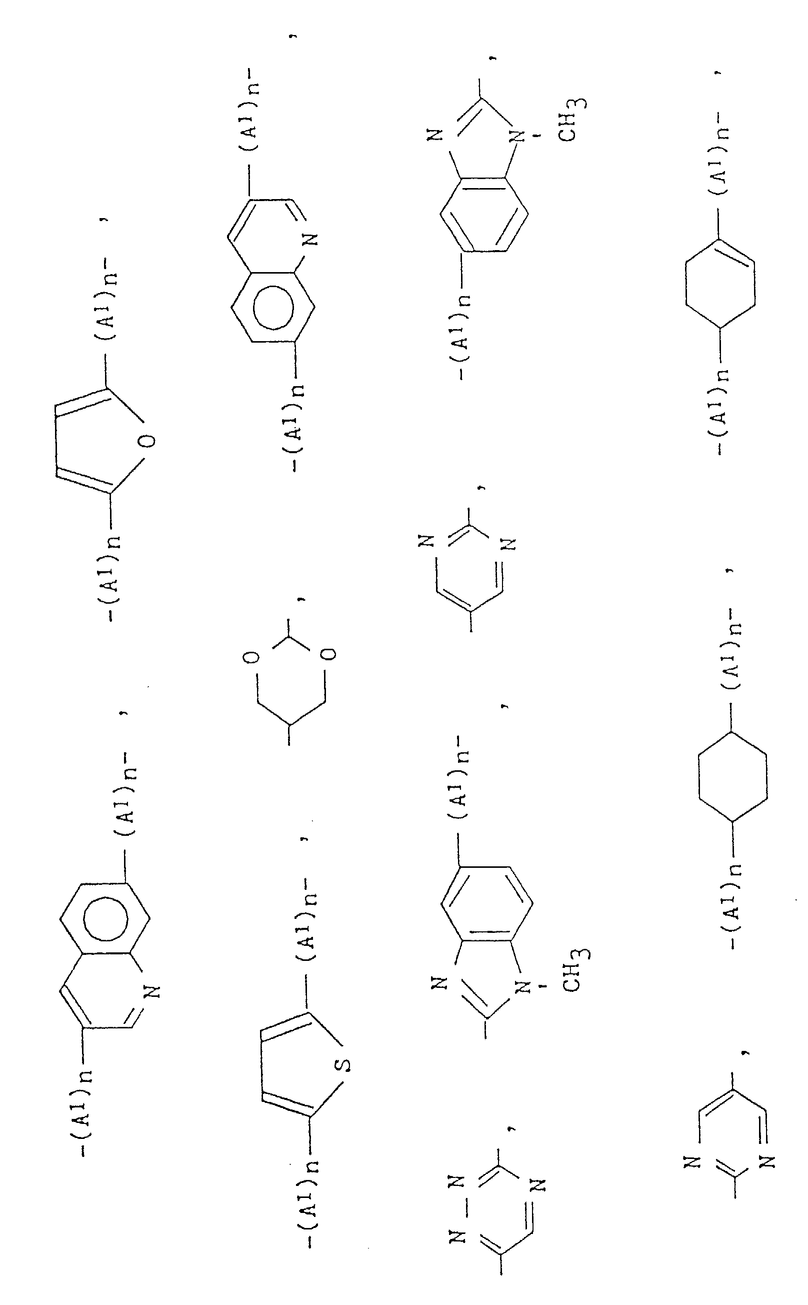 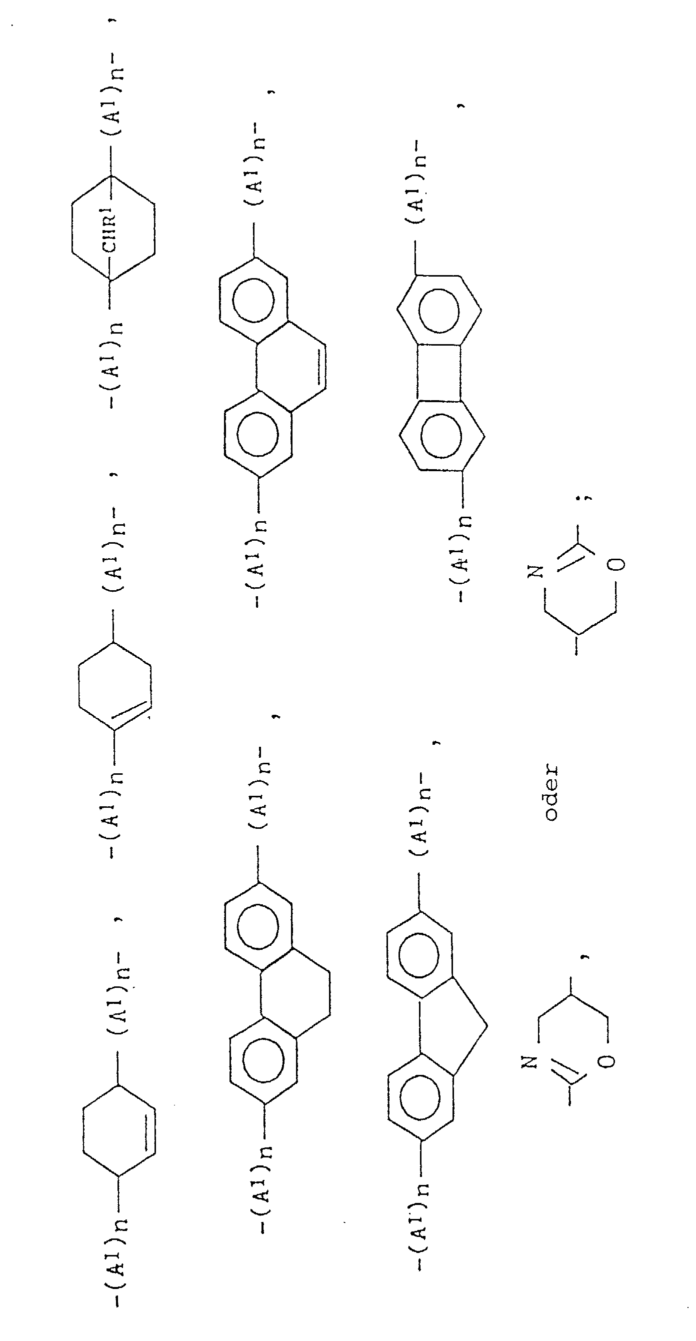 jedes A' unabhängig eine zweiwertige Hydrocarbylgruppe mit 1 bis 10 Kohlenstoffatomen ist; jedes A" unabhängig eine Alkylengruppe mit 1 bis 10 Kohlenstoffatomen, eine direkte Bindung, -O-, -CO-, -S-, -S-S-, -SO-, -SO₂- oder -O-CO-O- ist; jedes A¹ unabhängig eine -CO-, -O-CO-, -CO-O-, -CO-NR¹- oder -NR¹-CO- Gruppe ist; jedes R unabhängig Wasserstoff oder eine Hydrocarbyl- oder Hydrocarbyloxygruppe mit 1 bis 10 Kohlenstoffatomen, ein Halogenatom, eine Nitrogruppe, eine Nitrilgruppe, eine Phenylgruppe oder eine -CO-R¹ Gruppe ist; jedes R¹ unabhängig Wasserstoff oder eine Hydrocarbylgruppe mit 1 bis 3 Kohlenstoffatomen ist; n einen Wert von null oder 1 hat; n' einen Wert von 1 bis 6 hat; p einen Wert von 1 bis 30 hat; und worin die aromatischen Ringe auch ein oder mehrere Heteroatome, ausgewählt aus N, O und S, enthalten können; mit der Maßgabe, daß in der Formel I, falls R = H und Y = -O-C≡N sind, A nicht -CH=CH- sein kann, und mit der Maßgabe, daß falls in Formel I R = H und Y = -NH-C≡N sind, A nicht -N=N-, -NH-CO- oder -CO-O- sein kann, und mit der Maßgabe, daß falls in Formel IV R = -H oder -CH₃ ist, n = 1, Y = -O-C≡N und A" wie zuvor definiert ist, beide A Gruppen gleichzeitig nicht -O-CO- sein können, wobei das Dicyanat von Bis(4'-hydroxyphenyl)-1,4-benzoldiimin ausgeschlossen ist.

2. Polycyanat- oder Polycyanamidzusammensetzung nach Anspruch 1, in welcher dieses Polycyanat oder Polycyanamid das Dicyanat von 4,4'-Dihydroxy-α-methylstilben, das Dicyanat von 4,4'-Dihydroxybenzanilid oder eine beliebige Kombination hiervon ist.

3. Härtbare Zusammensetzung, umfassend eine Polycyanat- oder Polycyanamidzusammensetzung nach Anspruch 1 oder 2 und einen oder mehrere Härter oder Aushärtkatalysatoren hierfür.

4. Polymerisierbare Zusammensetzung, umfassend eine Mischung von:
(A) wenigstens einem hitzehärtbaren Polycyanat oder Polycyanamid, enthaltend eine oder mehrere mesogene oder stabförmige Einheiten, wie in Anspruch 1 definiert,
(B) wenigstens eine Komponente, ausgewählt der Gruppe, welche besteht aus:
(1) wenigstens einem Polycyanat oder Polycyanamid, das keine mesogenen oder stabförmigen Strukturen enthält;
(2) wenigstens einem Epoxyharz;
(3) wenigstens einem Polymaleinimid;
(4) wenigstens einem Polyamin;
(5) wenigstens einem Polyphenol;
(6) wenigstens einer Verbindung, die eine oder mehrere polymerisierbare ethylenartig ungesättigte Gruppe/n enthält;
(7) wenigstens einer Verbindung, die im selben Molekül sowohl eine Cyanat- oder Cyanamidgruppe als auch eine polymerisierbare ethylenartig ungesättigte Gruppe enthält;
(8) wenigstens einer Verbindung, die im selben Molekül sowohl eine 1,2-Epoxidgruppe als auch eine polymerisierbare ethylenartig ungesättigte Gruppe enthält;
(9) wenigstens einer Verbindung, die im selben Molekül sowohl eine Maleinimidgruppe als auch eine Cyanatgruppe enthält;
(10) wenigstens einer Verbindung, die eine oder mehrere mesogene oder stabförmige Einheiten und nur eine Cyanat- oder Cyanamidgruppe pro Molekül enthält;
(11) wenigstens einem Prepolymeren von einem beliebigen der zuvorgenannten Komponenten (1) bis (10) oder einer beliebigen Kombination von beliebig zwei oder mehreren dieser Komponenten; oder
(12) einer Mischung von beliebigen zwei oder mehreren der Komponenten (1) bis (11) in einem beliebigen Verhältnis und einer beliebigen Kombination mit der Maßgabe, daß Komponente (A) 4,4'-Dicyanatostilben (Formel I, R = H, Y = -O-C≡N, A = -CH=CH-) einschließen kann, falls Komponenten (B-4) oder (B-5) nicht eingesetzt werden, und mit der Maßgabe, daß Komponente (A) 4,4'-Dicyanamidoazobenzol (Formel I, R = H, Y = -NH-C≡N, A = -N=N-), 4,4'-Dicyanamidobenzanilid (Formel I, R = H, Y = -NH-C≡N, A = -NH-CO-) oder 4,4'-Dicyanamidophenylbenzoat (Formel I, R = H, Y = -NH-C≡N, A = -CO-O-) einschließen kann, falls Komponenten (B-3) oder (B-4) nicht eingesetzt werden, und mit der Maßgabe, daß Komponente (A) die Zusammensetzungen der Formel IV, worin R = -H oder -CH₃ ist, n = ist, Y = -O-C≡N ist, A" wie zuvor definiert ist, worin beide A Gruppen gleichzeitig -O-CO- sein können, einschließen kann.

5. Polymerisierbare Zusammensetzung nach Anspruch 4, in welcher die Komponenten (A) und (B) in Mengen von 50 bis 90 Gew.-Teilen der Komponente (A) und von 50 bis 10 Gew.-Teilen der Komponente (B) vorliegen, und dieses Polycyanat oder Polycyanamid das Dicyanat von 4,4'-Dihydroxy-α-methylstilben, das Dicyanat von 4,4'-Dihydroxybenzanilid oder eine beliebige Kombination hiervon ist.

6. Aushärtbare Zusammensetzung nach Anspruch 4 oder 5, umfassend einen oder mehrere geeignete Härter oder Aushärtkatalystoren hierfür.

7. Aushärtbare Zusammensetzung nach Anspruch 6, in welcher dieser Härter oder Aushärtkatalysator eine Lewissäure, eine Protonensäure, eine aromatische Hydroxyverbindung, eine Base einschließlich Alkalimetallhydroxiden, -alkoxiden und -phenaten, ein tertiäres Amin, ein quaternäres Ammoniumsalz, Pyridin-N-oxide, Trialkylphosphine, Metallsalze und Metallchelate oder ein organisches Peroxid oder Hydroperoxid oder eine Azo- oder Diazoverbindung oder eine beliebige Kombination hiervon ist.

8. Aushärtbare Zusammensetzung nach Anspruch 3 oder 6, in welcher dieser Härter oder Aushärtkatalysator Kobaltnaphthenat, Kobaltoctoat, Kobaltacetylacetonat oder eine beliebige Kombination hiervon ist.

9. Produkt, resultierend aus dem Polymerisieren der Zusammensetzung von Anspruch 1 oder 4.

10. Produkt nach Anspruch 9, bei welchem diese Zusammensetzung während des Polymerisierens einer Orientierung ausgesetzt wurde.

11. Produkt, resultierend aus dem Aushärten der aushärtbaren Zusammensetzung von Anspruch 3 oder 6.

12. Produkt nach Anspruch 11, bei welchem diese aushärtbare Zusammensetzung während des Aushärtens einer Orientierung ausgesetzt wurde.

13. Produkt nach Anspruch 10 oder 12, bei welchem diese Orientierung mittels eines elektrischen Feldes, eines magnetischen Feldes oder durch Scherspannungen herbeigeführt wurde.

## Revendications

1. Composition de polycyanate ou de polycyanamide comportant un ou plusieurs fragments mésogènes ou en farine de bâtonnets rigides, et représenté par les formules suivantes I, II, III ou IV : 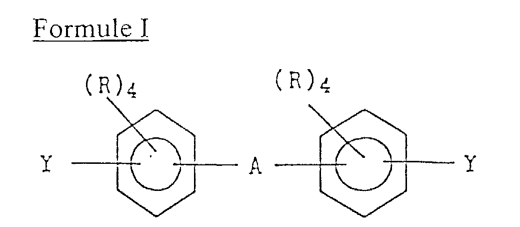 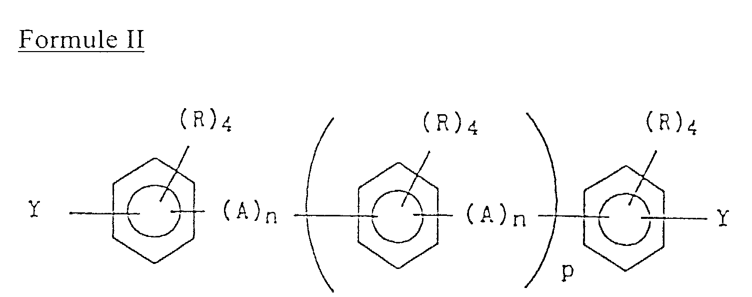 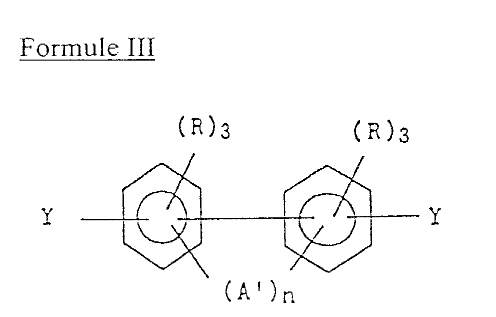 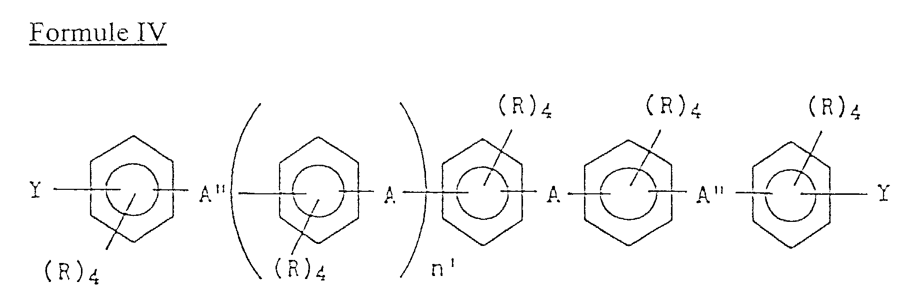 formules dans lesquelles les raccords -A-, ou la liaison directe de la formule III, et les groupes Y se trouvent, en une proportion d'au moins 80 % environ, en positions para les uns par rapport aux autres ; chaque symbole Y représente indépendamment un groupe -O-C≡N ou un groupe -R¹N-C≡N ; chaque symbole A représente indépendamment -CR¹=CR¹-, -C≡C-, -N=N-, -CR¹=N-, -O-CO-, -NR¹-CO, -CR¹=N-N=CR¹-, -CR¹=CR¹-CO-, -CO-O-, -CO-NR¹-, -CO-CR¹=CR¹-, -CR¹=CR¹-O-CO-(CH₂)_{n'}-, -N=CR¹-, -(CH₂)_{n'}-CO-O-CR¹=CR¹, -CR¹-CR¹-O-CO-, -CO-O-CR¹=CR¹-, -CO-O-N=CR¹-, -CR¹=N-O-CO-, -CR¹=CR¹-CO-O-, -CO-S-, -O-CO-CR¹=CR¹-, -CR¹=CR¹-CO-O-(CH₂)_{n'}-, -S-CO-, -(CH₂)_{n'}-O-CO-CR¹=CR¹-, -CHR¹-CHR¹-CO-O-, -O-CO-CHR¹-CHR¹-, -C≡C-C≡C-, -CR¹=CR¹-CR¹=CR¹-, -CO-NR¹-NR¹-CO-, 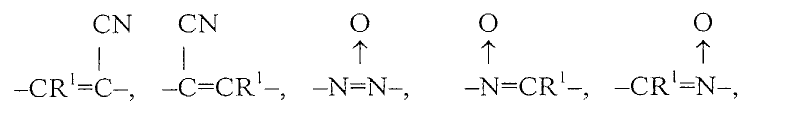 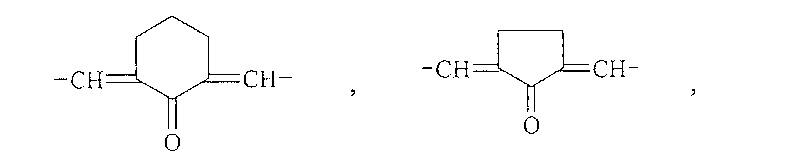 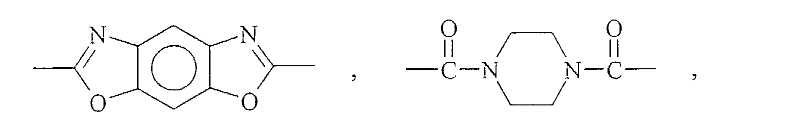 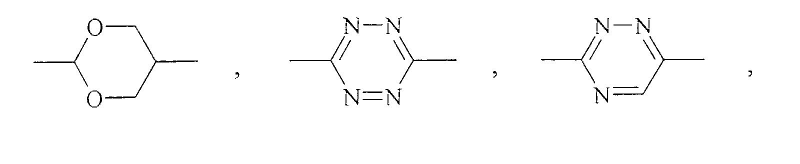 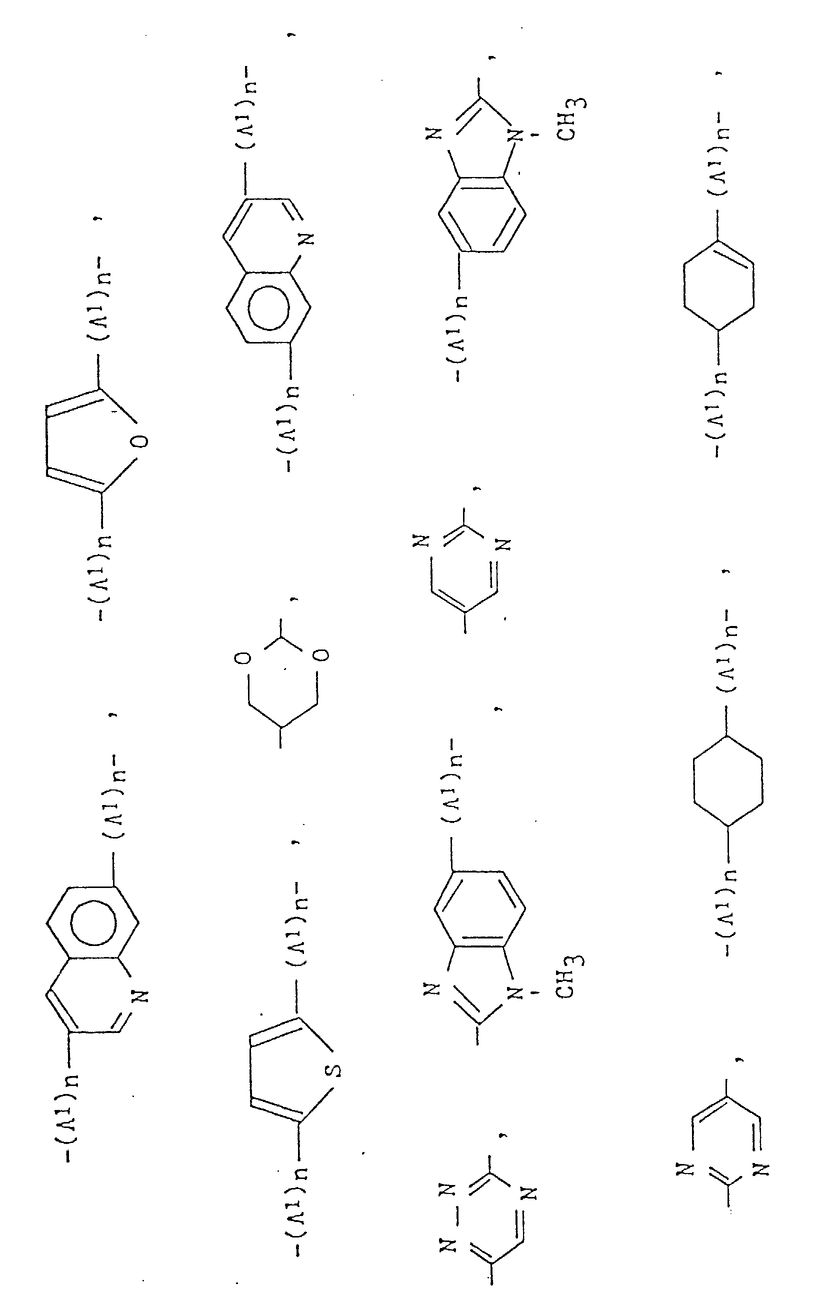 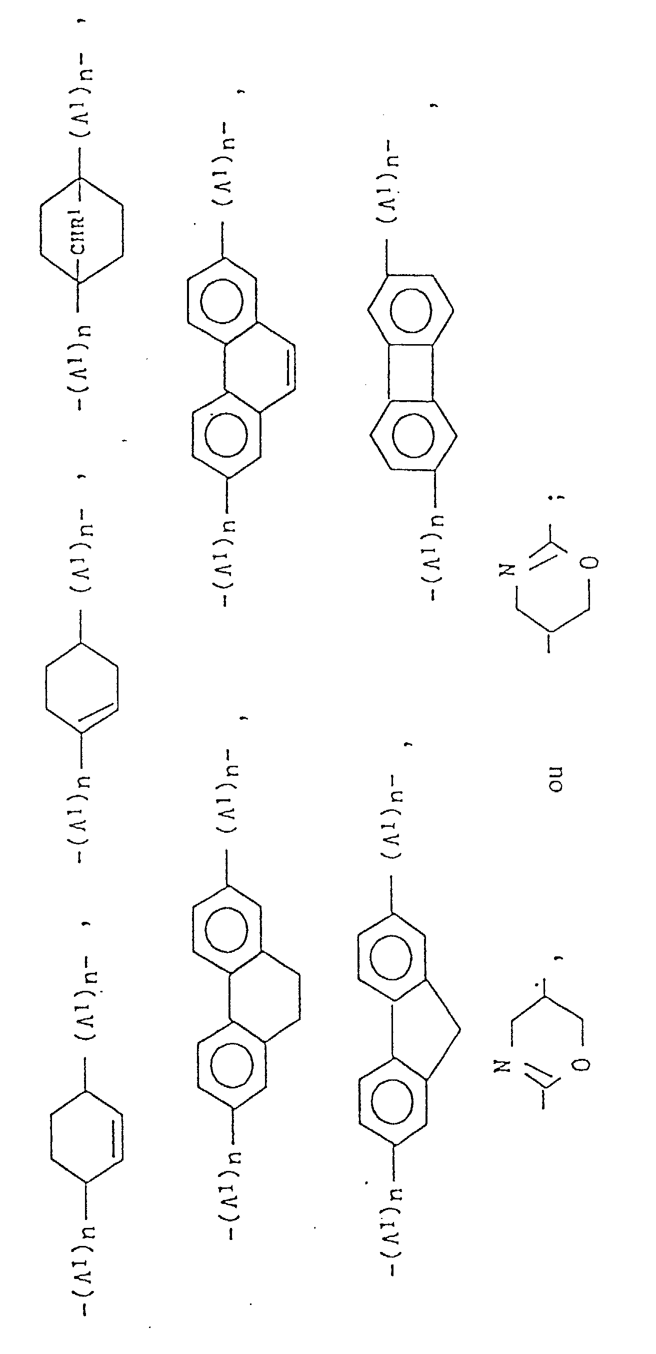 chaque symbole A' représente indépendamment un groupe hydrocarbyle divalent comportant de 1 à 10 atomes de carbone ; chaque symbole A" représente indépendamment un groupe alkylène comportant de 1 à 10 de carbone, une liaison directe, ou un chaînon -O-: -CO-, -S-, -S-S-, -SO-, -SO₂- ou -O-CO-O- ; chaque symbole A¹ représente indépendamment un groupe -CO-, -O-CO-, -CO-O-, -CO-NR¹- ou -NR¹-CO- ; chaque symbole R représente indépendamment un atome d'hydrogène, un groupe hydrocarbyle ou hydrocarbyloxy comportant de 1 à 10 atomes de carbone, un atome d'halogène, un groupe nitro, cyano ou phényle ou un groupe de formule -CO-R¹ ; chaque symbole R¹ représente indépendamment un atome d'hydrogène ou un groupe hydrocarbyle comportant de 1 à 3 atomes de carbone ; n vaut 0 ou 1 ; n' vaut de 1 à 6 ; p vaut de 1 à 30 ; et les cycles aromatiques peuvent aussi comporter un ou plusieurs hétéroatomes choisis parmi N, O et S ;
sous réserve que, dans la formule I, si R représente H et Y représente -O-C≡N, A, ne puisse pas représenter -CH=CH- ; sous réserve que, dans la formule I, si R représente H et Y représente -NH-C≡N, A ne puisse pas représenter -N=N-, -NH-CO- ou -CO-O- ; et sous réserve que, dans la formule IV, si R représente H ou CH₃, n vaut 1, Y représente -O-C≡N et A" a la signification indiquée plus haut, les deux groupes symbolisés par A ne puissent pas être simultanément des groupes -O-CO- ;
à l'exclusion du diisocyanate de bis(4'-hydroxyphényl)-1,4-diiminométhylbenzène.

2. Composition de polycyanate ou de polycyanamide conforme à la revendication 1, dans laquelle ledit polycyanate ou polycyanamide est le dicyanate de 4,4'-dihydroxy-α-méthylstilbène, le dicyanate de 4,4'-dihydroxybenzanilide ou n'importe laquelle de leurs combinaisons.

3. Composition durcissable comprenant une composition de polycyanate ou de polycyanamide conforme à la revendication 1 ou 2, ainsi qu'un ou plusieurs agents durcisseurs ou catalyseurs de durcissement appropriés.

4. Composition polymérisable comportant un mélange de
A) au moins un polycyanate ou polycyanamide thermodurcissable comportant un ou plusieurs fragments mésogènes ou en forme de bâtonnets rigides, du type défini dans la revendication 1, et
B) au moins l'un des constituants suivants :
1) au moins un polycyanate ou polycyanamide ne comportant pas de fragments mésogènes ou en forme de bâtonnets rigides ;
2) au moins une résine époxyde ;
3) au moins un polymaléimide ;
4) au moins une polyamine ;
5) au moins un polyphénol ;
6) au moins un composé comportant un ou plusieurs groupes polymérisables à insaturation éthylénique ;
7) au moins un composé comportant, dans la même molécule, à la fois un groupe cyanate ou cyanamide et un groupe polymérisable à insaturation éthylénique ;
8) au moins un composé comportant, dans la même molécule, à la fois un groupe 1,2-époxyde et un groupe polymérisable à insaturation éthylénique ;
9) au moins un composé comportant, dans la même molécule, à la fois un groupe maléimide et un groupe cyanate ;
10) au moins un composé comportant un ou plusieurs fragments mésogènes ou en forme de bâtonnets rigides et un seul groupe cyanate ou cyanamide par molécule ;
11) au moins un prépolymère de n'importe lequel des constituants (1) à (10) énumérés ci-dessus ou de n'importe quelle combinaison de deux de ces constituants ou plus ; ou
12) un mélange, en toutes proportions et toutes combinaisons, de deux quelconques des constituants (1) à (11) ou plus,
sous réserve que le composant (A) puisse contenir du 4,4'-dicyanatostilbène (formule I, R = H, Y = -O-C≡N, A =-CH=CH-) si l'on n'emploie pas de constituant (B-4) ou (B-5), et sous réserve que le composant (A) puisse contenir du 4,4'-dicyanamidoazobenzène (formule I, R = H, Y = -NH-C≡N, A = -N=N-), du 4,4'-dicyanamidobenzanilide (formule I, R = H, Y = -NH-C≡N, A = -NH-CO-) ou du 4-cyanamidobenzoate de 4'-cyanamidophényle (formule I, R = H, Y = -NH-C≡N, A = -CO-O-) si l'on n'emploie pas de constituant (B-3) ou (B-4), et sous réserve que le composant (A) puisse contenir des compositions de formule IV où R représente H ou CH₃, n vaut 1, Y représente -O-C≡N, A" a la définition indiquée plus haut, et où les deux groupes symbolisés par A peuvent être simultanément des groupes -O-CO-.

5. Composition polymérisable conforme à la revendication 4, dans laquelle les composants (A) et (B) se trouvent présents en des quantités de 50 à 90 parties en poids de composant (A) et de 50 à 10 parties en poids de composant (B), et ledit polycyanate ou polycyanamide est du dicyanate de 4,4'-dihydroxy-α-méthylstilbène, du dicyanate de 4,4'-dihydroxybenzanilide ou n'importe laquelle de leurs combinaisons.

6. Composition durcissable comportant une composition conforme à la revendication 4, et un ou plusieurs agents durcisseurs ou catalyseurs de durcissement appropriés.

7. Composition durcissable conforme à la revendication 6, dans laquelle ledit agent durcisseur ou catalyseur de durcissement est un acide de Lewis, un acide protonique, un composé aromatique hydroxylé, une base, y compris les hydroxydes, alcoolates et phénates de métal alcalin, amines tertiaires, sels d'ammonium quaternaire, pyridine-N-oxydes, trialkylphosphines, sels de métal et chélates de métal, ou encore un peroxyde ou hydroperoxyde organique ou un composé azoïque ou diazoïque, ou n'importe quelle combinaison de tels composés.

8. Composition durcissable conforme à la revendication 3 ou 6, dans laquelle ledit agent durcisseur ou catalyseur de durcissement est du naphténate de cobalt, de l'octanoate de cobalt ou de l'acétylacétonate de cobalt, ou une combinaison quelconque de ces composés.

9. Produit résultant de la polymérisation d'une composition conforme à la revendication 1 ou 4.

10. Produit conforme à la revendication 9, dans lequel ladite composition subit une orientation pendant sa polymérisation.

11. Produit résultant du durcissement d'une composition durcissable conforme à la revendication 3 ou 6.

12. Produit conforme à la revendication 11, dans lequel ladite composition durcissable subit une orientation pendant son durcissement.

13. Produit conforme à la revendication 10 ou 12, dans lequel ladite orientation est réalisée au moyen d'un champ électrique, d'un champ magnétique ou de contraintes de cisaillement.
